# EUROPEAN PATENT APPLICATION

(11) **EP 4 834 879 A1**
(43) Date of publication of application: **04.11.2026**
(21) Application number: 24913049.3
(22) Date of filing: 26.12.2024
(51) Int. Cl.: C07C 237/12, A61K 9/14, A61K 9/51, A61K 31/7088, A61K 31/7105, A61K 38/02, A61K 38/17, A61K 47/10, A61K 47/18, A61K 47/22, A61K 47/24, A61K 47/28, A61K 48/00, A61P 25/00, A61P 43/00, C07D 207/16, C07D 211/34, C07D 243/08, C07D 295/15, C07D 487/04, C12N 15/12

(54) **CARBAMOYL LIPID HAVING CYCLIC GROUP IN SIDE CHAIN, LIPID NANOPARTICLES THEREOF, AND PHARMACEUTICAL COMPOSITION THEREOF**

(30) Priority: 27.12.2023 JP 2023220683; 27.12.2023 JP 2023221885
(71) Applicant: Astellas Pharma Inc., Tokyo 103-8411 (JP)
(72) Inventor: IIKUBO Kazuhiko, Tokyo 103-8411 (JP); TOMINAGA Hiroaki, Tokyo 103-8411 (JP); YAMADA Hiroyoshi, Tokyo 103-8411 (JP); SHIMADA Itsuro, Tokyo 103-8411 (JP); OBA Yasunori, Tokyo 103-8411 (JP); UENO Takako, Tokyo 103-8411 (JP); MORIOKA Shunsuke, Tokyo 103-8411 (JP); MATSUEDA Katsuki, Tokyo 103-8411 (JP); KATO Tetsuro, Tokyo 103-8411 (JP)
(74) Representative: Hoffmann Eitle
(86) International application number: PCT/JP2024/046264
(87) International publication number: WO 2025/143163

(57) **Abstract**

[Object] An object of the invention is to provide a carbamoyl lipid having a cyclic group at a side chain, a lipid nanoparticle containing the lipid, and a pharmaceutical composition thereof.

[Solution] The present inventors have found a carbamoyl lipid having a cyclic group at a side chain which can form a lipid nanoparticle and elucidated that a lipid nanoparticle containing the carbamoyl lipid having a cyclic group at a side chain of the invention as a component can express a protein in an astrocyte or a liver cell. Moreover, a lipid nanoparticle containing the carbamoyl lipid having a cyclic group at a side chain of the invention as a component is expected to be a component of a pharmaceutical composition which encapsulates a nucleic acid and which is useful for preventing and/or treating an astrocyte-related disease.

## Description

### TECHNICAL FIELD

The present invention provides a cationic lipid which is useful as a component of a lipid nanoparticle (also called a compound or a salt thereof below), a lipid nanoparticle of the cationic lipid, a lipid nanoparticle further encapsulating a nucleic acid (also called nucleic acid lipid nanoparticle below), and a pharmaceutical composition containing a nucleic acid lipid nanoparticle.

### BACKGROUND ART

Because nucleic acid such as DNA and mRNA are easily decomposed in the living body, lipid nanoparticle are used for nucleic acid such as DNA and mRNA as pharmaceutical products. Lipid nanoparticle are used as a carrier of a drug delivery system (DDS). As examples of application of lipid nanoparticle, COVID-19 vaccines and cancer vaccines have been put on the market or clinically developed. In nucleic acid lipid nanoparticle, various lipids are used as a cationic lipid, which is one of the components.

A cationic lipid (also called an ionizable lipid) forms lipid nanoparticle and is believed to surround anionic DNA, mRNA, or the like. The optimum cationic lipid can encapsulate a nucleic acid such as DNA and mRNA. Furthermore, it is believed that a cationic lipid can deliver a nucleic acid such as DNA and mRNA to a cell or a tissue, produce a desired protein and exhibit the function thereof (PTL 1).

As a cationic lipid used for lipid nanoparticle, the lipid represented by the general formula below is described (PTL 1).

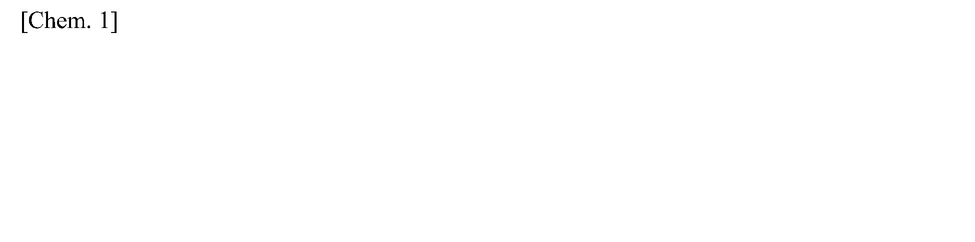

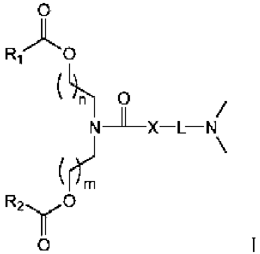

(Refer to PTL 1 for the symbols in the formula.)

As a cationic lipid used for lipid nanoparticle, the lipid represented by the general formula below is described (PTL 2).

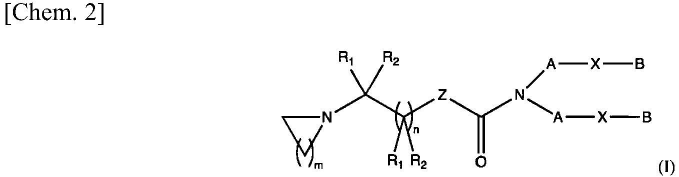

(Refer to PTL 2 for the symbols in the formula.)

There is a technique for inducing differentiation of somatic cells into target cells without through pluripotent stem cells such as iPS cells. When a protein involved in a disease can be produced in specific cells, a specific tissue, or a specific organ, it is expected that an effective therapeutic agent is provided.

Astrocytes (astroglia) are one type of cells which are the most abundant in the central nervous system, play an important role in the homeostasis of blood-brain barrier and synapse formation of neurons in normal environment and have an important role of supporting the normal brain function (Frontiers in Cellular Neuroscience, 2022, vol.16, p.850866 and Toxicologic Pathology, 2011, vol.39(1), p.115-123).

In the case of brain damage, however, astrocytes become activated, accumulate in the damaged site and form gliosis. Gliosis is believed to be a factor inhibiting neuronal regeneration and has been reported to be found in a wide range of central nervous system diseases such as neurodegenerative diseases including Alzheimer's disease (Neuron, 2014, vol. 81, p.229-248).

Accordingly, when an effective drug which is introduced into astrocytes and acts can be developed, a new therapeutic method for a wide range of central nervous system diseases may be provided.

### [Citation List]

### [Patent Literature]

[PTL 1] WO2013/185116
[PTL 2] WO2023/091490

### SUMMARY OF INVENTION

### [Technical Problem]

A main object of the invention is to provide a new cationic lipid which can be a component of a lipid nanoparticle and to create a lipid nanoparticle for delivering a nucleic acid to cells and expressing a protein. Another object of the invention is to provide a lipid nanoparticle encapsulating a nucleic acid (for example, mRNA) which is useful for preventing or treating various diseases and a pharmaceutical composition containing the same.

Another object of the invention is to provide a pharmaceutical composition containing a lipid nanoparticle encapsulating a nucleic acid (for example, mRNA) which is useful for preventing and/or treating an astrocyte-related disease.

Another object of the invention is to provide a lipid nanoparticle encapsulating a nucleic acid (for example, mRNA) which can be delivered to astrocytes and/or liver cells and a pharmaceutical composition containing the same.

### [Solution to Problem]

As a result of intensive examination, the present inventors have found the compound of the formula (I) or the salt thereof of the invention and found that a novel and useful lipid nanoparticle can be produced using the same as a cationic lipid. Moreover, the inventors have examined the proportions of the components of the lipid nanoparticle (a cationic lipid, a neutral lipid, a PEGylated lipid, and the like) and thus found an appropriate composition ratio. The invention can provide such a cationic lipid, a lipid nanoparticle, a nucleic acid lipid nanoparticle, a pharmaceutical composition containing the same, and the like. The invention can provide a lipid nanoparticle encapsulating a nucleic acid (for example, mRNA) which can be delivered to astrocytes and/or liver cells and a pharmaceutical composition containing the same.

That is, the invention relates to (1) to (16) below.

### (Invention)

(1) A compound of a formula (I) or a salt thereof: 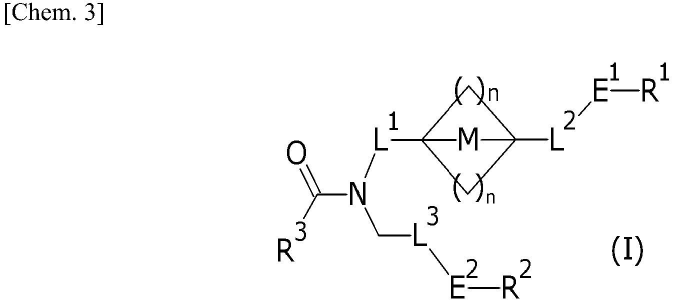 (wherein in the formula,
   L¹ and L² are the same or different and are -CH₂-, -CH₂CH₂-, or a bond,
   L³ is a bond or a C₁₋₁₀ alkylene,
   M is -CH₂- or absent,
   n is 1 or 2,
   wherein n is 1 when M is -CH₂-,
   E¹ and E² are the same or different and are -C(=O)O-*, -OC(=O)-*, -OC(=O)O-*, - C(=O)-*, or a bond,
   wherein * indicates that the group binds to R¹ or R² at the position, and
   one of E¹ and E² is -C(=O)O-* or -OC(=O)-*,
   R¹ and R² are the same or different and are -CH(-R^{x})R^{y}, -CH₂CH(-R^{x})R^{y}, - CH₂CH₂CH(-R^{x})R^{y}, -CH₂CH(-OR^{x})OR^{y}, -CH₂CH₂CH(-OR^{x})OR^{y}, -CH₂-(C₅₋₁₅ alkyl), -CH₂-(C₅₋₂₀ alkenyl), -N(-R^{x})R^{y}, -NR^{y}(-C(=O)R^{x}), or -NR^{y}C(=O)CH(-R^{x})R^{Z},
   wherein, when one of R¹ and R² is -N(-R^{x})R^{y}, one of E¹-R¹ and E²-R² is -OC(=O)-N(-R^{x})R^{y} or -C(=O)-N(-R^{x})R^{y},
   when one of R¹ and R² is -NR^{y}(-C(=O)R^{x}) or -NR^{y}C(=O)CH(-R^{x})R^{Z}, E¹ bonded to the R¹ or E² bonded to the R² is a bond,
   when E¹ and E² are both -OC(=O)-*, R¹ and R² are the same or different and are - CH₂CH₂CH(-OR^{x})OR^{y} or -CH₂-(C₅₋₂₀ alkenyl), and
   R^{x}, R^{y}, and R^{Z} are the same or different and are a C₅₋₁₅ alkyl, and
   R³ is a group selected from the group consisting of the formulae (a) to (i): 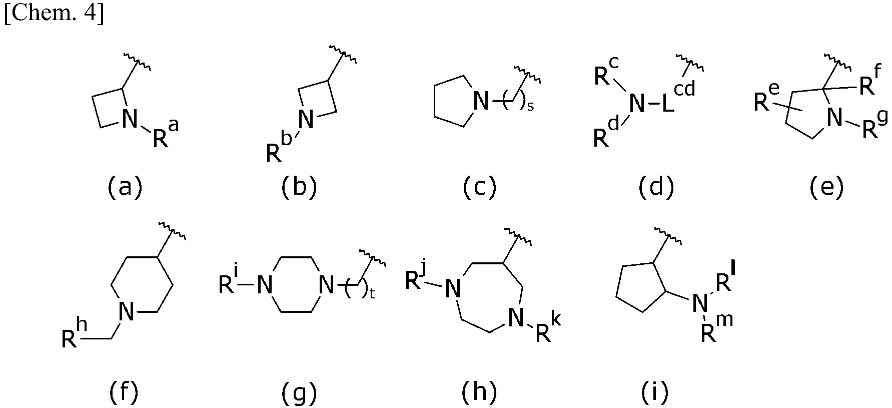
   wherein R^{a} is a C₁₋₆ alkyl,
   R^{b} is -CH₂-C₁₋₆ alkyl or -C(=O)CH₂N(CH₃)₂,
   R^{c} and R^{d} are the same or different and are -CH₃, -CH₂CH₃, or -CH₂CH₂OH, or
   R^{d} is -CH₂C(=O)NH₂ when R^{c} is H,
   L^{cd} is -CH₂-, -CH₂CH₂-, -CH₂CH₂CH₂-, or -CH₂CH(CH₃)-,
   R^{e} is H or OH,
   R^{f} is H,
   R^{g} is a C₁₋₆ alkyl,
   wherein R^{f} and R^{g} may form a pyrrolidine ring together with the carbon atom and the nitrogen atom to which they are bonded,
   R^{h} is a C₁₋₆ alkyl,
   Rⁱ is a C₁₋₆ alkyl or -CH₂CH₂OH,
   R^{j} and R^{k} are the same or different and are a C₁₋₆ alkyl,
   R^{l} and R^{m} are the same or different and are a C₁₋₆ alkyl, and
   s and t are the same or different and are 1 or 2.)

   (1a-1-1) The compound or the salt thereof described in (1) in which R³ is any of the formula (a) to (d) and (f) to (i).
   (1a-1-2) The compound or the salt thereof described in (1) in which R³ is the formula (e).
   (1a-2) The compound or the salt thereof described in any of (1) to (1a-1-2) in which -E¹-R¹ is -C(=O)O-CH₂CH(-R^{x})R^{y},

      -C(=O)O-CH₂CH₂CH(-R^{x})R^{y},

      -OC(=O)-C₃H₆-(C₁₋₆ alkylene)-CH=CH-CH=CH-(C₁₋₆ alkyl),

      -OC(=O)-N(-R^{x})R^{y},

      -OC(=O)O-CH(-R^{x})R^{y},

      -OC(=O)O-CH₂CH(-R^{x})R^{y},

      -NR^{y}(-C(=O)R^{x}),

      or

      -NR^{y}C(=O)CH(-R^{x})R^{Z},

      and

      -E²-R² is -C(=O)O-CH₂CH(-R^{x})R^{y},

      -C(=O)O-CH₂CH₂CH(-R^{x})R^{y},

      -C(=O)O-C₃H₆-(C₁₋₆ alkylene)-CH=CH-CH=CH-(C₁₋₆ alkyl),

      -OC(=O)-CH₂CH(-R^{x})R^{y},

      -OC(=O)-CH₂CH₂CH(-R^{x})R^{y},

      -OC(=O)-CH₂CH₂CH(-OR^{x})OR^{y},

      -OC(=O)-C₃H₆-(C₁₋₆ alkylene)-CH=CH-CH=CH-(C₁₋₆ alkyl),

      or

      -OC(=O)O-CH₂CH(-R^{x})R^{y}.
   (1a-2-1) The compound or the salt thereof described in (1a-2) in which R³ is any of the formula (a) to (d) and (f) to (i).
   (1a-2-2) The compound or the salt thereof described in (1a-2) in which R³ is the formula (e).
   (1a-3) The compound of the formula (I) or the salt thereof described in any of (1) to (1a-2-2)
      in which E¹ and E² are the same or different and are -C(=O)O-*, -OC(=O)-*, or - OC(=O)O-*,
      wherein * indicates that the group binds to R¹ or R² at the position, and
      one of E¹ and E² is -C(=O)O-*,
      R¹ and R² are the same or different and are -CH₂CH(-R^{x})R^{y}, -CH₂CH(-OR^{x})OR^{y}, - CH₂CH₂CH(-OR^{x})OR^{y}, -CH₂-(C₅₋₁₅ alkyl), or -CH₂-(C₅₋₂₀ alkenyl),
      wherein R^{x} and R^{y} are the same or different and are a C₅₋₁₅ alkyl, and
      R³ is a group selected from the group consisting of the formulae (a) to (h): 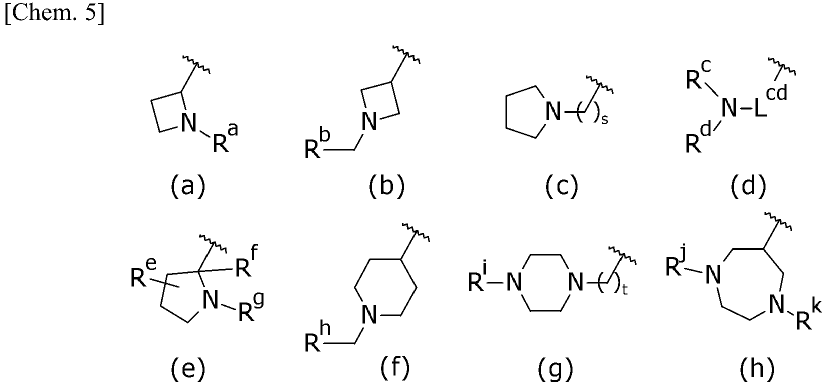
      wherein R^{c} and R^{d} are both -CH₃, -CH₂CH₃, or -CH₂CH₂OH, or
      R^{d} is -CH₂C(=O)NH₂ when R^{c} is H, and
      L^{cd} is -CH₂- or -CH₂CH₂-,
      wherein, when R^{c} and R^{d} are both -CH₃, -CH₂CH₃, or -CH₂CH₂OH, L^{cd} is -CH₂-, - CH₂CH₂-, or -CH₂CH₂-.
   (1a-3-1) The compound or the salt thereof described in (1a-3) in which R³ is any of the formula (a) to (d) and (f) to (h).
   (1a-3-2) The compound or the salt thereof described in (1a-3) in which R³ is the formula (e).
   (1a-4) The compound or the salt thereof described in any of (1a-3) to (1a-3-2) in which -E¹-R¹ is -C(=O)O-CH₂CH(-R^{x})R^{y},

      -OC(=O)-C₃H₆-(C₁₋₆ alkylene)-CH=CH-CH=CH-(C₁₋₆ alkyl),

      or

      -OC(=O)O-CH₂CH(-R^{x})R^{y},

      and

      -E²-R² is -C(=O)O-CH₂CH(-R^{x})R^{y},

      -C(=O)O-C₃H₆-(C₁₋₆ alkylene)-CH=CH-CH=CH-(C₁₋₆ alkyl),

      or

      -OC(=O)-C₃H₆-(C₁₋₆ alkylene)-CH=CH-CH=CH-(C₁₋₆ alkyl).
   (1a-4-1) The compound or the salt thereof described in (1a-4) in which R³ is any of the formula (a) to (d) and (f) to (h).
   (1a-4-2) The compound or the salt thereof described in (1a-4) in which R³ is the formula (e).
   (1a-5) The compound or the salt thereof described in (1a-2)
      in which L¹ is a bond,
      L² is -CH₂- or a bond,
      L³ is a C₁₋₆ alkylene,
      -E¹-R¹ is -C(=O)O-CH₂CH(-R^{x})R^{y},

         -C(=O)O-CH₂CH₂CH(-R^{x})R^{y},

         -OC(=O)O-CH(-R^{x})R^{y},

         or

         -OC(=O)O-CH₂CH(-R^{x})R^{y},
      -E²-R² is -C(=O)O-CH₂CH(-R^{x})R^{y},

         -C(=O)O-CH₂CH₂CH(-R^{x})R^{y},

         -OC(=O)-CH₂CH₂CH(-R^{x})R^{y},

         or

         -OC(=O)-CH₂CH₂CH(-OR^{x})OR^{y},
      wherein R^{x} and R^{y} are the same or different and are a C₅₋₁₅ alkyl, and
      R³ is a group selected from the group consisting of the formulae (d), (e), (g), and (h): 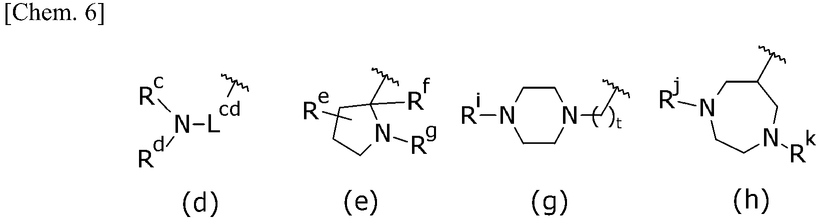
      wherein R^{c} and R^{d} are the same or different and are -CH₃, -CH₂CH₃, or - CH₂CH₂OH,
      L^{cd} is -CH₂-, -CH₂CH₂-, or -CH₂CH₂CH₂-,
      R^{e} is H,
      R^{f} is H,
      R^{g} is a C₁₋₆ alkyl,
      Rⁱ is a C₁₋₆ alkyl,
      R^{j} and R^{k} are the same or different and are a C₁₋₆ alkyl, and
      t is 1.
   (1a-5-1) The compound or the salt thereof described in (1a-5) in which R³ is the formula (d), (h), or (g).
   (1a-5-2) The compound or the salt thereof described in (1a-5) in which R³ is the formula (e).
   (1a-6) The compound or the salt thereof described in any of (1a-5) to (1a-5-2) in which L³ is a C₆ alkylene,
      -E¹-R¹ is -C(=O)O-CH₂CH(-R^{x})R^{y} or -OC(=O)O-CH₂CH(-R^{x})R^{y},
      -E²-R² is -C(=O)O-CH₂CH(-R^{x})R^{y},
      R^{c} and R^{d} are the same or different and are -CH₃, -CH₂CH₃, or -CH₂CH₂OH,
      L^{cd} is -CH₂-, -CH₂CH₂-, or -CH₂CH₂CH₂-,
      wherein, when R^{c} and R^{d} are both -CH₃, -CH₂CH₃, or -CH₂CH₂OH, L^{cd} is -CH₂-, - CH₂CH₂-, or -CH₂CH₂-.
   (1b-1) The compound or the salt thereof described in (1) in which one of -E¹-R¹ and -E²-R² is -C(=O)O-CH₂CH(-R^{x})R^{y}, and the other is -C(=O)O-CH₂CH(-R^{x})R^{y}, -OC(=O)O-CH₂CH(-R^{x})R^{y}, -OC(=O)-CH₂CH(-OR^{x})OR^{y}, -OC(=O)-CH₂CH₂CH(-OR^{x})OR^{y}, or -OC(=O)-CH₂-(C₅₋₁₅ alkenyl).
   (1b-2) The compound or the salt thereof described in (1) in which R³ is a group selected from the group consisting of the formulae (d), (e), (g), and (h). 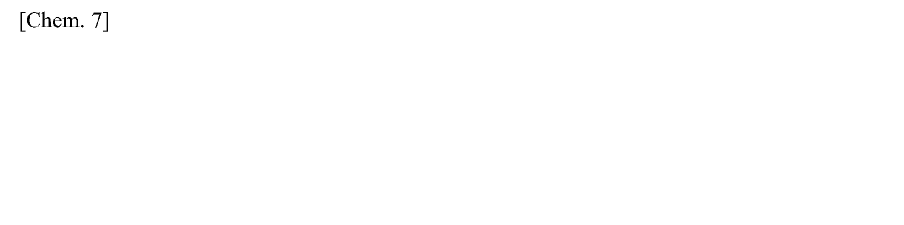 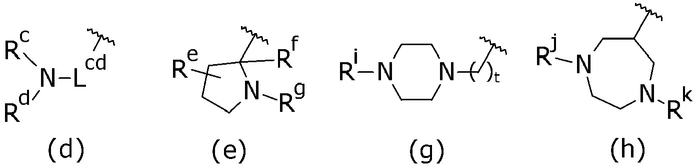
   (1b-3) The compound or the salt thereof described in (1b-2) in which R^{c} and R^{d} are both -CH₂CH₃, L^{cd} is -CH₂CH₂-, R^{e} is H, R^{f} is H, R^{g} is a C₁₋₆ alkyl, Rⁱ is -CH₃, t is 1, and R^{j} and R^{k} are -CH₂CH₃.
   (1b-4) The compound or the salt thereof described in (1b-3) in which R³ is the formula (e). The compound or the salt thereof described in (1b-3) in which R³ is the formula (d) or (e).
   (1b-5) The compound or the salt thereof described in (1b-4) in which R^{g} is -CH₃.
   (1b-6) The compound or the salt thereof described in (1b-3) in which R³ is the formula (h).
(2) The compound or the salt thereof described in (1) in which the compound is selected from the group consisting of
   2-nonylundecyl 8-{(1-methyl-L-prolyl)[(1*r*,3*S*)-3-{2-[(2-octyldecyl)oxy]-2-oxoethyl}cyclobutyl]amino}octanoate,
   2-nonylundecyl 8-[(1-methyl-L-prolyl)(3-{2-[(2-octyldecyl)oxy]-2-oxoethyl}bicyclo[1.1.1]pentan-1-yl)amino]octanoate,
   2-nonylundecyl 8-{(*N,N*-diethyl-β-alanyl)[(1*r*,3*r*)-3-{2-[(2-octyldecyl)oxy]-2-oxoethyl}cyclobutyl]amino}octanoate,
   2-nonylundecyl 8-{(1,4-diethyl-1,4-diazepane-6-carbonyl)[(1*r*,3*r*)-3-{2-[(2-octyldecyl)oxy]-2-oxoethyl } cyclobutyl] amino } octanoate,
   2-nonylundecyl 8-{[(4-methylpiperazin-1-yl)acetyl][(1*r*,3*r*)-3-{2-[(2-octyldecyl)oxy]-2-oxoethyl}cyclobutyl]amino } octanoate, and
   2-heptylnonyl (1*S*,4*r*)-4-[{8-[(2-heptylnonyl)oxy]-8-oxooctyl}(1-methyl-L-prolyl)amino]cyclohexane-1-carboxylate.

   (2-1) A compound or a salt thereof in which the compound is
      2-nonylundecyl 8-{(1-methyl-L-prolyl)[(1*r*,3*S*)-3-{2-[(2-octyldecyl)oxy]-2-oxoethyl}cyclobutyl]amino}octanoate,
      2-nonylundecyl 8-[(1-methyl-L-prolyl)(3-{2-[(2-octyldecyl)oxy]-2-oxoethyl}bicyclo[1.1.1]pentan-1-yl)amino]octanoate,
      2-nonylundecyl 8-{(1-ethyl-L-prolyl)[(1*r*,3*S*)-3-{2-[(2-octyldecyl)oxy]-2-oxoethyl}cyclobutyl]amino}octanoate,
      2-nonylundecyl 8-{(*N,N*-diethyl-β-alanyl)[(1*r*,3*r*)-3-{2-[(2-octyldecyl)oxy]-2-oxoethyl}cyclobutyl]amino}octanoate,
      2-nonylundecyl 8-{(1,4-diethyl-1,4-diazepane-6-carbonyl)[(1*r*,3*r*)-3-{2-[(2-octyldecyl)oxy]-2-oxoethyl}cyclobutyl]amino}octanoate,
      2-nonylundecyl 8-{[(4-methylpiperazin-1-yl)acetyl][(1*r*,3*r*)-3-{2-[(2-octyldecyl)oxy]-2-oxoethyl}cyclobutyl]amino}octanoate,
      2-nonylundecyl 8-[(1-methyl-L-prolyl){(1*r*,3*S*)-3-[({[(2-octyldecyl)oxy]carbonyl}oxy)methyl]cyclobutyl}amino]octanoate,
      2-heptylnonyl (1*S*,4*r*)-4-[{8-[(2-heptylnonyl)oxy]-8-oxooctyl}(1-methyl-L-prolyl)amino]cyclohexane-1-carboxylate,
      2-nonylundecyl 8-{(1-ethyl-D-prolyl)[(1*r*,3*R*)-3-{2-[(2-octyldecyl)oxy]-2-oxoethyl}cyclobutyl]amino}octanoate,
      3-decyltridecyl 8-{[(1*r*,3*S*)-3-{2-[(3-decyltridecyl)oxy]-2-oxoethyl}cyclobutyl](1-methyl-L-prolyl)amino}octanoate,
      2-{(1-ethyl-L-prolyl)[(1*r*,3*S*)-3-{2-[(2-octyldecyl)oxy]-2-oxoethyl}cyclobutyl]amino}ethyl 4,4-bis(octyloxy)butanoate, or
      4-[(1-ethyl-L-prolyl){(1*r*,3*S*)-3-[({[(2-nonylundecyl)oxy]carbonyl}oxy)methyl]cyclobutyl}amino]butyl 4-octyldodecanoate.
   (2-2) A compound or a salt thereof in which the compound is
      2-nonylundecyl 8-{(1-ethyl-L-prolyl)[(1*r*,3*S*)-3-{2-[(2-octyldecyl)oxy]-2-oxoethyl}cyclobutyl]amino}octanoate or
      2-nonylundecyl 8-{(*N,N*-diethyl-β-alanyl)[(1*r*,3*r*)-3-{2-[(2-octyldecyl)oxy]-2-oxoethyl}cyclobutyl]amino}octanoate.
(3) A lipid nanoparticle containing the compound or the salt thereof described in (1).
(4) A lipid nanoparticle containing the compound or the salt thereof described in (1), a neutral lipid, and a PEGylated lipid.
(5) The lipid nanoparticle described in (4) which encapsulates a nucleic acid.
(6) The lipid nanoparticle described in (5) in which the nucleic acid is mRNA.
(7) The lipid nanoparticle described in any of (4) to (6)
   in which the neutral lipid is a phospholipid and a sterol,
   wherein the phospholipid is DPPC, DSPC, SOPC, DoPhPE, DOPS, or DHSM, and
   the sterol is cholesterol, 7α-hydroxycholesterol, or β-sitosterol, and
   the PEGylated lipid is DMG-PEG2000, PEG monostearate, or C8 PEG2000 ceramide.
(8) The lipid nanoparticle described in any of (5) to (7) which can express a protein in an astrocyte.
(9) The lipid nanoparticle described in any of (5) to (8) in which the nucleic acid is mRNA which is useful for preventing and/or treating an astrocyte-related disease.
(10) The lipid nanoparticle described in any of (5) to (9)
   in which the nucleic acid is mRNA encoding NeuroD1 protein,
   the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof,
   the neutral lipid is DSPC and cholesterol, and
   the PEGylated lipid is DMG-PEG2000.
(11) The lipid nanoparticle described in any of (5) to (10) in which the nucleic acid is mRNA encoding NeuroD1 protein containing a base sequence encoding a protein having the amino acid sequence of SEQ ID NO: 2.
(12) The lipid nanoparticle described in any of (3) to (11) which contains 20.0 to 80.0 mol% of the compound or the salt thereof described in (1), 18.5 to 78.5 mol% of a neutral lipid, and 0.5 to 2.5 mol% of a PEGylated lipid based on the total amount of the lipid nanoparticle.
(13) The lipid nanoparticle described in any of (3) to (11) which contains 30.0 to 60.0 mol% of the compound or the salt thereof described in (1), 38.5 to 68.5 mol% of a neutral lipid, and 0.5 to 2.0 mol% of a PEGylated lipid based on the total amount of the lipid nanoparticle.
(14) A pharmaceutical composition containing the lipid nanoparticle described in any of (5) to (13).
(15) A pharmaceutical composition containing the lipid nanoparticle described in any of (5) to (13) and one or more pharmaceutically acceptable pharmaceutical additives.
(16) The pharmaceutical composition described in (15) which is a pharmaceutical composition for preventing and/or treating an astrocyte-related disease.

The invention also relates to
a pharmaceutical composition for preventing and/or treating an astrocyte-related disease which contains the lipid nanoparticle described in any of (5) to (13) containing the compound of the formula (I) or the salt thereof.

The pharmaceutical composition includes an agent for preventing and/or treating an astrocyte-related disease which contains the lipid nanoparticle described in any of (5) to (13) containing the compound of the formula (I) or the salt thereof.

The invention also relates to
use of the lipid nanoparticle described in any of (5) to (13) containing the compound of the formula (I) or the salt thereof for the manufacture of a pharmaceutical composition for preventing and/or treating an astrocyte-related disease,
the lipid nanoparticle described in any of (5) to (13) containing the compound of the formula (I) or the salt thereof for use in prevention and/or treatment of an astrocyte-related disease,
use of the lipid nanoparticle described in any of (5) to (13) containing the compound of the formula (I) or the salt thereof for the prevention and/or the treatment of an astrocyte-related disease, and
a method for preventing and/or treating an astrocyte-related disease including administering an effective amount of the lipid nanoparticle described in any of (5) to (13) containing the compound of the formula (I) or the salt thereof to a subject.

Moreover, the invention relates to a method for delivering a nucleic acid (for example, a nucleic acid which is useful for preventing and/or treating an astrocyte-related disease) to cells of the body, in particular astrocytes, using a nucleic acid lipid nanoparticle containing the compound of the formula (I) or the salt thereof.

The invention relates to a lipid nanoparticle encapsulating a nucleic acid (for example, mRNA) which can be delivered to astrocytes or liver cells and a pharmaceutical composition containing the same.

The "astrocyte-related disease" is a disease in which astrocytes are involved and is cerebral infarction, cerebral hemorrhage, traumatic brain injury, a neurodegenerative disease, amyotrophic lateral sclerosis, Duchenne muscular dystrophy, Alexander disease, multiple sclerosis, spinal cord injury, or neuromyelitis optica. The astrocyte-related disease is, in an aspect, cerebral hemorrhage, traumatic brain injury, a neurodegenerative disease, amyotrophic lateral sclerosis, Duchenne muscular dystrophy, Alexander disease, multiple sclerosis, or neuromyelitis optica.

Here, cerebral infarction is penetrating branch infarction or cerebral infarction having brain damage in the penetrating branch territory in an aspect, subacute-phase cerebral infarction in an aspect, subacute-phase to chronic-phase cerebral infarction in an aspect, chronic-phase cerebral infarction in an aspect, cerebral infarction having motor dysfunction with high severity in an aspect, cerebral infarction with modified Rankin Scale (mRS) of 2 or more in an aspect, cerebral infarction with mRS of 4 or more in an aspect, cerebral infarction of a combination thereof in an aspect, or penetrating branch infarction, cerebral infarction having brain damage in the penetrating branch territory, subacute-phase to chronic-phase cerebral infarction, and cerebral infarction having motor dysfunction with high severity in an aspect. The phases of cerebral infarction are classified as follows. The "acute-phase" is a period until 72 hours after the onset of cerebral infarction, in which the symptom may deteriorate. The "subacute-phase" is a period from 72 hours after the onset of cerebral infarction to before one month thereafter. The "subacute-phase to chronic-phase" is at and after 72 hours after the onset of cerebral infarction. The "chronic-phase" is the period at and after one month after the onset of cerebral infarction.

Here, "spinal cord injury" is subacute-phase to chronic-phase spinal cord injury in an aspect, chronic-phase spinal cord injury in an aspect, spinal cord injury with quadriplegia in an aspect, spinal cord injury with upper arm motor dysfunction in an aspect, spinal cord injury including cervical cord injury in an aspect, or spinal cord injury due to crush injury in an aspect. The phases of spinal cord injury are classified as follows. The "acute-phase" is a period from soon after the occurrence of spinal cord injury to before two weeks thereafter. The "subacute-phase" (also called recovery period) is a period from two weeks after the occurrence of spinal cord injury to before four weeks thereafter. The "chronic-phase" is at and after four weeks after the occurrence. The "subacute-phase to chronic-phase" is a period at and after two weeks after the occurrence of spinal cord injury in an aspect, a period at and after four weeks after the occurrence of spinal cord injury in an aspect, a period at and after three months after the occurrence of spinal cord injury in an aspect, or a period at and after six months after the occurrence of spinal cord injury in an aspect.

This specification includes the contents disclosed in Japanese patent application No. 2023-221885 and Japanese patent application No. 2023-220683, which the present application claims priorities from.

### [Advantageous Effects of Invention]

The invention is not limited to the aspects above and also includes aspects of appropriate combinations of the contents described in the detailed invention of the specification.

The lipid which is the compound of the formula (I) or a salt thereof can form a lipid nanoparticle as a component of the lipid nanoparticle. The obtained lipid nanoparticle can deliver a nucleic acid to target cells, such as astrocytes, and express a protein.

The lipid nanoparticle can deliver a nucleic acid which is useful for preventing and/or treating an astrocyte-related disease to cells of a subject and express a protein. That is, a pharmaceutical composition which is useful for preventing and/or treating an astrocyte-related disease can be provided. The pharmaceutical composition containing the nucleic acid lipid nanoparticle of the invention can be used as an agent for preventing and/or treating an astrocyte-related disease.

### BRIEF DESCRIPTION OF DRAWINGS

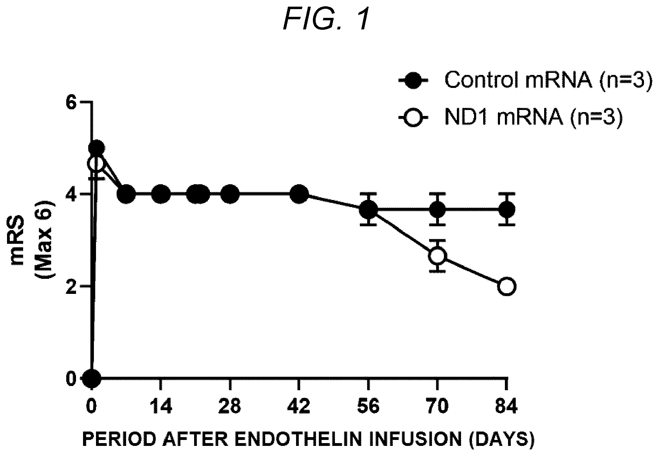

Fig. 1 shows the results of evaluation of the changes in motor dysfunction with days after endothelin-1 treatment (induction of cerebral ischemia) of the NeuroD1 (ND1) nucleic acid lipid nanoparticle group (ND1 mRNA) described in Test Example 4 and the control group (Control mRNA) using the modified Rankin Scale (mRS). The horizontal axis shows the period after the endothelin-1 infusion (days), and the vertical axis shows the mRS values (maximum value of 6). The white circles show the averages of the mRS of the ND1 nucleic acid lipid nanoparticle group at the points of evaluation. Here, the mRS was evaluated every two weeks on and after day 28 after the endothelin-1 infusion, and as the results on day 28, the average of the results of the evaluation of the individuals on day 28 or 29 is shown. The error bars indicate the standard errors (SEM). The black circles show the averages of the mRS of the control group at the points of evaluation. As the results on day 28, the average of the results of the evaluation of the individuals on day 28 or 29 is shown. The nucleic acid lipid nanoparticle were administered on day 21 after the endothelin-1 treatment.

### DESCRIPTION OF EMBODIMENTS

The invention will be explained in detail below.

In this specification, the terms below have the meanings below unless otherwise specifically noted. The definitions below are for clarifying the defined terms but do not limit the terms. When the terms used here are not specifically defined, the terms are used with the meanings which are generally accepted by one skilled in the art. Unless otherwise specifically noted, when a symbol in a chemical formula in this specification is also used in another chemical formula, the same symbol has the same meaning.

The "alkyl" is a linear or branched alkyl. The C₁₋₆ alkyl is an alkyl having one to six carbon atoms. The C₅₋₁₀ alkyl is an alkyl having five to 10 carbon atoms. In an aspect, the alkyl is a C₁₋₆ alkyl. In an aspect, the alkyl is a C₅₋₁₀ alkyl. In an aspect, the alkyl is a C₅₋₁₅ alkyl. In an aspect, the alkyl is a C₆₋₁₀ alkyl. In an aspect, the alkyl is a C₇₋₉ alkyl. In an aspect, the alkyl is a C₇₋₈ alkyl. In an aspect, the alkyl is a C₈₋₉ alkyl. In an aspect, the alkyl is a C₇ alkyl. In an aspect, the alkyl is a C₆₋₉ alkyl. In an aspect, the alkyl is a C₆ alkyl. In an aspect, the alkyl is a C₈ alkyl. In an aspect, the alkyl is a C₉ alkyl. In an aspect, the alkyl is n-hexyl. In an aspect, the alkyl is n-heptyl. In an aspect, the alkyl is n-octyl. In an aspect, the alkyl is n-nonyl.

The "alkylene" is a linear or branched alkylene. The C₅₋₁₀ alkylene is an alkylene having five to 10 carbon atoms. In an aspect, the alkylene is a C₁₋₁₀ alkylene. In an aspect, the alkylene is a C₁₋₆ alkylene. In an aspect, the alkylene is a C₅₋₈ alkylene. In an aspect, the alkylene is a C₇₋₉ alkylene. In an aspect, the alkylene is a C₆₋₈ alkylene. In an aspect, the alkylene is a C₆ alkylene. In an aspect, the alkylene is hexanediyl, heptanediyl, octanediyl, or nonanediyl. In an aspect, the alkylene is heptanediyl, octanediyl, or nonanediyl. In an aspect, the alkylene is heptane-1,7-diyl, octane-1,8-diyl, or nonane-1,9-diyl. In an aspect, the alkylene is n-hexane-1,6-diyl.

The "alkenyl" is a linear or branched alkenyl, and the C₅₋₂₀ alkenyl is a linear or branched alkenyl having five to 20 carbon atoms, such as vinyl, propenyl, butenyl, pentenyl, 1-methylvinyl, 1-methyl-2-propenyl, 1,3-butadienyl, and 1,3-pentadienyl. In an aspect, the alkenyl is -C₅₋₁₀ alkylene-CH=CH-CH=CH-C₅₋₁₀ alkyl. In an aspect, the alkenyl is a linear alkenyl. In an aspect, the alkenyl is -C₈ alkylene-(CH=CH-CH₂)₂-C₄ alkyl, -C₈ alkylene-(CH=CH-CH₂)₂-C₆ alkyl, -C₇ alkylene-CH=CH-C₈ alkyl, -C₇ alkylene-(CH=CH-CH₂)₂-C₄ alkyl, -C₇ alkylene-(CH=CH-CH₂)₃-C₄ alkyl, -C₃ alkylene-(CH=CH-CH₂)₄-C₄ alkyl, -C₃ alkylene-(CH=CH-CH₂)₅-C₁ alkyl, or -C₂ alkylene-(CH=CH-CH₂)₆-C₁ alkyl. In an aspect, the alkenyl is -C₈ alkylene-(CH=CH-CH₂)₂-C₄ alkyl (here, all the double bonds have *Z* configuration), -C₈ alkylene-(CH=CH-CH₂)₂-C₆ alkyl (here, all the double bonds have *Z* configuration), -C₇ alkylene-CH=CH-C₈ alkyl (here, all the double bonds have *Z* configuration), -C₇ alkylene-(CH=CH-CH₂)₂-C₄ alkyl (here, all the double bonds have *Z* configuration), -C₇ alkylene-(CH=CH-CH₂)₃-C₄ alkyl (here, all the double bonds have *Z* configuration), -C₃ alkylene-(CH=CH-CH₂)₄-C₄ alkyl (here, all the double bonds have *Z* configuration), -C₃ alkylene-(CH=CH-CH₂)₅-C₁ alkyl (here, all the double bonds have *Z* configuration), or -C₂ alkylene-(CH=CH-CH₂)₆-C₁ alkyl (here, all the double bonds have *Z* configuration). In an aspect, the alkenyl is -C₇ alkylene-(CH=CH-CH₂)₂-C₄ alkyl (here, all the double bonds have *Z* configuration). In an aspect, the alkenyl is -C₆ alkylene-(CH=CH-CH₂)₂-C₄ alkyl (here, all the double bonds have *Z* configuration). In an aspect, the alkenyl is -C₈ alkylene-(CH=CH-CH₂)₂-C₄ alkyl (here, all the double bonds have *Z* configuration). In an aspect, "-CH₂-(C₅₋₂₀ alkenyl)" is -C₃H₆-(C₁₋₆ alkylene)-CH=CH-CH₂-CH=CH-(C₁₋₆ alkyl).

The "halogen" is F, Cl, Br, or I.

The "lipid nanoparticle" is a nanoparticle containing lipid as a main component. In general, the lipid nanoparticle contains a cationic lipid, a neutral lipid, and a PEGylated lipid. In an aspect, the lipid nanoparticle is a nucleic acid lipid nanoparticle further encapsulating a nucleic acid. The "nucleic acid lipid nanoparticle" is a lipid nanoparticle encapsulating a nucleic acid which is useful for preventing and/or treating a disease, a lipid nanoparticle encapsulating a nucleic acid which is useful for preventing and/or treating an astrocyte-related disease in an aspect, or a lipid nanoparticle encapsulating mRNA in an aspect.

The "particle size" is the particle size of the lipid nanoparticle. The particle size is measured as hydrodynamic diameter (Dh) of the dispersion in a static state with a particle size analyzer (Zetasizer(registered trademark) Nano ZSP or Ultra, Malvern Panalytical) using the Dynamic Light Scattering (DLS) method. The particle size is in accordance with ISO22412. The "particle size" of the lipid nanoparticle is calculated as a Z-average particle size. The particle size of the lipid nanoparticle is 10 nm to 1000 nm in an aspect, 30 nm to 500 nm in an aspect, or 30 nm to 250 nm in an aspect. The particle size is 60 nm to 180 nm in an aspect. The particle size is 70 nm to 110 nm in an aspect. The particle size is 70 nm to 100 nm in an aspect. The particle size is 80 nm to 100 nm in an aspect. The particle size is 90 nm to 100 nm in an aspect.

The "cationic lipid" is a compound which can carry a cation (positive electric charge) in the molecule in response to the pH and which has a fatty acid chain or a salt thereof. In an aspect, the cationic lipid is the compound of the formula (I) or a salt thereof.

"Neutral lipid" are "phospholipid" and "sterol". In an aspect, the neutral lipid is a phospholipid and a sterol. In an aspect, the neutral lipid is a phospholipid, and in an aspect, the neutral lipid is a sterol.

The "phospholipid" is a lipid having a phosphoric acid ester group. The phospholipid is phosphatidylcholine (PC), phosphatidylethanolamine (PE), phosphatidylglycerol (PG), phosphatidylserine (PS), sphingomyelin (SM), or a combination thereof.

The phosphatidylcholine (PC) is 1,2-dierucoyl-sn-glycero-3-phosphocholine (DEPC), 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1-stearoyl-2-oleoyl-sn-glycero-3-phosphocholine (SOPC), or the like.

The phosphatidylethanolamine (PE) is 1,2-dierucoyl-sn-glycero-3-phosphoethanolamine (DEPE), 1,2-dimyristoyl-sn-glycero-3-phosphoethanolamine (DMPE), 1,2-dioleoyl-sn-glycero-3-phosphoethanolamine (DOPE), 1,2-dipalmitoyl-sn-glycero-3-phosphoethanolamine (DPPE), 1,2-di-O-phytanyl-sn-glycero-3-phosphoethanolamine (DoPhPE; CAS150135-14-1), 1,2-distearoyl-sn-glycero-3-phosphoethanolamine (DSPE), 1-palmitoyl-2-oleoyl-sn-glycero-3-phosphoethanolamine (POPE), or the like.

The phosphatidylglycerol (PG) is 1,2-dimyristoyl-sn-glycero-3-phospho-rac-(1-glycerol) sodium salt (DMPG), 1,2-dioleoyl-sn-glycero-3-phospho-rac-(1-glycerol) sodium salt (DOPG), 1,2-dipalmitoyl-sn-glycero-3-phospho-rac-(1-glycerol) sodium salt (DPPG), 1,2-distearoyl-sn-glycero-3-phospho-rac-(1-glycerol) sodium salt (DSPG), or the like.

The phosphatidylserine (PS) is phosphatidylserine (PS), 1,2-dioleoyl-sn-glycero-3-phospho-L-serine (DOPS), or the like.

The sphingomyelin (SM) is sphingomyelin (SM), dihydrosphingomyelin (DHSM), or the like. In an aspect, the phospholipid is DPPC, DSPC, SOPC, DOPE, DoPhPE, DOPS, or DHSM. In an aspect, the phospholipid is DSPC. In an aspect, the phospholipid is not used, and DOTAP or DOTMA is used.

The "sterol" is a steroid alcohol and is a compound having a hydroxy group on the A ring of a steroid skeleton. In an aspect, the sterol is cholesterol and corticosteroid. In an aspect, the sterol is cholesterol, 7α-hydroxycholesterol, brassicasterol, ergosterol, fecosterol, campesterol, sitosterol, β-sitosterol, stigmasterol, tomatidine, tomatine, ursolic acid, a mixture thereof, or the like. Alternatively, the sterol is a combination of two or more kinds. In an aspect, the sterol is cholesterol, 7α-hydroxycholesterol, campesterol, or β-sitosterol. In an aspect, the sterol is cholesterol.

The "PEGylated lipid" refers to a lipid having polyethylene glycol (PEG). The PEGylated lipid is a lipid modified with polyethylene glycol. The PEGylated lipid is PEG-modified ceramide, PEG-modified dialkylamine, PEG-modified diacylglycerol, PEG-modified dialkylglycerol, PEG-modified phosphatidic acid, PEG-modified phosphatidylethanolamine, a mixture thereof, or the like. In an aspect, the PEGylated lipid is 1,2-dilauroyl-sn-glycero-3-phosphoethanolamine-PEG (DLPE-PEG), 1,2-dimyristoyl-rac-glycero-3-methoxy-PEG (DMG-PEG2000, also known as DMG-PEG), 1,2-dimyristoyl-sn-glycero-3-phosphoethanolamine-PEG (DMPE-PEG), 1,2-dipalmitoyl-rac-glycero-3-methoxy PEG (DPG-PEG), 1,2-dipalmitoyl-sn-glycero-3-phosphocholine-PEG (DPPC-PEG), 1,2-distearoyl-rac-glycero-3-PEG (DSG-PEG), 1,2-distearoyl-sn-glycero-3-phosphoethanolamine-PEG (DSPE-PEG), PEG monostearate, or *N*-octanoyl-sphingosine-1-{succinyl[methoxy(polyethylene glycol)2000]} (C8 PEG2000 ceramide).

In an aspect, the PEGylated lipid is DMG-PEG2000, PEG monostearate, or *N-*octanoyl-sphingosine-1-{succinyl[methoxy(polyethylene glycol)2000]} (C8 PEG2000 ceramide), and in an aspect, the PEGylated lipid is DMG-PEG2000.

The "encapsulation efficiency" refers to the amount of the nucleic acid introduced into the lipid nanoparticle based on the total amount of the nucleic acid in the lipid nanoparticle dispersion. For example, when 98 mg of the nucleic acid of a total amount of 100 mg of the nucleic acid is introduced into the lipid nanoparticle, the encapsulation efficiency can be indicated as 98%. The "encapsulation", when used in this specification, means being completely or substantially contained in the inside or being encompassed In nucleic acid lipid nanoparticle containing mRNA or the like, the encapsulation efficiency of the nucleic acid is measured by the method described in detail below. The value of the encapsulation efficiency of the nucleic acid is 70% or more in an aspect, 80% or more in an aspect, 90% or more in an aspect, 93% or more in an aspect, or 95% or more in an aspect.

The "N/P ratio" is a value obtained by dividing the number of moles of the amino group of the cationic lipid in the nucleic acid lipid nanoparticle (N) by the number of moles of the phosphoric acid of RNA (P). In this specification, the value of the N/P ratio was converted, while the amino group was regarded as a nitrogen atom in which the value of ACD/pKa GALAS calculated using ACD/Percepta (ACD/Labs Release 2019.2.2 or 2023.1.1, registered trademark, Advanced Chemistry Development, Inc.) was six or larger. In an aspect, the N/P ratio is 1 to 12, and in an aspect, the N/P ratio is 6 to 12. In an aspect, the N/P ratio is 6 or 12, and in an aspect, the N/P ratio is 6.

"Nucleic acid" are deoxyribonucleic acid (DNA) and ribonucleic acid (RNA), and examples of the nucleic acid include messenger RNA (mRNA), microRNA (miRNA), small interfering RNA (siRNA), small hairpin RNA (shRNA), ribozymes, and antisense oligonucleotides. In an aspect, the nucleic acid is mRNA. In an aspect, the nucleic acid is siRNA. In an aspect, the nucleic acid is mRNA of Ascl1 (mRNA encoding Ascl1 protein is also called Ascl mRNA, and mRNA encoding xxx protein is also called xxx mRNA below), Dlx2 mRNA, NeuroD1 mRNA (mRNA encoding NeuroD1 protein), Ngn2 mRNA, BDNF mRNA, NGF mRNA, NT-3 mRNA, or the like.

In an aspect, the nucleic acid is NeuroD1 mRNA. In an aspect, the nucleic acid is Ascl1 mRNA. In an aspect, the nucleic acid is NeuroD1 mRNA containing a base sequence encoding a protein having the amino acid sequence of SEQ ID NO: 2. In an aspect, the nucleic acid is NeuroD1 mRNA containing the base sequence of SEQ ID NO: 1. In an aspect, the nucleic acid is NeuroD1 mRNA containing the base sequence of SEQ ID NO: 3. In an aspect, the nucleic acid is NeuroD1 mRNA containing the base sequence of SEQ ID NO: 4. In an aspect, the nucleic acid is NeuroD1 mRNA containing the base sequence of SEQ ID NO: 5. In an aspect, the nucleic acid is mRNA encoding NeuroD1 protein containing a sequence for cap addition. In an aspect, the nucleic acid is mRNA encoding NeuroD1 protein in which the 5' terminal sequence is a 5' terminal sequence suitable for the capping method using CleanCap (registered trademark), Reagent AG (TriLink BioTechnologies). In an aspect, the nucleic acid is mRNA encoding NeuroD1 protein in which the 5' terminal sequence is AGG. In an aspect, the nucleic acid is mRNA encoding NeuroD1 protein in which the 5' terminal sequence is (position 1 to position 3 of SEQ ID NO: 4). In an aspect, the mRNA is BDNF mRNA. In an aspect, two or more kinds of nucleic acid are contained in the lipid nanoparticle, and in an aspect, one kind of nucleic acid is contained in the lipid nanoparticle. The lipid nanoparticle encapsulating the nucleic acid contains, based on the total weight of the lipid nanoparticle, the nucleic acid in an amount of 0.001 to 60 weight% in an aspect, 0.1 to 40 weight% in an aspect, or 1.0 to 25 weight% in an aspect or contains 3.0 to 10 weight% of the nucleic acid in an aspect. The mRNA may be a natural nucleic acid or an artificial nucleic acid (for example, a nucleic acid containing natural nucleic acid bases and/or artificial nucleic acid bases).

In this specification, the "mRNA encoding xxx protein" means RNA which contains a polynucleotide containing a base sequence encoding xxx protein and which can be translated into xxx protein. In this specification, mRNA encoding xxx protein is also called xxx mRNA. That is, a polynucleotide encoding xxx protein in an expressible state is included. In an aspect, the mRNA encoding xxx protein in the invention is a polynucleotide encoding xxx protein containing functional sequences for expressing xxx protein (for example, a CDS, a 5' UTR, a 3' UTR, a 5' cap structure, and a poly(A) sequence are included, but the sequences are not limited thereto).

The polynucleotide in this specification is sometimes described as a base sequence containing "T's" as a typical DNA sequence, but for example, in RNA (for example, mRNA) containing a base sequence specified by a specific SEQ ID NO:, when the base sequence specified by the specific SEQ ID NO: represents a DNA sequence, the base sequence is understood as an RNA sequence in which the "T's" in the DNA sequence are replaced with "U's". The bases "adenine (A)", "thymine (T)", "guanine (G)", "cytosine (C)", and "uracil (U)" composing a base sequence (for example, the base sequences of SEQ ID NOs: 1 and 3) and nucleosides and nucleotides containing the bases may be of a natural type or a modified type for each base, each nucleoside, and each nucleotide and/or have another modification (methylation or the like), unless otherwise specified in the sequence listing.

The "mRNA" is a messenger ribonucleic acid, is translated into a desired protein and contains a 5' cap, a 5' untranslated region (an untranslated region is also called UTR below), a coding region (CDS), a 3' UTR, and a poly(A) tail in the structure.

The "5' cap" is a modified structure found at the 5' terminal and is involved in stability of mature mRNA, initiation of translation, and the like. A structure in which 7-methylguanosine (also called m⁷G) and mRNA are linked at 5' and 5' through triphosphoric acid (also called ppp) is called Cap-0. A structure in which position 2' of the ribose in the first nucleoside of mRNA is methylated in addition to the Cap-0 structure is called Cap-1, and a structure in which position 2' of the riboses of the first and second nucleosides are methylated is called Cap-2. Cap-0, Cap-1, and Cap-2 are sometimes called m⁷GpppNp, m⁷GpppN₁mp, and m⁷GpppN₁mpN₂mp, respectively (N₁ and N₂ each represent a nucleoside, and m represents a 2'-O methyl group) (Nature Reviews Molecular Cell Biology 2014, vol.15(5), p.313-326). In an aspect, the 5' cap is Cap-0, Cap-1, or Cap-2. In an aspect, the 5' cap is Cap-0. In an aspect, the 5' cap is Cap-1. In an aspect, the 5' cap is Cap-2.

The "UTRs" are untranslated regions and include a 5' UTR and a 3' UTR. The 5' UTR and the 3' UTR may be derived from a gene to be expressed or derived from a heterologous gene. The UTRs may be of a natural type or a modified type having a sequence of a natural-type UTR which has been changed by insertion, deletion, substitution, and/or addition of one or some (for example, two, three, four, five, or six) nucleotides. The "UTR derived from a gene" in this specification includes not only a natural-type UTR but also such a modified-type UTR. In an UTR, more than one UTR may be linked directly or through a spacer sequence. The start codon and the 5' UTR may contain a part of the Kozak sequence (for example, the sequence in the Kozak sequence at the 5' side from the start codon). In an aspect, the UTRs are globin gene-derived UTRs. In an aspect, the UTRs are α-globin gene-derived UTRs. In an aspect, the UTRs are human α-globin gene-derived UTRs. In an aspect, the UTRs are β-globin gene-derived UTRs. In an aspect, the UTRs are human β-globin gene-derived UTRs. In an aspect, the 5' UTR and the 3' UTR are human α-globin gene-derived 5' UTR and 3' UTR.

The "CDS" is a coding sequence and means a DNA sequence region translated into a protein, and the codons may be optimized. The stop codon at the 3' terminal of the CDS may be any stop codon of TAA, TGA, and TAG, and more than one stop codon may be used in succession (for example, TAATGATAG).

A "modified nucleotide" is a modified nucleotide. For example, the modified nucleotide is a nucleotide modified by methylation, atom exchange, saturation of a double bond, deamination, replacement of an oxygen atom or the like with a sulfur atom, or the like. In an aspect, the modified nucleotide is a nucleotide in which the nucleic acid base is modified. In an aspect, the modified nucleotide is a nucleotide in which the ribose is modified. In an aspect, the modified nucleotide is a nucleotide in which the phosphate group is modified. In an aspect, the modified nucleotide is a nucleotide containing 1-methyladenosine, pseudouridine, N1-methylpseudouridine (also called 1-methylpseudouridine), dihydrouridine, 5-methoxycytidine, 5-methylcytidine, 7-methylguanosine, N6-methyladenosine, inosine, or thiouridine. In an aspect, the modified nucleotide is a nucleotide containing 1-methyladenosine. In an aspect, the modified nucleotide is a nucleotide containing modified uridine. In an aspect, the modified nucleotide is a nucleotide containing pseudouridine or N1-methylpseudouridine. In an aspect, the modified nucleotide is a nucleotide containing pseudouridine. In an aspect, the modified nucleotide is a nucleotide containing N1-methylpseudouridine. In an aspect, the modified nucleotide is a nucleotide containing dihydrouridine. In an aspect, the modified nucleotide is a nucleotide containing inosine. In an aspect, the modified nucleotide is a nucleotide containing 4-thiouridine. Here, in this specification, the modified nucleotides with "a part or all" can include those in which some of the predetermined modified nucleotides are not substituted in the entire modified nucleotide sequence. The percentage of the residues substituted to modified nucleotides from the specific nucleotides in the entire sequence may be 1 to 100%, 1 to 90%, 1 to 80%, 1 to 70%, 1 to 60%, 1 to 50%, 1 to 40%, 1 to 30%, 1 to 20%, 1 to 10%, 1 to 5%, 75 to 99%, 50 to 75%, 25 to 50%, or 10 to 25% but is not limited thereto. In an aspect, "a part" as the percentage of the residues substituted to modified nucleotides of all the residues may be, for example, 60 to 99%, 70 to 99%, 80 to 99%, 90 to 99%, 95 to 99%, 60 to 99.9%, 70 to 99.9%, 80 to 99.9%, 90 to 99.9%, or 95 to 99.9% but is not limited thereto.

The "poly(A) tail" is a polyadenylic acid chain having the function of stabilization of mRNA, nuclear export, translation, or the like and is an mRNA region at the downstream of the 3'UTR, for example, direct downstream (namely, at the 3' side) containing successive (typically 10 or more although the number is not limited thereto) adenosine monophosphates. The poly(A) tail is also called a poly(A) sequence. In an aspect, the poly(A) tail length is 20 to 1000 bases. In an aspect, the poly(A) tail length is 30 to 500 bases, 50 to 200 bases, 60 to 150 bases, 70 to 130 bases, 70 to 120 bases, 70 to 80 bases, 120 bases, or 79 bases. In an aspect, the poly(A) tail length is 120 bases. In an aspect, the poly(A) tail length is 79 bases. In an aspect, the poly(A) tail length is 30 to 500 bases (in an aspect, 40 to 200, 50 to 200, 50 to 150, 50 to 100, 50 to 90, 60 to 150, 60 to 100, 60 to 90, 70 to 130, 70 to 120, 70 to 100, 70 to 90, 70 to 85, 70 to 80, 75 to 130, 75 to 120, 75 to 100, or 75 to 90, in an aspect, 74 to 84, 75 to 85, 75 to 83, or 76 to 82 bases). In an aspect, the poly(A) tail length is 120 bases. In an aspect, the poly(A) tail length is 79 bases. In an aspect, the poly(A) tail length is 74 to 84 bases. In an aspect, the poly(A) tail length is 75 to 85 bases.

In this specification, the "expression" of a nucleic acid, namely a nucleic acid sequence, means translation of mRNA into a polypeptide, formation of a protein from a polypeptide, or posttranslational modification of a polypeptide or a protein. Moreover, in this specification, expression and production, such as expression of a protein, are used with the same meanings.

"NeuroD1" is a neurogenic differentiation protein (neuronal differentiation 1) and is a basic helix-loop-helix (bHLH) transcription factor belonging to the NeuroD family. NeuroD1 protein activates transcription of a gene containing a specific DNA sequence known as E-box (also called transcription factor activity). NeuroD1 has a central function of inducing differentiation from neural stem cells into neurons. Glial cells are classified into four cell types of astrocytes, oligodendrocytes, ependymal cells, and microglia. Ectopic expression of NeuroD1 in glial cells such as NG2 cells and astrocytes is known to be able to convert glial cells into the nerve (WO2014/015261). In an aspect, NeuroD1 is a protein which has an amino acid sequence having a sequence identity of 90% or more (for example, 95% or more, 98% or more, 99% or more, 99.4% or more, or 99.5% or more) to the amino acid sequence of SEQ ID NO: 2 and which has transcription factor activity. In an aspect, NeuroD1 is a protein which has an amino acid sequence having insertion, deletion, substitution, and/or addition of one to 50 (for example, one or two, one to three, one to five, one to seven, one to 10, or one to 30) amino acids in the amino acid sequence of SEQ ID NO: 2 and which has transcription factor activity. In an aspect, NeuroD1 is a protein having the amino acid sequence of SEQ ID NO: 2.

The "transcription factor activity" means the ability of a protein to activate transcription of a related gene (namely, the ability of activating transcription). Whether a protein has transcription factor activity or not can be determined specifically, for example, by observing whether the expression level of the related gene is increased or decreased by a recombinant vector compared to that in cells without introduction of the vector. The recombinant vector can be produced by inserting a polynucleotide encoding the protein into a vector having any promotor, and the protein can be expressed by introducing the produced recombinant vector into cells. For example, when the expression of the related gene is increased in cells into which a vector including a polynucleotide encoding NeuroD1 has been introduced, it can be determined that NeuroD1 protein has transcription factor activity.

The "sequence identity" means the percentage (%) of the matching residues between sequences when a reference biological sequence (a base sequence, an amino acid sequence, or the like) and a target biological sequence are aligned (generally, the percentage % of the matching residues based on the entire length of the target sequence). The "sequence identity" can be calculated, for example, as an identity value obtained using EMBOSS Needle (Nucleic Acids Res., 2015; Vol.43: pW580-W584) using the parameters provided as default. The parameters are as follows (Gap Open Penalty = 10, Gap Extend Penalty = 0.5, Matrix = EBLOSUM62, End Gap Penalty = false).

"Penetrating branch infarction" is a disease in which infarction is caused in the thalamus, the caudate nucleus, the putamen, the globus pallidus, and/or the internal capsule, which are controlled by the penetrating branch, and in which the function at the infarction site is damaged. The "penetrating branch" are fine arteries branched from the middle cerebral artery constituting the major cerebral artery and control the basal ganglia/thalamus area and the internal capsule. Here, that a brain area is "controlled" by a vessel means that blood is supplied to the brain area by the vessel, and the brain area is in the vascular territory of the vessel.

The "penetrating branch territory " means the area controlled by penetrating branch and means the basal ganglia/thalamus area and the internal capsule.

"Cerebral infarction having brain damage in the penetrating branch territory " means cerebral infarction in which brain damage caused by infarction is mainly caused in the penetrating branch territory. Cerebral infarction having brain damage in the penetrating branch territory includes not only cerebral infarction in which brain damage is caused in the penetrating branch territory due to infarction caused in the penetrating branch territory but also cerebral infarction in which brain damage is secondarily caused in the penetrating branch territory, for example, as in the case in which cell necrosis caused by infarction caused around the penetrating branch territory spreads to the penetrating branch territory to damage the function of the site. The brain damage in this specification means functional damage in the brain due to cell necrosis caused by infarction. The brain damage generally has cell necrosis in the area having the brain damage. "Cerebral infarction having brain damage in the penetrating branch territory " may be cerebral infarction having cell necrosis in the penetrating branch territory.

Aspects of the compound of the formula (I), which is the compound of the invention, or a salt thereof, a lipid nanoparticle containing the compound or the salt, and a pharmaceutical composition are shown below. In this regard, all the aspects can be freely combined when the combinations are combinations of any compatible two or more. Even when the combination is not specifically described, one aspect or two or more aspects can be combined with another aspect.

### 1. Cationic Lipid

Aspects of the compound of the formula (I), which is the compound of the invention, or a salt thereof are shown below. Aspects of the compound are shown below. "The compound or the salt thereof" is referred to as "the compound".

### (Aspects of Cationic Lipid of Invention)

(1) The compound of the formula (I) in which L¹ is -CH₂-. The compound of the formula (I) in which L¹ is a bond.
(2) The compound of the formula (I) in which L² is -CH₂-. The compound of the formula (I) in which L² is a bond. The compound of the formula (I) in which L² is -CH₂- or a bond.
(3) The compound of the formula (I) in which L³ is a C₆₋₉ alkylene. The compound of the formula (I) in which L³ is a C₆ alkylene.
(4) The compound of the formula (I) in which M is absent, and n is 1 or 2. The compound of the formula (I) in which M is absent, and n is 1. The compound of the formula (I) in which M is absent, and n is 2. The compound of the formula (I) in which M is -CH₂-, and n is 1. The compound of the formula (I) in which M is -CH₂- or absent, and n is 1.
(5) The compound of the formula (I) in which E¹ is -C(=O)O-* or -OC(=O)- *, and * indicates that the group binds to R¹ at the position. The compound of the formula (I) in which E¹ is -OC(=O)-* or -OC(=O)O-*, and * indicates that the group binds to R¹ at the position. The compound of the formula (I) in which E¹ is -OC(=O)O-* or -C(=O)O-*, and * indicates that the group binds to R¹ at the position. The compound of the formula (I) in which E¹ is -C(=O)O-*, and * indicates that the group binds to R¹ at the position. The compound of the formula (I) in which E¹ is -OC(=O)- *, and * indicates that the group binds to R¹ at the position. The compound of the formula (I) in which E¹ is -OC(=O)O-*, and * indicates that the group binds to R¹ at the position. The compound of the formula (I) in which E¹ and E² are the same or different and are -C(=O)O-*, -OC(=O)-*, or -OC(=O)O-*, and * indicates that the group binds to R¹ or R² at the position, wherein one of E¹ and E² is -C(=O)O-*.
(6) The compound of the formula (I) in which E² is -C(=O)O-* or -OC(=O)-*, and * indicates that the group binds to R² at the position. The compound of the formula (I) in which E² is -OC(=O)-* or -OC(=O)O-*, and * indicates that the group binds to R² at the position. The compound of the formula (I) in which E² is -OC(=O)O-* or -C(=O)O-*, and * indicates that the group binds to R² at the position. The compound of the formula (I) in which E² is - C(=O)O-*, and * indicates that the group binds to R² at the position. The compound of the formula (I) in which E² is -OC(=O)O-*, and * indicates that the group binds to R² at the position. The compound of the formula (I) in which E² is -OC(=O)O-*, and * indicates that the group binds to R² at the position.
(7) The compound of the formula (I) in which R¹ is -CH₂CH(-R^{x})R^{y} or -CH₂-(C₅₋₁₅ alkenyl). The compound of the formula (I) in which R¹ is -CH₂CH(-R^{x})R^{y}. The compound of the formula (I) in which R¹ is -CH₂CH(-OR^{x})OR^{y}. The compound of the formula (I) in which R¹ is -CH₂-(C₅₋₁₅ alkyl). The compound of the formula (I) in which R¹ is -CH₂-(C₅₋₁₅ alkenyl). The compound of the formula (I) in which R¹ is -CH₂CH(-R^{x})R^{y}. The compound of the formula (I) in which R¹ and R² are the same or different and are -CH₂CH(-R^{x})R^{y}, -CH₂CH(-OR^{x})OR^{y}, -CH₂CH₂CH(-OR^{x})OR^{y}, -CH₂-(C₅₋₁₅ alkyl), or -CH₂-(C₅₋₂₀ alkenyl).
(8) The compound of the formula (I) in which R² is -CH₂CH(-R^{x})R^{y} or -CH₂-(C₅₋₁₅ alkenyl). The compound of the formula (I) in which R² is -CH₂CH(-R^{x})R^{y}, -CH₂CH(-OR^{x})OR^{y}, -CH₂-(C₅₋₁₅ alkyl), or -CH₂-(C₅₋₁₅ alkenyl). The compound of the formula (I) in which R² is -CH₂CH(-R^{x})R^{y}. The compound of the formula (I) in which R² is -CH₂CH(-OR^{x})OR^{y}. The compound of the formula (I) in which R² is -CH₂-(C₅₋₁₅ alkyl). The compound of the formula (I) in which R² is -CH₂-(C₅₋₁₅ alkenyl).
(9) The compound of the formula (I) in which one of -E¹-R¹ and -E²-R² is -C(=O)O-CH₂CH(-R^{x})R^{y}.

The compound of the formula (I) in which -E¹-R¹ is -C(=O)O-CH₂CH(-R^{x})R^{y}, - OC(=O)O-CH₂CH(-R^{x})R^{y}, -OC(=O)-CH₂CH(-OR^{x})OR^{y}, or -OC(=O)-(C₅₋₁₅ alkenyl). The compound of the formula (I) in which -E¹-R¹ is -C(=O)O-CH₂CH(-R^{x})R^{y}. The compound of the formula (I) in which -E¹-R¹ is -C(=O)O-CH₂CH(-R^{x})R^{y}, -C(=O)O-CH₂CH₂CH(-R^{x})R^{y}, - OC(=O)-C₃H₆-(C₁₋₆ alkylene)-CH=CH-CH=CH-(C₁₋₆ alkyl), -OC(=O)-N(-R^{x})R^{y}, -OC(=O)O-CH(-R^{x})R^{y}, -OC(=O)O-CH₂CH(-R^{x})R^{y}, -NR^{y}(-C(=O)R^{x}), or -NR^{y}C(=O)CH(-R^{x})R^{Z}. The compound of the formula (I) in which -E¹-R¹ is -C(=O)O-CH₂CH(-R^{x})R^{y}, -OC(=O)-C₃H₆-(C₁₋₆ alkylene)-CH=CH-CH=CH-(C₁₋₆ alkyl), or -OC(=O)O-CH₂CH(-R^{x})R^{y}. The compound of the formula (I) in which -E¹-R¹ is -C(=O)O-CH₂CH(-R^{x})R^{y}, -C(=O)O-CH₂CH₂CH(-R^{x})R^{y}, - OC(=O)O-CH(-R^{x})R^{y}, or -OC(=O)O-CH₂CH(-R^{x})R^{y}. The compound of the formula (I) in which -E¹-R¹ is -C(=O)O-CH₂CH(-R^{x})R^{y} or -OC(=O)O-CH₂CH(-R^{x})R^{y}.

(10) The compound of the formula (I) in which the other of -E¹-R¹ and -E²-R² is - C(=O)O-CH₂CH(-R^{x})R^{y}, -OC(=O)O-CH₂CH(-R^{x})R^{y}, -OC(=O)-CH₂CH(-OR^{x})OR^{y}, or - OC(=O)-CH₂-(C₅₋₁₅ alkenyl). The compound of the formula (I) in which -E²-R² is -C(=O)O-CH₂CH(-R^{x})R^{y}, -OC(=O)O-CH₂CH(-R^{x})R^{y}, -OC(=O)-CH₂CH(-OR^{x})OR^{y}, or -OC(=O)-CH₂-(C₅₋₁₅ alkenyl). The compound of the formula (I) in which -E²-R² is -C(=O)O-CH₂CH(-R^{x})R^{y}. The compound of the formula (I) in which -E²-R² is -C(=O)O-CH₂CH(-R^{x})R^{y}, -C(=O)O-CH₂CH₂CH(-R^{x})R^{y}, -C(=O)O-C₃H₆-(C₁₋₆ alkylene)-CH=CH-CH=CH-(C₁₋₆ alkylene)-, - OC(=O)-CH₂CH(-R^{x})R^{y}, -OC(=O)-CH₂CH₂CH(-R^{x})R^{y}, -OC(=O)-CH₂CH₂CH(-OR^{x})OR^{y}, - OC(=O)-C₃H₆-(C₁₋₆ alkylene)-CH=CH-CH=CH-(C₁₋₆ alkylene)-, or -OC(=O)O-CH₂CH(-R^{x})R^{y}. The compound of the formula (I) in which -E²-R² is -C(=O)O-CH₂CH(-R^{x})R^{y}, - C(=O)O-C₃H₆-(C₁₋₆ alkylene)-CH=CH-CH=CH-(C₁₋₆ alkylene)-, or -OC(=O)-C₃H₆-(C₁₋₆ alkylene)-CH=CH-CH=CH-(C₁₋₆ alkylene)-. The compound of the formula (I) in which -E²-R² is -C(=O)O-CH₂CH(-R^{x})R^{y}, -C(=O)O-CH₂CH₂CH(-R^{x})R^{y}, -OC(=O)-CH₂CH₂CH(-R^{x})R^{y}, or -OC(=O)-CH₂CH₂CH(-OR^{x})OR^{y}. The compound of the formula (I) in which -E²-R² is - C(=O)O-CH₂CH(-R^{x})R^{y}.

(11) The compound of the formula (I) in which R^{x} is a C₅₋₁₀ alkyl. The compound of the formula (I) in which R^{x} is a C₇₋₉ alkyl. The compound of the formula (I) in which R^{x} is a C₇ alkyl. The compound of the formula (I) in which R^{x} is a C₈ alkyl. The compound of the formula (I) in which R^{x} is a C₉ alkyl. The compound of the formula (I) in which R^{x} and R^{y} are the same or different and are a C₅₋₁₅ alkyl.

(12) The compound of the formula (I) in which R^{y} is a C₅₋₁₀ alkyl. The compound of the formula (I) in which R^{y} is a C₇₋₉ alkyl. The compound of the formula (I) in which R^{y} is a C₇ alkyl. The compound of the formula (I) in which R^{y} is a C₈ alkyl. The compound of the formula (I) in which R^{y} is a C₉ alkyl.

(13) The compound of the formula (I) in which R³ is a group selected from the group consisting of the formulae (d), (e), (g), and (h) of the formulae below.

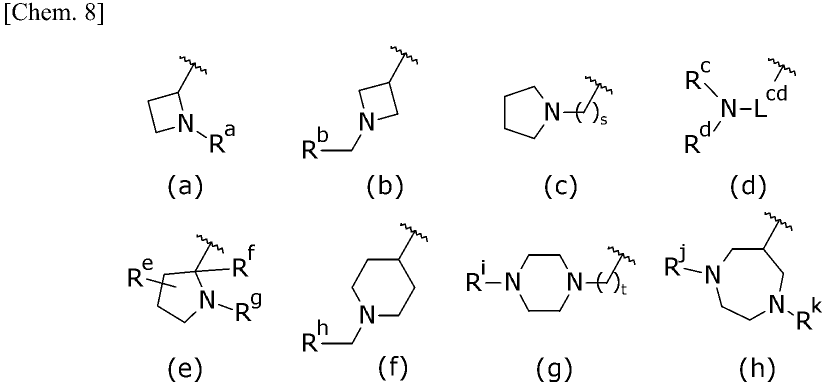

The compound of the formula (I) in which R³ is a group selected from the group consisting of the formulae (e), (g), and (h). The compound of the formula (I) in which R³ is the formula (d). The compound of the formula (I) in which R³ is the formula (e). The compound of the formula (I) in which R³ is the formula (g). The compound of the formula (I) in which R³ is the formula (h). The compound of the formula (I) in which R³ is a group selected from the group consisting of the formulae (d), (e), (g), and (h).

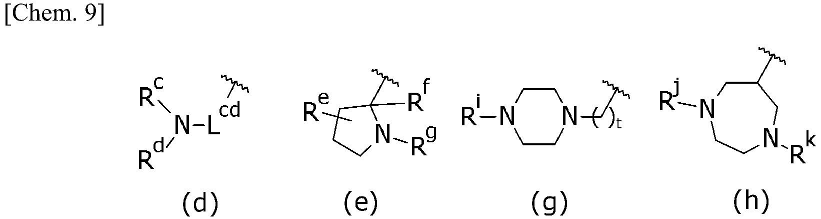

The compound of the formula (I) in which R³ is a group selected from the group consisting of the formulae (e), (g), and (h). The compound of the formula (I) in which R³ is the formula (d).

(14) The compound of the formula (I) in which R^{a} is -CH₃.

(15) The compound of the formula (I) in which R^{b} is -CH₂-C₁₋₆ alkyl. The compound of the formula (I) in which R^{b} is -C(=O)CH₂N(CH₃)₂.

(16) The compound of the formula (I) in which R^{c} and R^{d} are both -CH₃, and L^{cd} is - CH₂-. The compound of the formula (I) in which R^{c} and R^{d} are both -CH₂CH₃, and L^{cd} is - CH₂CH₂-. The compound of the formula (I) in which R^{c} and R^{d} are both -CH₂CH₂OH, and L^{cd} is -CH₂CH₂-. The compound of the formula (I) in which R^{c} is H, and R^{d} is -CH₂C(=O)NH₂. The compound of the formula (I) in which R^{c} and R^{d} are both -CH₃, -CH₂CH₃, or - CH₂CH₂OH, or R^{d} is -CH₂C(=O)NH₂ when R^{c} is H, and L^{cd} is -CH₂- or -CH₂CH₂-, wherein when R^{c} and R^{d} are both -CH₃, -CH₂CH₃, or -CH₂CH₂OH, L^{cd} is -CH₂-, -CH₂CH₂-, or - CH₂CH₂-.

(17) The compound of the formula (I) in which R^{e} is H. The compound of the formula (I) in which R^{e} is OH.

(18) The compound of the formula (I) in which R^{f} is H.

(19) The compound of the formula (I) in which R^{g} is -CH₃. The compound of the formula (I) in which R^{f} and R^{g} are a pyrrolidine ring together with the carbon atom and the nitrogen atom to which they are bonded. The compound of the formula (I) in which R^{g} is a C₁₋₆ alkyl.

(20) The compound of the formula (I) in which R^{h} is -CH₂CH₃.

(21) The compound of the formula (I) in which Rⁱ is -CH₃. The compound of the formula (I) in which Rⁱ is a C₁₋₆ alkyl.

(22) The compound of the formula (I) in which R^{j} and R^{k} are both -CH₂CH₃. The compound of the formula (I) in which R^{j} and R^{k} are the same or different and are a C₁₋₆ alkyl.

(23) The compound of the formula (I) in which s is 1. The compound of the formula (I) in which s is 2.

(24) The compound of the formula (I) in which t is 1.

(25) The compound of the formula (I) which is a combination of compatible groups described in (1) to (24) above.

Examples of the combinations of the above aspects are specifically the compounds below or salts thereof.

The compound of the formula (I) in which L¹ is a bond, L² is -CH₂-, L³ is a C₆ alkylene, M is absent, n is 1, E¹ is -C(=O)O-*, E² is -C(=O)O-*, R¹ is -CH₂CH(-R^{x})R^{y}, R² is - CH₂CH(-R^{x})R^{y}, R^{x} and R^{y} are a C₈ alkyl, R³ is the formula (e), R^{e} is H, R^{f} is H, and R^{g} is - CH₃.

The compound of the formula (I) in which L¹ is a bond, L² is -CH₂-, L³ is a C₆ alkylene, M is absent, n is 1, -E¹-R¹ is -C(=O)O-CH₂CH(-R^{x})R^{y}, R^{x} and R^{y} are a C₈ alkyl, R³ is the formula (e), R^{e} is H, R^{f} is H, and R^{g} is -CH₃.

Aspects of the specific compounds of the invention are the compounds below or the salts thereof.

A compound or a salt thereof in which the compound is
2-nonylundecyl 8-{(1-methyl-L-prolyl)[(1*r*,3*S*)-3-{2-[(2-octyldecyl)oxy]-2-oxoethyl}cyclobutyl]amino}octanoate,
2-nonylundecyl 8-[(1-methyl-L-prolyl)(3-{2-[(2-octyldecyl)oxy]-2-oxoethyl}bicyclo[1.1.1]pentan-1-yl)amino]octanoate,
2-nonylundecyl 8-{(1-ethyl-L-prolyl)[(1*r*,3*S*)-3-{2-[(2-octyldecyl)oxy]-2-oxoethyl}cyclobutyl]amino}octanoate,
2-nonylundecyl 8-{(*N,N*-diethyl-β-alanyl)[(1*r*,3*r*)-3-{2-[(2-octyldecyl)oxy]-2-oxoethyl}cyclobutyl]amino}octanoate,
2-nonylundecyl 8-{(1,4-diethyl-1,4-diazepane-6-carbonyl)[(1*r*,3*r*)-3-{2-[(2-octyldecyl)oxy]-2-oxoethyl}cyclobutyl]amino}octanoate,
2-nonylundecyl 8-{[(4-methylpiperazin-1-yl)acetyl][(1*r*,3*r*)-3-{2-[(2-octyldecyl)oxy]-2-oxoethyl}cyclobutyl]amino}octanoate,
2-nonylundecyl 8-[(1-methyl-L-prolyl){(1*r*,3*S*)-3-[({[(2-octyldecyl)oxy]carbonyl}oxy)methyl]cyclobutyl}amino]octanoate,
2-heptylnonyl (1*S*,4*r*)-4-[{8-[(2-heptylnonyl)oxy]-8-oxooctyl}(1-methyl-L-prolyl)amino]cyclohexane-1-carboxylate,
2-nonylundecyl 8-{(1-ethyl-D-prolyl)[(1*r*,3*R*)-3-{2-[(2-octyldecyl)oxy]-2-oxoethyl}cyclobutyl]amino}octanoate,
3-decyltridecyl 8-{[(1*r*,3*S*)-3-{2-[(3-decyltridecyl)oxy]-2-oxoethyl}cyclobutyl](1-methyl-L-prolyl)amino}octanoate,
2-{(1-ethyl-L-prolyl)[(1*r*,3*S*)-3-{2-[(2-octyldecyl)oxy]-2-oxoethyl}cyclobutyl]amino}ethyl 4,4-bis(octyloxy)butanoate, or
4-[(1-ethyl-L-prolyl){(1*r*,3*S*)-3-[({[(2-nonylundecyl)oxy]carbonyl}oxy)methyl]cyclobutyl}amino]butyl 4-octyldodecanoate.

Aspects of the specific compounds of the invention are the compounds below or salts thereof.

A compound or a salt thereof in which the compound is
2-nonylundecyl 8-{(1-ethyl-L-prolyl)[(1*r*,3*S*)-3-{2-[(2-octyldecyl)oxy]-2-oxoethyl}cyclobutyl]amino}octanoate or
2-nonylundecyl 8-{(*N,N*-diethyl-β-alanyl)[(1*r*,3*r*)-3-{2-[(2-octyldecyl)oxy]-2-oxoethyl}cyclobutyl]amino}octanoate.

In this specification, the compound of the formula (I) or a salt thereof may be described only as one of isomers, but the invention includes isomers other than that and also includes separated isomers or mixtures thereof.

The compound of the formula (I) may have tautomers or geometrical isomers depending on the type of the substituent. In this specification, the compound of the formula (I) may be described only as one of isomers, but the invention includes isomers other than that and also includes separated isomers or mixtures thereof. In this specification, when it is indicated that "all the double bonds have *Z* configuration", the double bonds have (*Z*) configuration, namely *cis* configuration. Moreover, when it is indicated that "all the double bonds have *E* configuration", the double bonds have (*E*) configuration, namely *trans* configuration.

In addition, the compound of the formula (I) or a salt thereof may have an asymmetric center or an axial chirality and may have enantiomers (optical isomers) based thereon. The compound of the formula (I) or a salt thereof includes both isolated enantiomers such as (*R*)-form and (*S*)-form and mixtures thereof (including a racemic mixture or a non-racemic mixture). In an aspect, the enantiomer is "stereochemically pure". The "stereochemically pure" means the purity to a degree that one skilled in the art can recognize substantially stereochemically pure. In an aspect, the enantiomer is, for example, a compound having a stereochemical purity of 90% ee (enantiomeric excess) or more, 95% ee or more, 98% ee or more, or 99% ee or more.

The salt of the compound of the formula (I) is a pharmaceutically acceptable salt and may form an acid addition salt depending on the type of the substituent. Specific examples include an acid addition salt with an inorganic acid, such as hydrochloric acid, hydrobromic acid, hydroiodic acid, sulfuric acid, nitric acid, and phosphoric acid, or with an organic acid, such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid, and the like.

Furthermore, the invention also includes various hydrates, solvates, and crystal polymorphism substances of the compound of the formula (I) or a salt thereof.

The invention also includes the compound of the formula (I) or a salt thereof which is pharmaceutically acceptable and labeled with one or more radioactive or nonradioactive isotopes. Examples of preferable isotopes used for isotope labeling of the compound of the invention include isotopes of hydrogen (²H, ³H, and the like), carbon (¹¹C, ¹³C, ¹⁴C, and the like), nitrogen (¹³N, ¹⁵N, and the like), oxygen (¹⁵O, ¹⁷O, ¹⁸O, and the like), fluorine (¹⁸F and the like), chlorine (³⁶Cl and the like), iodine (¹²³I, ¹²⁵I, and the like), phosphorus (³²P and the like), and sulfur (³⁵S and the like). The isotopically labeled compound of the invention of the present application can be used for studies such as histological distribution study of drugs and/or substrates and the like. For example, a radioisotope such as tritium (³H) and carbon-14 (¹⁴C) can be used for the purpose due to easiness of labeling and convenience of detection. Replacement with a heavier isotope, for example replacement of hydrogen with deuterium (²H), is sometimes therapeutically advantageous because metabolic stability improves (for example, increased *in vivo* half-life, decreased required dose, and declined drug interaction). Replacement with a positron-emitting isotope (¹¹C, ¹⁸F, ¹⁵O, ¹³N, or the like) can be used for positron emission tomography (PET) for testing the substrate acceptor occupancy. The isotopically labeled compound of the invention can be generally produced by a conventional method known to one skilled in the art or by a production method similar to those in Examples or Production Examples or the like using an appropriate isotopically labeled reagent instead of an unlabeled reagent.

### 2. Lipid Nanoparticle

Aspects of the lipid nanoparticle containing the compound of the formula (I) or the salt thereof of the invention are shown below. In this regard, "the compound of the formula (I) or the salt thereof" in the aspects below include the aspects of "the compound of the formula (I) or the salt thereof" described in (Aspects of Cationic Lipid of Invention) above.

### (Aspects of Lipid Nanoparticle of Invention)

(1) A lipid nanoparticle containing the compound of the formula (I) or the salt thereof.
(2) A lipid nanoparticle containing the compound of the formula (I) or the salt thereof, a neutral lipid, and a PEGylated lipid.
(3) The lipid nanoparticle described in (2) which encapsulates a nucleic acid.
(4) The lipid nanoparticle described in (2) or (3) in which the neutral lipid is a phospholipid and a sterol. The lipid nanoparticle described in (2) or (3) in which the neutral lipid is DSPC and cholesterol.
(4a) The lipid nanoparticle described in (4) in which the phospholipid is DSPC.
(4b) The lipid nanoparticle described in (4) in which the sterol is cholesterol.
(5) The lipid nanoparticle described in (3) in which the PEGylated lipid is DMG-PEG2000.
(6) A lipid nanoparticle in which the composition ratios of the components are the mol% shown in (6a) to (6e) based on the total amount of the lipid nanoparticle.
(6a) The lipid nanoparticle described in (3) in which the composition ratio of the compound of the formula (I) or the salt thereof is 20.0 to 80.0 mol% based on the total amount of the lipid nanoparticle. A lipid nanoparticle containing the compound of the formula (I) or a salt thereof in which the composition ratio of the compound of the formula (I) or the salt thereof is 30.0 to 60.0 mol% based on the total amount of the lipid nanoparticle.
(6b) The lipid nanoparticle described in (3) in which the composition ratio of the neutral lipid is 18.5 to 78.5 mol% based on the total amount of the lipid nanoparticle. The lipid nanoparticle described in (5) in which the composition ratio of the neutral lipid is 38.5 to 68.5 mol% based on the total amount of the lipid nanoparticle.
(6c) The lipid nanoparticle described in (6b) in which the composition ratio of the phospholipid is 4.0 to 18.1 mol% based on the total amount of the lipid nanoparticle. The lipid nanoparticle described in (6b) in which the composition ratio of the phospholipid is 5.0 to 18.1 mol% based on the total amount of the lipid nanoparticle.
(6d) The lipid nanoparticle described in (6b) in which the composition ratio of the sterol is 14.5 to 62.3 mol% based on the total amount of the lipid nanoparticle. The lipid nanoparticle described in (6b) in which the composition ratio of the sterol is 25.5 to 54.4 mol% based on the total amount of the lipid nanoparticle.
(6e) The lipid nanoparticle described in (3) in which the composition ratio of the PEGylated lipid is 0.5 to 2.5 mol% based on the total amount of the lipid nanoparticle. The lipid nanoparticle described in (5) in which the composition ratio of the PEGylated lipid is 0.5 to 2.0 mol% based on the total amount of the lipid nanoparticle.
(7) The lipid nanoparticle described in (3) in which the nucleic acid is mRNA. The lipid nanoparticle described in (3) in which the nucleic acid is a nucleic acid which is useful for preventing and/or treating an astrocyte-related disease. The lipid nanoparticle described in (3) in which the nucleic acid is mRNA which is useful for preventing and/or treating an astrocyte-related disease. The lipid nanoparticle described in (3) in which the nucleic acid is Ascl1 mRNA, Dlx2 mRNA, NeuroD1 mRNA, Ngn2 mRNA, BDNF mRNA, NGF mRNA, or NT-3 mRNA. The lipid nanoparticle described in (3) in which the nucleic acid is BDNF mRNA or NeuroD1 mRNA. The lipid nanoparticle described in (3) in which the nucleic acid is NeuroD1 mRNA.
(8) The lipid nanoparticle described in (7) in which the nucleic acid is NeuroD1 mRNA containing a base sequence encoding a protein having a sequence identity of 90% or more (for example, 95% or more, 98% or more, 99% or more, 99.4% or more, or 99.5% or more) to a protein having the amino acid sequence of SEQ ID NO: 2. The lipid nanoparticle described in (7) in which the nucleic acid is NeuroD1 mRNA containing a base sequence having a sequence identity of 70% or more (for example, 80% or more, 90% or more, or 95% or more) to the base sequence of SEQ ID NO: 1 or 3 and encodes the amino acid sequence of SEQ ID NO: 2. The lipid nanoparticle described in (7) in which the nucleic acid is NeuroD1 mRNA containing a base sequence having a sequence identity of 70% or more (for example, 80% or more, 90% or more, or 95% or more) to the base sequence of SEQ ID NO: 1 and encodes the amino acid sequence of SEQ ID NO: 2. The lipid nanoparticle described in (7) in which the nucleic acid is NeuroD1 mRNA containing a base sequence having a sequence identity of 70% or more (for example, 80% or more, 90% or more, or 95% or more) to the base sequence of SEQ ID NO: 3 and encodes the amino acid sequence of SEQ ID NO: 2.
(8a) The lipid nanoparticle described in (7) in which the nucleic acid is NeuroD1 mRNA containing a base sequence encoding a protein having the amino acid sequence of SEQ ID NO: 2. The lipid nanoparticle described in (7) in which the nucleic acid is NeuroD1 mRNA containing the base sequence of SEQ ID NO: 1. The lipid nanoparticle described in (7) in which the nucleic acid is NeuroD1 mRNA containing the base sequence of SEQ ID NO: 3. The lipid nanoparticle described in (7) in which the nucleic acid is NeuroD1 mRNA containing the base sequence of SEQ ID NO: 4. The lipid nanoparticle described in (7) in which the nucleic acid is NeuroD1 mRNA containing the base sequence of SEQ ID NO: 5. The lipid nanoparticle described in (7) in which the nucleic acid is NeuroD1 mRNA containing a base sequence having a sequence identity of 70% or more (for example, 80% or more, 90% or more, or 95% or more) to the base sequence at positions 44 to 1114 of SEQ ID NO: 4 and encodes the amino acid sequence of SEQ ID NO: 2. The lipid nanoparticle described in (7) in which the nucleic acid is NeuroD1 mRNA containing a base sequence having a sequence identity of 70% or more (for example, 80% or more, 90% or more, or 95% or more) to the base sequence at positions 44 to 1114 of SEQ ID NO: 5 and encodes the amino acid sequence of SEQ ID NO: 2.
(9) The lipid nanoparticle described in (7) in which the nucleic acid is NeuroD1 mRNA.
(9a) The lipid nanoparticle described in (7) in which the nucleic acid is NeuroD1 mRNA having a 5' cap that is Cap-0, Cap-1, or Cap-2. The lipid nanoparticle described in (9) in which the 5' cap is Cap-1.
(9b) The lipid nanoparticle described in (9) in which the 5' UTR contains the Kozak sequence. The lipid nanoparticle described in (9) in which the nucleic acid has a 5' UTR or a 3' UTR that is derived from α-globin gene. The lipid nanoparticle described in (9) in which the nucleic acid has a 5' UTR or a 3' UTR that is derived from human α-globin gene. The lipid nanoparticle described in (9) in which the nucleic acid has a 5' UTR or a 3' UTR that is derived from β-globin gene. The lipid nanoparticle described in (9) in which the 5' UTR or the 3' UTR is derived from human β-globin gene. The lipid nanoparticle described in (9) in which the 5' UTR contains the Kozak sequence. The lipid nanoparticle described in (9) in which the 5' UTR is derived from α-globin gene. The lipid nanoparticle described in (9) in which the 5' UTR is derived from human α-globin gene. The lipid nanoparticle described in (9) in which the 5' UTR is derived from β-globin gene. The lipid nanoparticle described in (9) in which the 5' UTR is derived from human β-globin gene. The lipid nanoparticle described in (9) in which the 3' UTR is derived from human α-globin gene. The lipid nanoparticle described in (9) in which the 3' UTR is derived from human β-globin gene.
(9c) The lipid nanoparticle described in (9) in which the nucleic acid is NeuroD1 mRNA having a poly(A) tail of 20 to 1000 bases. The lipid nanoparticle described in (9) in which the nucleic acid is NeuroD1 mRNA having a poly(A) tail of 30 to 500 bases, 50 to 200 bases, 60 to 150 bases, 70 to 130 bases, 70 to 120 bases, 70 to 80 bases, 120 bases, or 79 bases. The lipid nanoparticle described in (9) in which the nucleic acid is NeuroD1 mRNA having a poly(A) tail of 120 bases. The lipid nanoparticle described in (9) in which the nucleic acid is NeuroD1 mRNA having a poly(A) tail of 79 bases.
(9d) The lipid nanoparticle described in (9) in which the nucleic acid contains a modified nucleotide. The lipid nanoparticle described in (9) in which the nucleic acid contains an N1-methylpseudouridine. The lipid nanoparticle described in (9) in which a part or all of the uridines are N1-methylpseudouridines. The lipid nanoparticle described in (9) in which a part of the uridines have been substituted with N1-methylpseudouridines. The lipid nanoparticle described in (9) in which all the uridines have been substituted with N1-methylpseudouridines.
(10) A lipid nanoparticle of a combination of compatible two or more of (1) to (9) described in (Aspects of Lipid Nanoparticle of Invention) above. A lipid nanoparticle of a compatible combination of an aspect described in (Aspects of Cationic Lipid of Invention) and an aspect described in (Aspects of Lipid Nanoparticle of Invention).

Specific examples of the combinations of (10) above are the aspects below.
(10a) A lipid nanoparticle encapsulating a nucleic acid and containing the compound of the formula (I) or a salt thereof, neutral lipids, and a PEGylated lipid
   in which the nucleic acid is mRNA encoding NeuroD1 protein and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(10b) The lipid nanoparticle described in (8a) in which the nucleic acid is mRNA encoding NeuroD1 protein containing a base sequence encoding a protein having the amino acid sequence of SEQ ID NO: 2.
(10c) The lipid nanoparticle described in (8a) in which the nucleic acid is the mRNA encoding NeuroD1 protein described in (8a) containing the base sequence of SEQ ID NO: 1.
(10d) The lipid nanoparticle described in (8a) in which the nucleic acid is the mRNA encoding NeuroD1 protein described in (8a) containing the base sequence of SEQ ID NO: 3.
(10e) The lipid nanoparticle described in (8a) in which the nucleic acid is the mRNA encoding NeuroD1 protein described in (8a) containing the base sequence of SEQ ID NO: 4.
(10f) The lipid nanoparticle described in (8a) in which the nucleic acid is the mRNA encoding NeuroD1 protein described in (8a) containing the base sequence of SEQ ID NO: 5.
(10g) A lipid nanoparticle containing 20.0 to 70.0 mol% of 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, 27.0 to 79.5 mol% of the neutral lipids (namely DSPC and cholesterol), and 0.5 to 3.0 mol% of DMG-PEG2000 based on the total amount of the lipid nanoparticle in (10a) to (10f).
(10h) A lipid nanoparticle containing 35.0 to 50.0 mol% of 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, 47.5 to 64.0 mol% of the neutral lipids (namely DSPC and cholesterol), and 1.0 to 2.5 mol% of DMG-PEG2000 based on the total amount of the lipid nanoparticle in (10a) to (10f).
(10i) A lipid nanoparticle which encapsulates a nucleic acid and contain a neutral lipid, a PEGylated lipid, and the compound of the formula (I) or a salt thereof and
   in which the nucleic acid is mRNA encoding NeuroD1 protein in which in the base sequence, the nucleotides at positions 1 to 3 are a 5' terminal sequence suitable for the capping method using CleanCap (registered trademark) Reagent AG (TriLink BioTechnologies), positions 4 to 43 are a 5' UTR, positions 44 to 1114 are CDS of human NeuroD1 gene (SEQ ID NO: 3), positions 1115 to 1120 are two successive stop codons, positions 1121 to 1231 are a 3' UTR, and positions 1232 to 1310 correspond to a poly(A) tail.

Aspects of the specific lipid nanoparticle included in the invention are as follows.
(11a) A lipid nanoparticle encapsulating a nucleic acid and containing neutral lipids, a PEGylated lipid, and the compound of the formula (I) or a salt thereof
   in which the nucleic acid is mRNA encoding human ND1 (here, the 5' cap structure is Cap-1, the CDS is CDS of human ND1 gene (SEQ ID NO: 1), and the poly(A) tail has 120 bases) and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(11b) A lipid nanoparticle encapsulating a nucleic acid and containing neutral lipids, a PEGylated lipid, and the compound of the formula (I) or a salt thereof
   in which the nucleic acid is mRNA encoding human ND1 (which has a base sequence (SEQ ID NO: 5) in which all the uridines are N1-methylpseudouridines in a base sequence (SEQ ID NO: 4) having a 5' cap structure that is Cap-1, a 5' UTR that is derived from human α-globin gene, a CDS that is CDS of human ND1 gene (SEQ ID NO: 3), a 3' UTR that is derived from human α-globin gene, and a poly(A) tail of 79 polynucleotides, where in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) sequence) and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(11c) A lipid nanoparticle encapsulating a nucleic acid and containing neutral lipids, a PEGylated lipid, and the compound of the formula (I) or a salt thereof
   in which the nucleic acid is mRNA encoding human ND1 (which has a 5' cap structure that is Cap-1, the base sequence of positions 1 to 1231 of SEQ ID NO: 4 or the base sequence of positions 1 to 1231 of SEQ ID NO: 5, and a poly(A) tail) and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(11d) The lipid nanoparticle described in (11a) to (11c) encapsulating a nucleic acid and containing neutral lipids, a PEGylated lipid, and the compound of the formula (I) or a salt thereof which contain 50 mol% of 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, 48.5 mol% of DSPC and cholesterol, and 1.5 mol% of DMG-PEG2000 based on the total amount of the lipid nanoparticle.

### 3. Pharmaceutical Composition

Aspects of the pharmaceutical composition containing a lipid nanoparticle containing the compound of the formula (I), which is the compound of the invention, or a salt thereof and one or more pharmaceutically acceptable pharmaceutical additives are shown below.

### (Aspects of Pharmaceutical Composition of Invention)

(1) A pharmaceutical composition containing the lipid nanoparticle described in any of (3) to (11) of (Aspects of Lipid Nanoparticle of Invention).
(2) A pharmaceutical composition containing the lipid nanoparticle described in any of (3) to (11) of (Aspects of Lipid Nanoparticle of Invention) and one or more pharmaceutically acceptable pharmaceutical additives.
(3) The pharmaceutical composition described in (1) or (2) for the prevention and/or the treatment of an astrocyte-related disease.

Aspects of the specific pharmaceutical compositions included in the invention are as follows.
(A) A pharmaceutical composition containing a lipid nanoparticle encapsulating a nucleic acid and containing neutral lipids, a PEGylated lipid, and the compound of the formula (I) or a salt thereof
   in which the nucleic acid is mRNA encoding NeuroD1 protein and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(B) A pharmaceutical composition containing a lipid nanoparticle encapsulating a nucleic acid and containing the compound of the formula (I) or a salt thereof, neutral lipids, and a PEGylated lipid
   in which the nucleic acid is mRNA encoding NeuroD1 protein containing a base sequence encoding a protein having the amino acid sequence of SEQ ID NO: 2 and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(C) A pharmaceutical composition containing a lipid nanoparticle encapsulating a nucleic acid and containing the compound of the formula (I) or a salt thereof, neutral lipids, and a PEGylated lipid
   in which the nucleic acid is mRNA encoding human ND1 (here, the 5' cap structure is Cap-1, the CDS is CDS of human ND1 gene (SEQ ID NO: 1), and the poly(A) tail has 120 bases) and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(D) A pharmaceutical composition containing a lipid nanoparticle encapsulating a nucleic acid and containing the compound of the formula (I) or a salt thereof, neutral lipids, and a PEGylated lipid
   in which the nucleic acid is mRNA encoding human ND1 (which has a base sequence (SEQ ID NO: 5) in which all the uridines are N1-methylpseudouridines in a base sequence (SEQ ID NO: 4) having a 5' cap structure that is Cap-1, a 5' UTR that is derived from human α-globin gene, a CDS that is CDS of human ND1 gene (SEQ ID NO: 3), a 3' UTR that is derived from human α-globin gene, and a poly(A) tail of 79 polynucleotides, where in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) sequence) and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.
(E) A pharmaceutical composition containing a lipid nanoparticle encapsulating a nucleic acid and containing the compound of the formula (I) or a salt thereof, neutral lipids, and a PEGylated lipid
   in which the nucleic acid is mRNA encoding human ND1 (which has a 5' cap structure that is Cap-1, the base sequence of positions 1 to 1231 of SEQ ID NO: 4 or the base sequence of positions 1 to 1231 of SEQ ID NO: 5, and a poly(A) tail) and
   in which, in the lipid nanoparticle, the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof, the neutral lipids are DSPC and cholesterol, and the PEGylated lipid is DMG-PEG2000.

Although the administration form of the pharmaceutical composition is not limited, examples thereof include parenteral administration by intracerebral, intraarticular, intravenous, intramuscular, subcutaneous or other injection, a transmucosal liquid agent, an inhalant, or the like. In general, through intracerebral administration, the pharmaceutical composition is delivered to the central nervous system. In an aspect, the administration is administration into the brain parenchyma. In an aspect, the administration is intraventricular administration. In an aspect, the administration is intraspinal administration. In an aspect, the administration is intramedullary administration. In an aspect, the administration is administration into the spinal cord parenchyma.

In general, in the case of intracerebral administration, the pharmaceutical composition containing the lipid nanoparticle of the invention is administered at about 0.001 to 10 mg per 1 kg of the brain in an aspect, at about 0.001 to 50 mg per 1 kg of the brain in an aspect, or about 0.001 to 100 mg per 1 kg of the brain in an aspect, once a day or in several divided portions. The dose is appropriately decided depending on the individual case considering the disease, the symptom, the age, the gender, and the like.

In general, in the case of intramuscular administration, the daily dose of the pharmaceutical composition of the invention is appropriately about 1 to 10 mg/kg body weight, and the administration is once a day or in several divided portions. The dose is appropriately decided depending on the individual case considering the disease, the symptom, the age, the gender, and the like.

Although it depends on the route of administration, the dosage form, the site of administration, and the types of the excipient and the additive, the pharmaceutical composition of the invention contains, based on the total weight of the pharmaceutical composition, 5 to 50 weight% of the lipid nanoparticle in an aspect, and the amount of the lipid nanoparticle or the like is 3 to 70 weight% in an aspect or 10 to 50 weight% in an aspect.

The pharmaceutical composition of the invention can be used in combination with various therapeutic agents or preventive agents for a disease to which the pharmaceutical composition is considered to have an effectiveness. The combination use may be simultaneous administration or separate administration which is sequential or at a desired interval. A simultaneous administration preparation may be a formulated agent or may be separately formulated.

### 4. Production Method of Cationic Lipid of Invention

The compound of the formula (I), which is the compound of the invention, or a salt thereof can be produced by applying various known synthetic methods using characteristics based on the basic structure or the type of substituent thereof. Here, depending on the type of the functional group, it is sometimes effective as a production technique to substitute the functional group with an appropriate protective group (a group that can be easily converted to the functional group) in the process from a raw material to an intermediate.

Examples of the protective group include protective groups described in Wuts (P. G. M. Wuts) and Greene (T. W. Greene), "Greene's Protective Groups in Organic Synthesis (5th edition, 2014)" and the like, and a group may be appropriately selected and used depending on the reaction conditions. In such a method, a desired compound can be obtained by conducting a reaction with the protective group introduced and then removing the protective group, when required.

Typical methods for producing the compound of the formula (I) or a salt thereof are explained below. The production methods can also be carried out referring to the reference documents attached to the descriptions. In this regard, the production method of the invention is not limited to the examples shown below.

The compound of the invention or a salt thereof can be produced by the production methods below.

### (First Production Method)

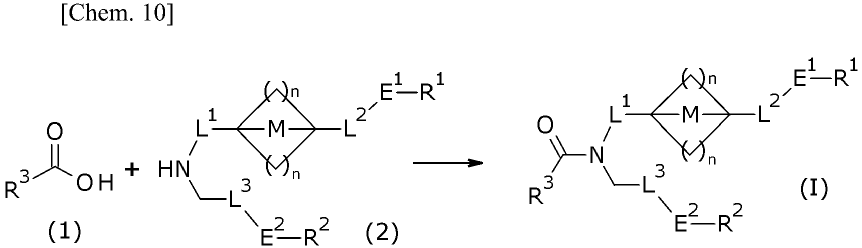

The compound (I) of the invention can be produced by an amidation reaction of a compound (1) and a compound (2).

This reaction is conducted in a solvent using a mixture of the compound (1) and the compound (2) with a condensing agent. The solvent is an aromatic hydrocarbon such as benzene, toluene, and xylene, a halogenated hydrocarbon such as DCM, DMF, DMSO, or a mixed solvent thereof. The condensing agent is HATU, EDCI·HCl, or the like. An additive (for example, HOBt or DMAP) sometimes promotes the reaction. An organic base (TEA, DIPEA, or the like) and an inorganic base (K₂CO₃, Na₂CO₃, or KOH) sometimes further promote the reaction.

The compound (I) can also be produced by converting the carboxylic acid (1) to a reactive derivative and then reacting the amine (2).

Examples of the reactive derivative of the carboxylic acid include an acid halogenation product obtained by a reaction with a halogenating agent such as phosphorus oxychloride and thionyl chloride, a mixed acid anhydride obtained by a reaction with isobutyl chloroformate or the like, an active ester obtained by condensation with HOBt, and the like. The reaction of such a reactive derivative and the compound (2) can be conducted in a solvent at a temperature of -20°C to 60°C in an aspect. The solvent is a halogenated hydrocarbon such as DCM, an aromatic hydrocarbon, an ether, or the like.

In this reaction, instead of using the compound (1), the compound (I) can also be produced using a group protected with a protective group which can be converted to R³ through deprotection or a desired reaction to induce the group protected with the protective group which can be converted to R³ to R³. In this reaction, the compound (I) can also be produced by conducting this reaction using a group which can be converted to R³ instead of the compound (1) and then inducing to R³.

[Documents] S. R. Sandler and W. Karo, "Organic Functional Group Preparations", Academic Press Inc., 1991, 2nd edition, Vol. 1 and "Jikken Kagaku Koza (Courses in Experimental Chemistry) (5th edition)" edited by The Chemical Society of Japan, 2005, Vol. 16, Maruzen

### (Second Production Method)

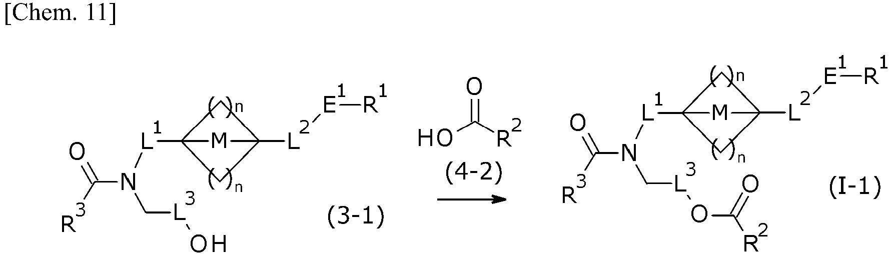

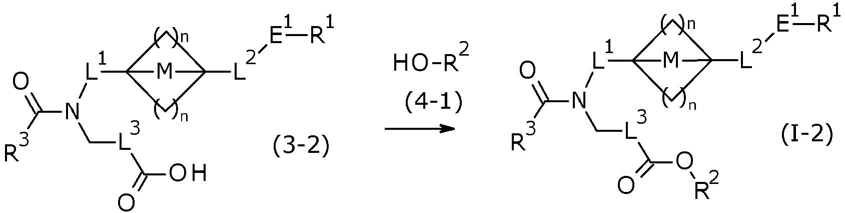

A compound (I-1) of the invention can be produced by an esterification reaction of a compound (3-1) and a compound (4-2).

This reaction is conducted in a solvent using a mixture of the compound (3-1) and the compound (4-2) with a condensing agent. The solvent is a halogenated hydrocarbon such as DCM, DMF, DMSO, or a mixed solvent thereof. The condensing agent is HATU, EDCI·HCl, CDI, DPPA, or phosphorus oxychloride. An additive (for example, HOBt or DMAP) sometimes promotes the reaction. An organic base (TEA, DIPEA, or the like) and an inorganic base (K₂CO₃, Na₂CO₃, or KOH) sometimes further promote the reaction.

The compound (I-1) can also be produced by converting the carboxylic acid (4-2) to a reactive derivative and then reacting the alcohol (3-1).

Examples of the reactive derivative of the carboxylic acid include an acid halogenation product obtained by a reaction with a halogenating agent such as phosphorus oxychloride and thionyl chloride, a mixed acid anhydride obtained by a reaction with isobutyl chloroformate or the like, an active ester obtained by condensation with HOBt, and the like. The reaction of such a reactive derivative and the compound (3-1) can be conducted in a solvent at a temperature of -20°C to 60°C in an aspect. The solvent is a halogenated hydrocarbon such as DCM or the like.

A compound (I-2) of the invention can be produced by an esterification reaction of a compound (3-2) and a compound (4-1). The same conditions as those in the second production method can be used for this reaction.

### (First Raw Material Production Method)

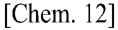

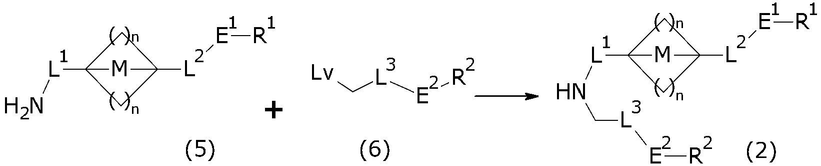

(In the formula, Lv represents a leaving group.)

The compound (2) can be produced by a reaction of a compound (5) and a compound (6). Here, examples of the leaving group Lv include a halogen, a methanesulfonyloxy group, and the like.

In this reaction, the compound (5) and the compound (6) are used in an equal amount or with either thereof in an excess amount, and the mixture thereof is reacted in a solvent. The reaction temperature is 0°C to 100°C in an aspect. In an aspect, the reaction temperature is 50°C to 90°C. The solvent is a halogenated hydrocarbon such as DCM and 1,2-dichloroethane, DMF, DMSO, MeCN, CPME, or a mixture thereof. An organic base (TEA, DIPEA, or the like) and an inorganic base (K₂CO₃, Na₂CO₃, or KOH) or an additive (KI or NaI) and the like sometimes further promote the reaction.

Here, the raw material compound (6) can be produced using a corresponding raw material by esterification or carbonation to form E2. The conditions described above can be used for the esterification.

[Documents] S. R. Sandler and W. Karo, "Organic Functional Group Preparations", 1991, 2nd edition, Vol. 1, Academic Press Inc. and "Jikken Kagaku Koza (Courses in Experimental Chemistry) (5th edition)" edited by The Chemical Society of Japan, 2005, Vol. 14, Maruzen

### (Second Raw Material Production Method)

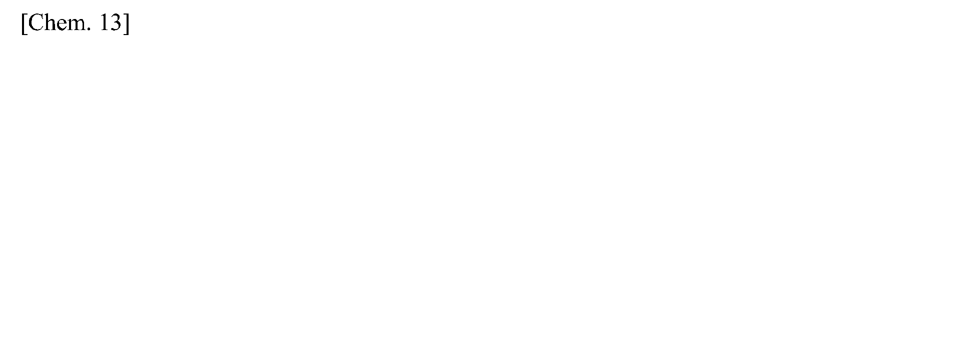

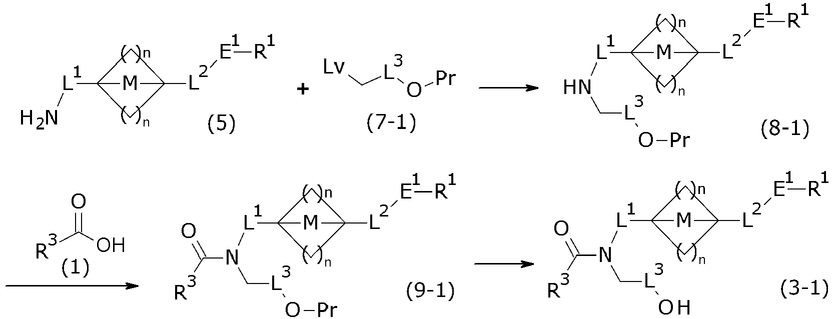

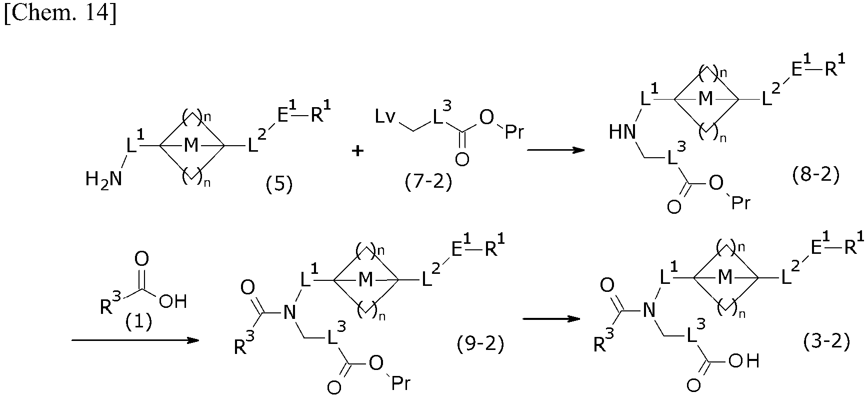

The compound (3-1) can be produced by an alkylation reaction of the compound (5) and a compound (7-1), an amidation reaction of the obtained compound (8-1) and the compound (1), and deprotection of the obtained compound (9-1). The alkylation reaction can be conducted in the same manner as in the method of the first raw material production method. The amidation can be conducted in the same manner as that described in the first production method. The compound (3-2) can be produced in the same manner as in the production method of the compound (3-1). The deprotection can be conducted by general deprotection. "Protective Groups in Organic Synthesis" written by Greene and Wuts, 3rd edition, John Wiley & Sons Inc, 1999 can be referred to.

### (Third Raw Material Production Method)

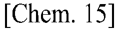

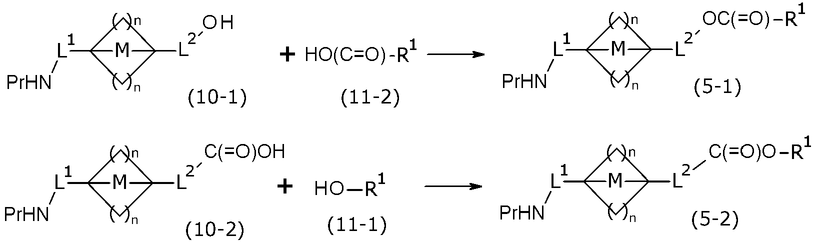

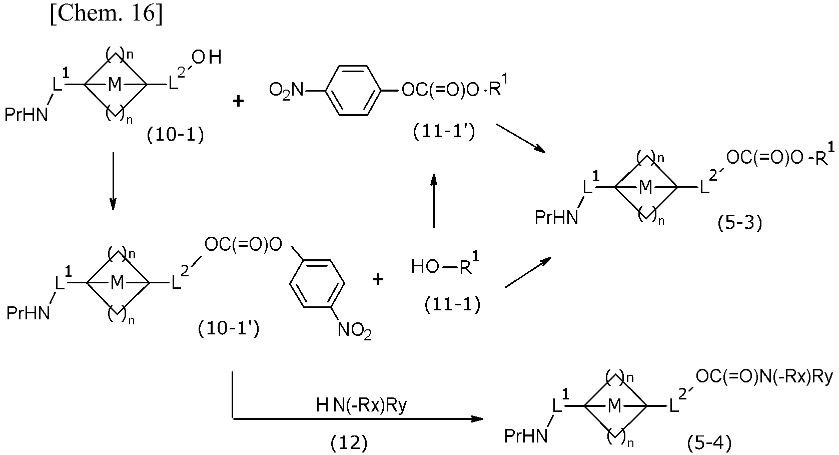

(In the formulae, Pr represents a protective group.)

Compounds (5-1) to (5-4), which are aspects of the compound (5) that are protected with a protective group, can be produced by esterification, carbonation, or carbamation. The compound (5-3) can also be produced from a compound (10-1) and a compound (11-1'). A compound (10-1') can be produced from the compound (10-1). The compound (5-3) can be produced from the compound (10-1') and a compound (11-1). The compound (11-1') can be produced from the compound (11-1). The compound (5-4) can be produced from the compound (10-1') and a compound (12). In the above production method, an example in which the compound (10-1') and the compound (11-1') are isolated once before the next reaction is shown, but the next reaction can be conducted subsequently without isolation depending on the compound.

### (Fourth Raw Material Production Method)

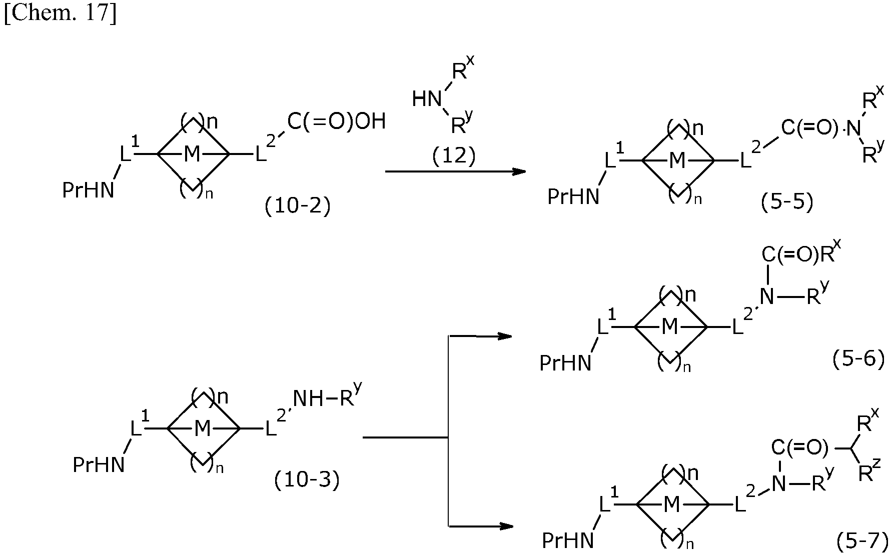

(In the formulae, Pr represents a protective group.)

Compounds (5-5) to (5-7), which are aspects of the compound (5) that are protected with a protective group, can be produced by amidation.

The compound of the formula (I) or a salt thereof is isolated and purified as a free compound or a salt, a hydrate, a solvate, or a crystal polymorphous substance thereof. The compound of the formula (I) or a salt thereof can also be produced by subjecting to a salt formation reaction which is an ordinary method. The isolation and purification are performed by applying a general operation, such as extraction, fractional crystallization, and various types of fraction chromatography (silica gel chromatography and the like).

Various isomers can be produced by selecting an appropriate raw material compound or can be separated using a difference in physicochemical properties between the isomers. For example, an optical isomer can be obtained by a general optical resolution method of a racemate (for example, fractional crystallization for inducing to a diastereomer salt with an optically active base or acid, chromatography using a chiral column or the like, and the like) and can also be produced from an appropriate optically active raw material compound.

### 5. Production Method of Lipid Nanoparticle of Invention

Lipid nanoparticle can be produced by adding a component such as a nucleic acid to components of the compound of the formula (I) or a salt thereof, a neutral lipid, and a PEGylated lipid and dispersing in a medium.

For example, the compound of the formula (I) or a salt thereof, DSPC, a sterol, a PEGylated lipid, and the like are dissolved in a solvent to obtain an oil phase, and the oil phase is mixed with or suspended in an aqueous phase such as a buffer solution containing a nucleic acid. Then, the solvent in the mixture solution is removed by a method such as dialysis and ultrafiltration, and lipid nanoparticle are thus obtained.

Moreover, a pharmaceutical composition can be produced by a known method using an additive such as the excipient described above and the nucleic acid lipid nanoparticle.

### 6. Production Method of Pharmaceutical Composition

The pharmaceutical composition can be prepared by a generally used method using a pharmaceutical additive (an excipient or the like) generally used in the field, namely, a pharmaceutical excipient or the like. Regarding the pharmaceutical additive, although the components contained in the pharmaceutical composition are not limited, an additive, such as a preservative, a stabilizer, an antioxidant, and an antiseptic, may be contained in addition to the excipient. Another additive may be added to the pharmaceutical composition. Moreover, a pharmaceutically acceptable medium may be added to the pharmaceutical composition.

The pharmaceutical composition is refrigerated or frozen for storage and/or transport. The temperature is about -150°C to about 0°C in an aspect, about -80°C to about - 20°C in an aspect, about -40°C to about -20°C, or 4°C or lower in an aspect. In an aspect, the solution is PBS.

The injection preferably contains a sterile aqueous or nonaqueous solution, suspension, or emulsion. Examples of the aqueous solvent include distilled water for injection or physiological saline. An example of the nonaqueous solvent is an alcohol such as ethanol. Such a composition may further contain an isotonizing agent, an antiseptic, a wetting agent, an emulsifier, a dispersant, a stabilizer, or a solubilizing agent. These are sterilized, for example, by filtration through a bacteria keeping filter, incorporation of a microbicide, or irradiation. In addition, such a composition can be produced as a sterile solid composition which is dissolved or suspended in sterile water or a sterile solvent for injection before use.

The transmucosal agent such as an inhalant or a transnasal agent is used in a liquid form and can be produced according to a conventionally known method. For example, a known excipient and in addition, a pH modifier, an antiseptic, a surfactant, a lubricant, a stabilizer, a thickener, or the like may be appropriately added. The administration can be performed using an appropriate device for inhalation or insufflation. For example, the agent can be administered using a known device such as a metering and administering inhalation device or an atomizer, as a compound alone or a powder of a formulated mixture, or as a solution or a suspension in combination with a pharmaceutically acceptable pharmaceutical additive. A dry powder inhaler or the like may be for a single administration or multiple administrations. The agent may be used in a form of a pressurized aerosol spray or the like using an appropriate ejection agent, for example, a suitable gas, such as a chlorofluoroalkane or carbon dioxide.

### 7. Production Method of Nucleic Acid

The nucleic acid, such as mRNA for example, can be produced using a known technique in the field. Examples are shown below, but the production is not limited to the examples. mRNA can be produced using a linear plasmid as a DNA template by *in vitro* transcription (IVT). As the production methods, (i) the post-transcriptional capping method (in the process, plasmid production, *in vitro* transcription, and 5' capping are conducted in this order), (ii) the co-transcriptional capping method (in the process, plasmid production and then *in vitro* transcription together with 5' capping are conducted), and the like are known. In the co-transcriptional capping method, Cap-0 is added to the 5' terminal of the RNA when the anti-reverse cap analog (ARCA) technique is used, and Cap-1 can be added to the 5' terminal of the RNA when the CleanCap (registered trademark, TriLink) capping technique is used.

### (in vitro Transcription)

In IVT, a transcription buffer solution, nucleoside triphosphates (NTPs), an RNase inhibitor, and a polymerase (example: T7 RNA polymerase) can be generally used for the reaction. The NTPs may be of natural type or unnatural type (modified type).

### (Capping)

The capping can be conducted according to a method using vaccinia capping enzyme, 2'O-methyltransferase, CleanCap, or the like or another method. When the sequence transcribed by IVT does not contain any poly(A) sequence, poly(A) addition reaction can also be conducted. Here, regarding the sequence, when the capping method using CleanCap (registered trademark) Reagent AG (TriLink BioTechnologies) is used, the 5' terminal sequence suitable for the capping method using CleanCap (registered trademark) Reagent AG (TriLink BioTechnologies) is, for example, AGG (position 1 to position 3 of SEQ ID NO: 4).

### (Plasmid Production)

The linear plasmid can be produced by the following processes. A plasmid obtained by inserting an UTR and a base sequence (example: a base sequence encoding NeuroD1 protein) into a plasmid (example: a high-copy plasmid for *Escherichia coli* (pUC18, pCU18, or the like)) can be produced and can be amplified using *Escherichia coli* or the like. After purification, the linear plasmid can be produced from the plasmid using a restriction enzyme and a buffer solution. After purification, the linear plasmid can be used directly as a DNA template. Alternatively, the linear plasmid can be used as a DNA template after subjecting to polymerase chain reaction (PCR).

### (Purification)

The mRNA and the intermediates in the production processes can be purified by a known method in the field. Examples are shown below, but the purification is not limited to the examples. The DNA template can be removed with deoxyribonuclease I (DNase I). The transcribed RNA can be purified using a silica gel column or the like. The RNA containing a poly(A) sequence can be purified with an Oligo dT column. The linear plasmid can be purified using PureLink (registered trademark) (Thermo Fisher Scientific) or the like.

### [Test Examples]

The pharmacological activities of nucleic acid lipid nanoparticle composed of the compounds of the formula (I) or salts thereof were examined by the following tests. In the Test Examples and the like below in this specification, the abbreviations below are sometimes used.

### (Abbreviations)

B-27: B-27 serum-free supplement, BDNF: brain-derived neurotrophic factor, DMEM: Dulbecco's modified Eagle medium, DMG-PEG2000: 1,2-dimyristoyl-rac-glycero-3-methoxypolyethylene glycol 2000, DOTAP: 1,2-dioleoyloxy-3-trimethylammonium propane, DOTMA: 1,2-di-O-octadecenyl-3-trimethylammonium propane, DSPC: 1,2-distearoyl-sn-glycero-3-phosphocholine, eGFP: enhanced green fluorescent protein, FBS: fetal bovine serum, FLuc nucleic acid lipid nanoparticle: lipid nanoparticle encapsulating FLuc mRNA, ND1 nucleic acid lipid nanoparticle: lipid nanoparticle encapsulating NeuroD1 mRNA, FLuc: luciferase (firefly), LNP: lipid nanoparticle, mRNA: messenger ribonucleic acid, ND1: NeuroD1, PBS: phosphate-buffered saline, RLA: relative luciferase activity, RLU: relative light unit, Tris buffer: tris(hydroxymethyl)aminomethane buffer, FLuc eGFP mixture mRNA: a mRNA mixture of FLucmRNA and eGFP mRNA (also called FLuc eGFP mixture nucleic acid), and FLuc eGFP mixture nucleic acid lipid nanoparticle: lipid nanoparticle encapsulating a mRNA mixture of FLuc mRNA and eGFP mRNA.

### Test Example 1: in vitro Luciferase Assay

### Test Example 1-1 (Evaluation of Introduction of Lipid Nanoparticle Using Mouse Primary Astrocytes)

Regarding the compounds of the formula (I) or salts thereof composing lipid nanoparticle, the efficiencies of introduction of a nucleic acid into mouse primary astrocytes using lipid nanoparticle were evaluated. As the mRNA, FLuc mRNA (TriLink BioTechnologies) was used, and nucleic acid lipid nanoparticle were produced using NanoAssemblr ((registered trademark), manufactured by Precision NanoSystems). The efficiencies of introduction of the test drugs (nucleic acid lipid nanoparticle) were calculated by evaluating the expression levels of luciferase encoded by the mRNA. Three wells were used for each group. The methods and the results are shown.

### (Acquisition of Mouse Primary Astrocytes)

Mouse primary astrocytes were acquired in accordance with Lynette C. Foo., Purification of Rat and Mouse Astrocytes by Immunopanning, Cold Spring Harbor Protocols, 2013, vol.5, p.421-432. In this regard, however, the cells were recovered in 5% CO₂. As the mouse, newborn P1-P10 of C57BL/6J (The Jackson Laboratory Japan, Inc.) was used.

### (Test Method)

The mouse primary astrocytes acquired by the above method were seeded at 2000 cells/well in a poly-D-lysine coated 96-well plate (IWAKI, Greiner) and cultured in 5% CO₂ at 37°C overnight. As the medium, 100 µL/well of DMEM/F-12 medium (Thermo Fisher Scientific) containing 2% B-27 (Thermo Fisher Scientific), 10% FBS (Cytiva), and 1% penicillin-streptomycin (PS) (Thermo Fisher Scientific) was used. On the next day, 100 µL of the test drugs (FLuc nucleic acid lipid nanoparticle) diluted with PBS and the medium were added to a final concentration of 0.8 µg/mL, and the cells were cultured at 200 µL/well overnight. After removing all the medium on the next day, ONE-Glo Luciferase assay system (Promega) was diluted two-fold with PBS, and 100 µL thereof was added to each well. After mixing for a minute, 80 µL thereof were transferred to a 96-well white plate (Thermo Fisher Scientific), and the luminescence thereof was detected with Envision (Revvity). RLU (Relative Light Unit) was used as the indicator of activity.

### (Evaluation of Expression)

As a result of the above test, luciferase activity was observed with the nucleic acid lipid nanoparticle containing typical Example compounds of the invention as a component. This suggests that the lipid nanoparticle encapsulating FLuc mRNA were introduced into the cells and translated and that luciferase protein was thus expressed. In the tables below, the results of the above test on the nucleic acid lipid nanoparticle (nucleic acid: Fluc mRNA) containing the Example compounds as a component are shown. Here, NUM indicates the lipid nanoparticle containing the Example compounds, of the compounds of the formula (I) or salts thereof, as a component. ExA-LB (A and B are numbers) indicates lipid nanoparticle containing Example compound ExA, of the compounds of the formula (I) or salts thereof, and having the composition LB. L1 indicates the lipid nanoparticle containing ExA, DSPC, cholesterol, and DMG-PEG2000 (ratio by mole of 50/10/38.5/1.5) as components. For example, Ex1-L1 to Ex5-L1 indicate the lipid nanoparticle containing Example compounds Ex1, Ex2, Ex3, Ex4, and Ex5, respectively, of the compounds of the formula (I) or salts thereof, as a component and having the lipid composition of L1.

**[Table 1]**

| NUM | RLU |
|---|---|
| Ex1-L1 | 49964933 |
| Ex2-L1 | 40064533 |
| Ex3-L1 | 1315333 |
| Ex4-L1 | 10491067 |
| Ex5-L1 | 24935867 |
| Ex6-L1 | 73601600 |
| Ex7-L1 | 62458267 |
| Ex8-L1 | 26825600 |
| Ex9-L1 | 4458800 |
| Ex10-L1 | 7839333 |
| Ex11-L1 | 27729200 |
| Ex12-L1 | 9107067 |
| Ex13-L1 | 2543067 |
| Ex14-L1 | 17064933 |
| Ex15-L1 | 38131067 |
| Ex16-L1 | 10069067 |
| Ex17-L1 | 2284667 |
| Ex18-L1 | 24459867 |
| Ex19-L1 | 28337467 |
| Ex20-L1 | 12622933 |
| Ex21-L1 | 15351067 |
| Ex22-L1 | 6480800 |
| Ex23-L1 | 18430667 |
| Ex24-L1 | 13300400 |
| Ex25-L1 | 20712933 |
| Ex26-L1 | 3170133 |
| Ex27-L1 | 3673467 |
| Ex28-L1 | 6195467 |
| Ex29-L1 | 2079600 |
| Ex30-L1 | 18768800 |

**[Table 2]**

| NUM | RLU |
|---|---|
| Ex31-L1 | 64790500 |
| Ex33-L1 | 46617900 |
| Ex34-L1 | 26026300 |
| Ex35-L1 | 16592000 |
| Ex36-L1 | 28621100 |
| Ex37-L1 | 51429300 |
| Ex38-L1 | 22455600 |
| Ex39-L1 | 9518100 |
| Ex40-L1 | 17413600 |
| Ex41-L1 | 31716800 |
| Ex42-L1 | 40070000 |
| Ex43-L1 | 42878000 |
| Ex44-L1 | 22490900 |
| Ex45-L1 | 38534300 |
| Ex46-L1 | 32306400 |
| Ex47-L1 | 43886000 |
| Ex48-L1 | 44250800 |
| Ex49-L1 | 29234100 |
| Ex50-L1 | 47043600 |
| Ex51-L1 | 42326100 |
| Ex52-L1 | 27303300 |
| Ex53-L1 | 46107100 |
| Ex54-L1 | 55414400 |
| Ex55-L1 | 46786400 |
| Ex56-L1 | 52673500 |
| Ex57-L1 | 8328000 |
| Ex58-L1 | 48463700 |
| Ex59-L1 | 6029200 |
| Ex60-L1 | 31469900 |
| Ex61-L1 | 14001200 |
| Ex62-L1 | 30160100 |
| Ex63-L1 | 43167200 |
| Ex64-L1 | 45327700 |
| Ex65-L1 | 49223600 |
| Ex66-L1 | 52186300 |
| Ex67-L1 | 47706400 |
| Ex68-L1 | 22798400 |
| Ex69-L1 | 34872700 |
| Ex70-L1 | 1874300 |
| Ex71-L1 | 49428400 |
| Ex72-L1 | 39885500 |
| Ex73-L1 | 40784500 |
| Ex74-L1 | 53311700 |
| Ex75-L1 | 57106000 |

In the table below, the results of the above test on the nucleic acid lipid nanoparticle (nucleic acid: Fluc mRNA) containing the Example compounds as a component are shown. Ex1-L1 to Ex1-L54 shown in NUM in the table below indicate Ex1-L1-FLuc mRNA to Ex1-L54-FLuc mRNA, respectively. This means that Ex1-L1 to Ex1-L54 are lipid nanoparticle each encapsulating FLuc mRNA, having the lipid composition described below and containing Example compound Ex1. The average values of RLU on the groups of the nucleic acid lipid nanoparticle were calculated, and the relative values (RLA (Relative Luciferase Activity)) are shown, where the value of Ex1-L1 is regarded as 1. In this regard, Ex1-L1a, Ex1-L1b, and Ex1-L1c in the table below all show Ex1-L1 with different preparation lot numbers. Of Ex1-L2 to Ex1-L48, for Ex1-L2, Ex1-L3, Ex1-L5, Ex1-L8, Ex1-L12, Ex1-L16, Ex1-L21, Ex1-L24, Ex1-L26, Ex1-L29, Ex1-L35, Ex1-L39, Ex1-L45, Ex1-L47, and Ex1-L48, the relative values are shown as RLAs, where the value of Ex1-L1a is regarded as 1: for Ex1-L4, Ex1-L6, Ex1-L7, Ex1-L9 to Ex1-L11, Ex1-L13 to Ex1-L15, Ex1-L17 to Ex1-L20, Ex1-L22, Ex1-L23, Ex1-L25, Ex1-L27, Ex1-L28, Ex1-L30 to Ex1-L34, Ex1-L36 to Ex1-L38, Ex1-L40 to Ex1-L44, and Ex1-L46, the relative values are shown as RLAs, where the value of Ex1-L1b is regarded as 1: and for Ex1-L51 to Ex1-L54, the relative values are shown as RLAs, where the value of Ex1-L1c is regarded as 1. Here, although RLAs were not calculated for Ex1-L49 and Ex1-L50, it was found that a certain amount of luciferase protein was expressed as a result of the above test.

**[Table 3]**

| NUM | RLA |
|---|---|
| Ex1-L1a | 1.00 |
| Ex1-L1b | 1.00 |
| Ex1-L1c | 1.00 |
| Ex1-L2 | 0.34 |
| Ex1-L3 | 0.61 |
| Ex1-L4 | 1.08 |
| Ex1-L5 | 0.54 |
| Ex1-L6 | 1.04 |
| Ex1-L7 | 0.96 |
| Ex1-L8 | 0.86 |
| Ex1-L9 | 1.02 |
| Ex1-L10 | 1.04 |
| Ex1-L11 | 1.17 |
| Ex1-L12 | 0.52 |
| Ex1-L13 | 0.37 |
| Ex1-L14 | 0.55 |
| Ex1-L15 | 1.37 |
| Ex1-L16 | 0.92 |
| Ex1-L17 | 0.90 |
| Ex1-L18 | 0.77 |
| Ex1-L19 | 1.01 |
| Ex1-L20 | 0.84 |
| Ex1-L21 | 0.41 |
| Ex1-L22 | 0.86 |
| Ex1-L23 | 0.45 |
| Ex1-L24 | 0.35 |
| Ex1-L25 | 0.26 |
| Ex1-L26 | 0.59 |
| Ex1-L27 | 0.58 |
| Ex1-L28 | 0.80 |
| Ex1-L29 | 0.79 |
| Ex1-L30 | 0.92 |
| Ex1-L31 | 0.71 |
| Ex1-L32 | 1.09 |
| Ex1-L33 | 0.06 |
| Ex1-L34 | 0.70 |
| Ex1-L35 | 0.43 |
| Ex1-L36 | 0.48 |
| Ex1-L37 | 0.20 |
| Ex1-L38 | 0.42 |
| Ex1-L39 | 0.74 |
| Ex1-L40 | 0.62 |
| Ex1-L41 | 0.75 |
| Ex1-L42 | 0.01 |
| Ex1-L43 | 0.15 |
| Ex1-L44 | 0.52 |
| Ex1-L45 | 0.02 |
| Ex1-L46 | 0.64 |
| Ex1-L47 | 0.33 |
| Ex1-L48 | 0.35 |
| Ex1-L51 | 0.99 |
| Ex1-L52 | 0.05 |
| Ex1-L53 | 0.04 |
| Ex1-L54 | 0.07 |

### Test Example 1-2 (Evaluation of Introduction of Lipid Nanoparticle Using Hepa 1-6 Cells)

Regarding the compounds of the formula (I) or salts thereof composing lipid nanoparticle, the efficiencies of introduction of nucleic acids into Hepa 1-6 cell line (ATCC, a mouse hepatoma cell line) using lipid nanoparticle were evaluated. As the mRNA, a mixture of FLuc mRNA and eGFP mRNA (ratio by mole of 1:1) (TriLink BioTechnologies) was used, and nucleic acid lipid nanoparticle were produced using NanoAssemblr ((registered trademark), manufactured by Precision NanoSystems). The efficiencies of introduction of the test drugs (nucleic acid lipid nanoparticle) were calculated by evaluating the expression levels of luciferase encoded by the mRNA. Three wells were used for each group. The methods and the results are shown below.

### (Test Method)

Hepa 1-6 cells were seeded at 2000 cells/well in a 96-well plate (Corning) and cultured in 5% CO₂ at 37°C overnight. As the medium, 100 µL/well of DMEM medium (Sigma) containing 10% FBS (Cytiva) and 1% penicillin-streptomycin (PS) (Thermo Fisher Scientific) was used. On the next day, 100 µL of the test drugs (FLuc_eGFP nucleic acid lipid nanoparticle) diluted with PBS and the medium were added to a final concentration of 0.8 µg/mL, and the cells were cultured at 200 µL/well two nights. After removing all the medium two days later, ONE-Glo Luciferase assay system (Promega) was diluted two-fold with PBS, and 100 µL thereof was added to each well. After mixing for three minutes, 80 µL thereof were transferred to a 96-well white plate (Nunc), and the luminescence thereof was detected with Envision (Revvity). RLU (Relative Light Unit) was used as the indicator of activity.

### (Evaluation of Expression)

As a result of the above test, luciferase activity was observed with the nucleic acid lipid nanoparticle containing typical Example compounds of the invention as a component. This suggests that the lipid nanoparticle encapsulating the mixture of FLuc mRNA and eGFP mRNA were introduced into the cells and translated and that luciferase protein was thus expressed. In the table below, the results of the nucleic acid lipid nanoparticle (nucleic acids: FLuc eGFP mixture mRNA) containing the Example compounds as a component are shown. Ex6-L1 shown in NUM in the table below indicates Ex6-L1-FLuc eGFP mixture mRNA. Ex6-L1 is lipid nanoparticle encapsulating the mixture of FLuc eGFP mixture mRNA, having the same lipid composition as that of Ex1-L1 and containing Example compound Ex6. Similarly, Ex7-L1 shown in NUM in the table below is lipid nanoparticle encapsulating Ex7-L1-FLuc eGFP mixture mRNA and containing Example compound Ex7. In the table below, the average values of RLU of the groups of the expression of the nucleic acid lipid nanoparticle are shown.

**[Table 4]**

| NUM | RLU |
|---|---|
| Ex6-L1 | 4298480 |
| Ex7-L1 | 6199627 |

### Test Example 2: in vivo Luciferase Assay

### (Test Method)

The efficiencies of introduction of nucleic acid lipid nanoparticle into the brain were evaluated using IVIS spectrum (Revvity). Nucleic acid lipid nanoparticle were administered into the brain parenchyma of 6-12-week-old BALB/c mice (The Jackson Laboratory Japan, Inc., CLEA Japan, Inc., n=3) under isoflurane (isoflurane inhalation anesthetic solution "VTRS"; Mylan N.V., the same applies below) anesthesia. For the administration into the brain parenchyma, the coordinates for administration were determined at 0.0 mm anterior-posterior from the bregma, 2.0 mm to the left, and 2.5 mm deep based on the mouse brain atlas (Academic press). Under isoflurane anesthesia, after shaving with clippers, the skull was fixed with a brain stereotaxic apparatus (KOPF), and a hole with a diameter of around 1 mm was made with a drill (Foredom). An Ito syringe (Ito Corporation) was filled with a test drug (FLuc nucleic acid lipid nanoparticle) of 0.3 mg/mL, and 1 µL thereof was administered to the set coordinates using an injection pump (Narishige Group) at a flow rate of 0.2 µL/min. After leaving still for a minute after the administration, the needle was pulled out slowly, and the scalp was stitched together. After a day, 250 µL/animal of 15 mg/mL luciferin (Promega) was intraperitoneally administered under isoflurane anesthesia, and the luminescence was observed after 20 minutes using IVIS spectrum. The obtained luminescence values were converted to digits with photons/sec unit with Living image software and used for the analysis.

### (Evaluation of Expression)

As a result of the above test, luciferase activity was observed with the lipids of typical Examples of the invention. This suggests that the lipid nanoparticle encapsulating FLuc mRNA were introduced into the cells in the brain and translated and that luciferase protein was thus expressed. The data of expression of the nucleic acid lipid nanoparticle (the average values of three animals of the evaluated FLuc nucleic acid lipid nanoparticle) are shown in the table below. For example, Ex1-L1 shown in NUM in the table below indicates Ex1-L1-FLuc mRNA. This means that Ex1-L1 is lipid nanoparticle encapsulating FLuc mRNA, having the same lipid composition as that of Ex1-L1 and containing Example compound Ex1.

**[Table 5]**

| NUM | Bioluminescence imaging (photons/sec) |
|---|---|
| Ex1-L1 | 102700000 |
| Ex4-L1 | 171900000 |
| Ex7-L1 | 103900000 |
| Ex12-L1 | 52860000 |
| Ex21-L1 | 116400000 |
| Ex25-L1 | 114000000 |
| Ex30-L1 | 184900000 |
| Ex31-L1 | 217400000 |
| Ex49-L1 | 204100000 |
| Ex55-L1 | 179200000 |
| Ex58-L1 | 200500000 |

### Test Example 3: in vitro Conversion of Rat Primary Astrocytes into Neurons

### (Addition of Nucleic Acid Lipid Nanoparticle to Rat Primary Astrocytes)

Rat primary astrocytes were acquired in accordance with Lynette C. Foo. Purification of Rat and Mouse Astrocytes by Immunopanning. Cold Spring Harbor Protocols, 2013, vol. 5, p421-432 and used. In this regard, however, the cells were recovered in 5% CO₂. As the rat, a newborn Wistar rat (CLEA Japan, Inc., P1-P10) was used. The acquired rat primary astrocytes were seeded at 40,000 cells/well in a poly-D-lysine coated 96-well plate (Corning, 354640) and cultured in 5% CO₂ at 37°C overnight. For the culture, 100 µL/well of DMEM/F-12 medium (Thermo Fisher Scientific, 11320033) containing 2% B-27 (registered trademark) supplement (Thermo Fisher Scientific, A1895601), 10% fetal bovine serum (FBS, Cytiva, SH30070.03), and 1% penicillin-streptomycin (PS) (Thermo Fisher Scientific, 15070063) was used.

On the next day, after removing all the culture medium, ND1 nucleic acid lipid nanoparticle (Ex1-L1-ND1 mRNA) diluted with a transdifferentiation medium were added to a final concentration of 0.5 µg/mL, and the cells were cultured. PBS was diluted with the transdifferentiation medium and added to the control. The transdifferentiation medium was Neurobasal (trademark) medium (Thermo Fisher Scientific, 21103049) containing 2% B-27 (registered trademark) supplement, 1% GlutaMAX (trademark) supplement (Thermo Fisher Scientific, 35050061), 2% fetal bovine serum, 1% MEM nonessential amino acids (Fujifilm, 139-15651), 1% PS, and 0.02% BDNF solution. Here, the BDNF solution was prepared by dissolving BDNF powder (PeproTech, 450-02) in pure water to be 100 µg/mL. The whole amount of the medium was replaced with the transdifferentiation medium on the next day (day 1) and on day 3 after the addition of the ND1 nucleic acid lipid nanoparticle.

### (Examination of Expression of NeuroD1 Protein and TUJ1 Protein by Immunoblotting)

Eight hours after the addition of the ND1 nucleic acid lipid nanoparticle and on day 7, the transdifferentiation medium was removed, and the cells were washed with PBS and lysed using RIPA Buffer (Thermo Fisher Scientific, 89901). After protein quantification by the Lowry method, 10 µg of each protein was analyzed by electrophoresis, and the expression of NeuroD1 protein was detected by the immunoblotting method using an anti-NeuroD1 antibody (Abcam, ab60704). Moreover, the expression of TUJ1 protein was detected by the immunoblotting method using an anti-TUJ1 antibody (Abcam, ab78078).

As a result, ND1 protein was expressed eight hours after the addition of the ND1 nucleic acid lipid nanoparticle. The results suggest that the ND1 nucleic acid lipid nanoparticle were introduced into the rat primary astrocytes and translated and that ND1 protein was thus expressed in the rat primary astrocytes. It was also found that the expression of TUJ1 protein increased from eight hours after the addition of the ND1 nucleic acid lipid nanoparticle compared to that of the samples with PBS addition and that the expression increased also on day 7 after the addition of the ND1 nucleic acid lipid nanoparticle. The results show that ND1 mRNA could be delivered to the rat primary astrocytes using the lipid nanoparticle containing Ex1-L1 and that the rat primary astrocytes were converted to neurons because ND1 protein was expressed from the ND1 mRNA in the rat primary astrocytes and exhibited the functions.

### Test Example 4: Action of ND1 Nucleic Acid Lipid Nanoparticle on Cynomolgus Monkey Cerebral Infarction Model

### (Production of Cynomolgus Monkey Cerebral Infarction Model)

Six individuals of male cynomolgus monkey (three years old or older, Shin Nippon Biomedical Laboratories, Ltd.) were subjected to induction anesthesia by intramuscular injection (i.m.) using ketamine hydrochloride (Ketalar for muscular injection 500 mg; Daiichi Sankyo Propharma Co., Ltd.) in an amount of about 10 mg/kg body weight. The cynomolgus monkeys under anesthesia were intratracheally intubated and subjected to maintenance anesthesia by inhalation of isoflurane (isoflurane inhalation anesthetic solution "VTRS"; Mylan N.V.) through the tracheal tube under mechanical ventilation or with spontaneous breathing. After the hair on the head of each cynomolgus monkey under anesthesia was shaved with clippers, the cynomolgus monkey was fixed with a brain stereotaxic apparatus (Narishige Group). Then, with consideration for bleeding, the skin in the head of the cynomolgus monkey was cut open, and the bregma, which is the intersection of the sagittal suture and the coronal suture in the front of the skull surface, was observed. The infusion sites are as shown below based on the bregma (B: 0 mm, ML coordinate: 0 mm (median)).
B: the distance in the anterior-posterior axial direction based on the bregma (a plus value indicates the distance in the front)
L: the distance to the left based on the ML coordinate
D: the depth in the brain from the dura mater in the vertical downward direction

An endothelin-1 (Peptide Institute, Inc. code 4198-v) solution was infused to the six individuals of male cynomolgus monkey. Specifically, in the skull of each cynomolgus monkey, holes with a diameter of around 1 mm were made with a drill at locations (i) B: 2.0 mm, L: 8.0 mm, (ii) B: 5.0 mm, L: 8.0 mm, (iii) B: 9.0 mm, L: 5.0 mm, and (iv) B: 9.0 mm, L: 12.0 mm (four locations in total) based on the bregma for the infusion sites. A micro syringe (HAMILTON) connected to a microinjector (Narishige Group) was inserted from each hole and placed in such a manner that the needle tip was at the depth (D) 19.0 mm ((i) and (ii) above), the depth (D) 13.0 mm ((iii) above), or the depth (D) 15.0 mm ((iv) above) from the dura mater. Endothelin-1 was infused to the four sites in total corresponding to the locations of each individual using the micro syringes. Endothelin-1 was infused at a concentration of 2.0 µg/µL (dissolved in 1% acetic acid (prepared by diluting acetic acid (Wako, 017-00256) with Milli-Q (registered trademark) water)) in an amount of 30 µL/site at an infusion rate of 1.5 µL/min. After the completion of the infusion, the syringes were left still for about 20 minutes to prevent any liquid leakage. The infusion sites (i) to (iv) above target (i) the ventral lateral thalamic nucleus and the ventral posterior thalamic nucleus, (ii) the globus pallidus, (iii) the caudate nucleus, and (iv) the putamen, respectively. By infusing endothelin to the infusion sites as described above, damage was caused to the internal capsule in addition to the targeted sites. In this manner, a cerebral infarction model having basal ganglia disorder, thalamus disorder, and internal capsule disorder was produced. This model can evaluate penetrating branch infarction, cerebral infarction having brain damage in the penetrating branch territory, subacute-phase to chronic-phase cerebral infarction, and cerebral infarction having motor dysfunction with high severity.

### (Administration of ND1 Nucleic Acid Lipid Nanoparticle to Model)

On day 21 after the endothelin-1 infusion treatment (chronic phase), the six individuals of the cerebral infarction model produced by the above method were divided into two groups (a control group with n=3 and an ND1 nucleic acid lipid nanoparticle group with n=3) in such a manner that there was no difference between the groups regarding the mRS for monkeys (see the table below). Here, Ex1-L1-eGFP mRNA was administered to the control group (three individuals), and Ex1-L1-ND1 mRNA was administered to the ND1 nucleic acid lipid nanoparticle group (three individuals).

For medical examination of human stroke, the mRS (for human clinical uses) described in the table below is widely used as an indicator of disability. The mRS (for monkeys) is an indicator in which the mRS (for human clinical uses) is modified for monkeys. As an indicator used for scoring monkeys' motor dysfunction, the Non-Human Primate Stroke Scale (NHPSS), which is an evaluation scale obtained by adopting the National Institutes of Health Stroke Scale (NIHSS), which is an evaluation scale for stroke neurological severity of humans, to nonhuman primate models, is known. The mRS (for monkeys) can evaluate the durability of motor dysfunction like the NHPSS, and the mRS can be used for evaluating the durability of motor dysfunction of this model.

**[Table 6]**

| Score | mRS (for human clinical practice) | mRS (for monkeys) |
|---|---|---|
| 0 | No symptoms at all | No symptoms at all |
| 1 | No significant disability: | Mild paralysis¹ of a forelimb or hindlimb on impaired side |
| | despite symptoms, able to carry out all usual duties and activities | |
| 2 | Slight disability: | Slight decline in locomotor activity³, or mild paralysis of a forelimb and hindlimb on impaired side |
| | unable to perform all previous activities but able to look after own affairs without assistance | |
| 3 | Moderate disability: | (i) Decline in locomotor activity⁴, or (ii) seated posture (including incomplete standing-up motion), or (iii) paralysis² of a forelimb and hindlimb on impaired side. No decreased level of consciousness⁵ |
| | requiring some help but able to walk without assistance | |
| 4 | Moderately severe disability: | (i) Decline in locomotor activity and (ii) seated posture/recumbent posture⁶ (including incomplete standing-up motion), and (iii) paralysis of a forelimb and hindlimb on impaired side or a slightly decreased level of consciousness⁷ |
| | unable to walk without assistance and unable to attend to own bodily needs without assistance | |
| 5 | Severe disability: | Recumbent posture (including incomplete standing-up motion), and paralysis of a forelimb and hindlimb on impaired side, and a decreased level of consciousness⁸ |
| | bedridden, incontinent and requiring constant nursing care and attention | |
| 6 | Death | Death |

| | | |
|---|---|---|
| ¹Mild paralysis: No significant paralysis is observed, but a mild symptom of paralysis is observed. ²Paralysis: Significant paralysis is observed. ³Slightly reduced locomotion: No significantly reduced locomotion is observed, but a mild symptom of reduced locomotion is observed. ⁴Reduced locomotion: Significantly reduced locomotion is observed. ⁵Level of consciousness (monkeys): Determined based on the response level to a human. ⁶Sitting position/recumbent position: At least either sitting position or recumbent position. ⁷Mildly depressed level of consciousness: No significantly depressed level of consciousness is observed, but a mild symptom of depressed level of consciousness is observed. ⁸Depressed level of consciousness: Significantly depressed level of consciousness is observed. | | |

The cerebral infarction model individuals of the groups were subjected to induction anesthesia through i.m. using ketamine hydrochloride in an amount of about 10 mg/kg body weight and maintenance anesthesia through isoflurane inhalation. Under continuous anesthesia, the hair on the head was shaved with clippers, and then each model individual was fixed with a brain stereotaxic apparatus. Next, the operation part was disinfected, and the skin of the head was cut open to expose the bregma with consideration for bleeding.

For each of the cerebral infarction model individuals of the groups, a micro syringe was placed in each of the four holes in total through which endothelin-1 was infused when the cerebral infarction model was produced in such a manner that the needle tip was at the same depth (D) as that for the infusion of endothelin-1. Ex1-L1-ND1 mRNA was administered to three individuals, and Ex1-L1-eGFP mRNA was administered to three individuals. The nucleic acid lipid nanoparticle were prepared at 0.3 mg/mL with the solvent (PBS or Tris buffer) and administered at 30 µL/site at an infusion rate of 1.5 µL/min. After the completion of the administration, the syringes were left still for about 20 minutes.

### (Observation of Motor Function)

The individuals of the ND1 nucleic acid lipid nanoparticle group and the control group in (Administration of ND1 Nucleic Acid Lipid Nanoparticle to Model) above were observed before the endothelin-1 infusion treatment (day 0) and on days 1, 7, 14, 21, 22, 28, 42, 56, 70, and 84 after the endothelin-1 infusion treatment based on the mRS. Here, the behaviors were evaluated every two weeks on and after day 28 after the endothelin-1 infusion treatment, and as the evaluation of the behaviors on day 28 after the endothelin-1 infusion (day 7 after the administration of the nucleic acid lipid nanoparticle), the individuals were evaluated on day 28 or 29 after the endothelin-1 infusion (day 7 or 8 after the administration of the nucleic acid lipid nanoparticle). The results were used in this Example as the results on day 28 after the endothelin-1 infusion (day 7 after the administration of the nucleic acid lipid nanoparticle).

As a result, in both of the ND1 nucleic acid lipid nanoparticle group and the control group, there was no difference in the motor function on day 1 after the administration, and similar motor dysfunction to that before the administration of the nucleic acid lipid nanoparticle was observed. In the control group, however, the motor function was not improved even on day 84 after the endothelin-1 infusion (day 63 after the administration of the nucleic acid lipid nanoparticle), while in the ND1 nucleic acid lipid nanoparticle group, the motor function was recovered on and after day 70 after the endothelin-1 infusion (day 49 after the administration of the nucleic acid lipid nanoparticle), and significant recovery of the motor function was observed on day 84 after the endothelin-1 infusion (day 63 after the administration of the nucleic acid lipid nanoparticle) (Fig. 1). This shows that administration of the ND1 nucleic acid lipid nanoparticle can recover motor dysfunction following brain damage.

From the above results, administration of ND1 nucleic acid lipid nanoparticle can treat cerebral infarction (especially, penetrating branch infarction, cerebral infarction having brain damage in the penetrating branch territory, subacute-phase to chronic-phase cerebral infarction, and cerebral infarction having motor dysfunction with high severity).

### Test Example 5: Action of ND1 Nucleic Acid Lipid Nanoparticle on Spinal Cord Injury Model

### (Evaluation Method)

The drug efficacy on spinal cord injury can be evaluated using a spinal cord injury model. The state of motor dysfunction of the spinal cord injury model can be evaluated using the Field Rating Scale as an indicator. The Field Rating Scale of Original open field rating scale (refer to PLoS ONE, 2011, vol. 6, 11, e27706) can be used with modification. When the individual cannot maintain the sitting position and has a total score of the Field Rating Scale of less than 10 points, the animal can be determined to have quadriplegia.

**[Table 7]**

| 1. Trunk Evaluation (Lower Limbs Score) |
|---|
| supine or prone position +0 |
| can roll-over from supine to prone position (reverse) +1 |
| keep sitting position +1 |
| can be in standing position (keep the position) +1 |

**[Table 8]**

| 2. Upper Limbs Evaluation |
|---|
| in prone position, anterior chest on the floor, no weight support of upper limbs/no movement +0 |
| in prone position, anterior chest not on the floor +1 |
| in prone position, weight support of upper limbs +1 |
| anterior chest lifted up from the floor with weight support of upper limbs, with dragging with upper limbs +1 |
| anterior chest lifted up from the floor with weight support of upper limbs, standing with lower limbs with hand clearance from the floor +1 |
| upper limb position in walking: below elbow +0 |
| upper limb position in walking: between shoulder and elbow +1 |
| upper limb position in walking: above elbow +1 |
| shoulder position: hands down/palms inverted +0 |
| shoulder position: not turned inward +1 |
| shoulder position: turned inward, without palm inversion +1 |
| without palm inversion (normal) +1 |
| cannot grasp a parallel stick +0 |
| grasp a parallel stick +1 |
| elevate a grasped parallel stick above head-height +1 |
| cannot grasp a perpendicular stick +0 |
| grasp a perpendicular stick +1 |
| grasp a perpendicular stick and elevate about 3 cm +1 |
| cannot grasp a stick above head-height +0 |
| grasp a stick above head-height +1 |
| drop upper limbs from the floor of the cage (cannot move upper limbs)^{*1} +0 |
| drop upper limbs from the floor of the cage up to elbow^{*2} +1 |
| drop upper limbs from the floor of the cage up to wrist^{*3} +1 |
| not drop hands from the floor of the cage^{*4} +1 |

**[Table 9]**

| 3. Lower Limbs Evaluation |
|---|
| cannot grasp a parallel stick +0 |
| grasp a parallel stick +1 |
| cannot grasp a perpendicular stick +0 |
| grasp a perpendicular stick +1 |
| drop lower limbs from the floor of the cage (cannot move lower limbs)^{*5} +0 |
| drop lower limbs from the floor of the cage up to knee^{*6} +1 |
| drop lower limbs from the floor of the cage up to ankle^{*7} +1 |
| not drop feet from the floor of the cage^{*8} +1 |
| cannot support body with lower limbs at all +0 |
| walk with lower limbs +1 |
| jump with lower limbs (once) +1 |
| jump with lower limbs (successively) +1 |
| Total Score (28 points) |

| |
|---|
| *1 Drop upper limbs from the floor of the cage (cannot move upper limbs): When the animal is placed in prone position in a cage with a lattice floor, the upper limbs drop through gaps in the bars of the cage floor and cannot be lifted. *2 Drop upper limbs from the floor of the cage up to elbow: When the animal is placed in prone position in the cage, the animal can lift the upper limbs up to elbow above the floor through gaps in the bars of the cage floor. *3 Drop upper limbs from the floor of the cage up to wrist: When the animal is placed in prone position in the cage, the animal can lift the upper limbs up to wrist above the floor through gaps in the bars of the cage floor. *4 Not drop hands from the floor of the cage: When the animal is placed in prone position in the cage, the animal can support the body without dropping the upper limbs through gaps in the bars of the cage floor. *5 Drop lower limbs from the floor of the cage (cannot move lower limbs): When the animal is placed in prone position in the cage, the lower limbs drop through gaps in the bars of the cage floor and cannot be lifted. *6 Drop lower limbs from the floor of the cage up to knee: When the animal is placed in prone position in the cage, the animal can lift the lower limbs up to knee above the floor through gaps in the bars of the cage floor. *7 Drop lower limbs from the floor of the cage up to ankle: When the animal is placed in prone position in the cage, the animal can lift the lower limbs up to ankle above the floor through gaps in the bars of the cage floor. *8 Not drop feet from the floor of the cage: When the animal is placed in prone position in the cage, the animal can support the body without dropping the lower limbs through gaps in the bars of the cage floor. |

### (Production of Spinal Cord Injury Model)

A common marmoset is subjected to induction anesthesia with ketamine hydrochloride and xylazine. The marmoset under anesthesia is intratracheally intubated using an Atom nutrition catheter (6Fr), which is an endotracheal tube, and subjected to maintenance anesthesia by inhalation of isoflurane. The hair on the back of the marmoset under anesthesia is shaved, and the marmoset is kept in a prone position on a thermal mat at 37°C. The skin on the neck is cut open, and the vertebrae are exposed by removing connective tissue. The vertebral arches including the spine of the vertebra C5 are cut, and the spinal cord (cervical spinal cord) is exposed.

Using a 2.5-mm round tip as a tip attached to the impactor, the spinal cord is crushed from both right and left sides under the crushing conditions of applying pressure of 280 kdyn for several seconds to the parts on both right and left sides of the spinal cord midline on the surface of the cervical spinal cord on the back side at the part exposed by cutting the vertebral arches of the vertebra C5.

After the crush injury, to prevent a contracture or spasticity from occurring, the jointsthe fingers, the wrists (ankles), the elbows (knees), and the shoulders (hip joints) of the left and right upper and lower limbs of the common marmoset are bent and stretched several times each (bending and stretching exercise) as rehabilitation. The rehabilitation is started on day 5 to day 7 after the crush injury and conducted once or twice a day every day during the observation period.

### (Administration of Nucleic Acid Lipid Nanoparticle to Injured Area)

The motor dysfunction of common marmosets after crush injury is evaluated with the Field Rating Scale, and the individuals which cannot keep sitting position several weeks after the crush injury and which have a total Field Rating Scale score of less than 10 points are divided into a control group and a drug administration group in such a manner that there is no difference in the scores between the groups.

After the crush injury, under induction anesthesia with ketamine hydrochloride and xylazine and maintenance anesthesia through isoflurane inhalation, the crushed site is exposed again, and nucleic acid lipid nanoparticle are administered using a syringe connected to a microinjector to the center of the pressurized site on the left of the spinal cord to a depth of about 1 mm from the surface of the cervical spinal cord on the back side. Next, the nucleic acid lipid nanoparticle are administered similarly to the center of the pressurized site on the right of the spinal cord. The syringe is left still for several minutes after the administration, and then the muscles and the skin are stitched together. The nucleic acid lipid nanoparticleolution is administered to several individuals of the drug administration group, and eGFP mRNA is administered to several individuals of the control group.

The motor dysfunction is evaluated with the Field Rating Scale, and the grip strength of both upper limbs is measured at the same time. The grip strength is measured using a device obtained by attaching a basket which marmosets can easily grasp with both upper limbs to a digital force meter (also called a grip test below). Specifically, a marmoset is caused to grasp the basket, and then a tester pulls the marmoset until the marmoset stops grasping the basket. The strength at which the marmoset grasps the basket is measured with the digital force meter, and the measured value is regarded as the grip strength of the marmoset. The scoring of the motor dysfunction and the grip test are conducted at the same timing, and observation and evaluation are conducted for several weeks after the drug administration.

### [Examples]

The production method of the compound of the formula (I) or a salt thereof will be explained in further detail below based on Examples.

In this regard, the invention is not limited to the compounds described in the Examples. The production methods of raw material compounds are also shown in Production Examples. The production method of the compound of the formula (I) or a salt thereof is not limited only to the production methods of specific Examples, and the compound of the formula (I) or a salt thereof can also be produced by a combination of the production methods or a method that is obvious to one skilled in the art.

The abbreviations below are sometimes used in this specification, Examples, Production Examples, and the tables.

CDI: 1,1'-carbonyldiimidazole, CPME: cyclopentyl methyl ether, DCM: dichloromethane, DIPEA: *N,N*-diisopropylethylamine, DMAP: 4-(dimethylamino)pyridine, DMF: *N,N*-dimethylformamide, DMSO: dimethyl sulfoxide, DPPA: diphenylphosphoryl azide, EDCI·HCl: 1-ethyl-3-(3-dimethylaminopropyl)carbodiimide hydrochloride, EtOH: ethanol, HATU: 1-[bis(dimethylamino)methylene]-1*H*-1,2,3-triazolo[4,5-b]pyridinium 3-oxide hexafluorophosphate, HOBt: 1-hydroxybenzotriazole, MeCN: acetonitrile, TEA: triethylamine, TFA: trifluoroacetic acid, NUM: Example number or Production Example number (/HCl means that the Example or the Production Example is a hydrochloride), REF: the Production Example number or the Example number referred to for the production method, PEx: Production Example number, Ex: Example number, STR: chemical structural formula, DAT: physicochemical data, NMR: chemical shift δ value ppm of 500 MHz ¹H-NMR (The material in the parentheses in NMR shows the measurement solvent, and for example, (CDCl₃) means the value measured with deuterated chloroform. NMR signal shows a typical signal.) s: singlet, t: triplet, m: multiplet, br: broad, ESI+: m/z value in ESI-MS+, CI+: m/z value in CI-MS+, HPLC Rt: retention time (minute) in high-performance liquid chromatography (measured using CHIRAL ART Amylose-SA 250 mm × 4.6 mm I.D., S-3 µm and 2-propanol solution of 10 mM ammonium acetate as a mobile phase at a column temperature of 12°C, and flow rate of 0.25 mL/min with a Corona (registered trademark) Charged Aerosol Detector (CAD)). In this specification, a compound is sometimes named using naming software, such as ACD/Name (registered trademark, Advanced Chemistry Development, Inc.). For the purpose of convenience, the concentration mol/L is shown as M. For example, 1 M aqueous sodium hydroxide solution means an aqueous sodium hydroxide solution of 1 mol/L.

### Production Example 1-1

After 8-bromooctanoic acid (2.97 g), DIPEA (4.7 mL), HATU (5.4 g), and DMAP (135 mg) were added to a mixture of 2-nonylundecan-1-ol (3.25 g) and DCM (40 mL) in a water bath, the mixture was stirred at room temperature for 6.5 hours. After chloroform and water were added to the reaction mixture, the organic layer was separated, and the aqueous layer was subjected to extraction with chloroform. The combined organic layer was washed with saturated brine, dried over anhydrous magnesium sulfate and then concentrated under reduced pressure. The obtained residue was purified by silica gel column chromatography (hexane/ethyl acetate) to obtain 2-nonylundecyl 8-bromooctanoate (5.27 g) as an oil.

### Production Example 1-2

To a mixture of 2-octyldecan-1-ol (500 mg) in DCM (5 mL), {(1*r*,3*r*)-3-[(*tert-*butoxycarbonyl)amino]cyclobutyl}acetic acid (511 mg), DMAP (24 mg), DIPEA (798 µL), and HATU (918 mg) were added at room temperature, and the mixture was stirred at room temperature for six hours. Chloroform and water were added to the reaction mixture, and the organic layer was separated. The aqueous layer was subjected to extraction with chloroform. The combined organic layer was washed with saturated brine, dried over anhydrous magnesium sulfate and then concentrated under reduced pressure. The obtained residue was purified by silica gel column chromatography (hexane/ethyl acetate) to obtain 2-octyldecyl {(1*r*,3*r*)-3-[(*tert*-butoxycarbonyl)amino]cyclobutyl}acetate (879 mg) as an oil

### Production Example 1-3

After TFA (1.5 mL) was added to a mixture of 2-octyldecyl {(1*r*,3*r*)-3-[(*tert-*butoxycarbonyl)amino]cyclobutyl}acetate (877 mg) in DCM (8 mL) at room temperature, the mixture was stirred at room temperature for six hours. After the reaction mixture was added to a mixture of chloroform and aqueous saturated sodium hydrogen carbonate solution, the organic layer was separated, and the aqueous layer was subjected to extraction with chloroform. The combined organic layer was dried over anhydrous sodium sulfate and then concentrated under reduced pressure to obtain 2-octyldecyl [(1*r*,3*r*)-3-aminocyclobutyl]acetate (661 mg) as an oil.

### Production Example 1-4

After a mixture of 2-nonylundecyl 8-bromooctanoate (430 mg) and CPME (4 mL); DIPEA (731 µL); and KI (30 mg); were added to a mixture of 2-octyldecyl [(1*r*,3*r*)-3-aminocyclobutyl]acetate (660 mg) in MeCN (4 mL) at room temperature, the mixture was stirred in an oil bath at 80°C for 48 hours. After the reaction mixture was added to a mixture of chloroform and aqueous saturated sodium hydrogen carbonate solution, the organic layer was separated, and the aqueous layer was subjected to extraction with chloroform. The combined organic layer was dried over anhydrous sodium sulfate and then concentrated under reduced pressure. After the obtained residue was purified by amino silica gel column chromatography (hexane/ethyl acetate), the crudely purified product was purified by silica gel column chromatography (chloroform/methanol) to obtain 2-nonylundecyl 8-{[(1*r*,3*r*)-3-{2-[(2-octyldecyl)oxy]-2-oxoethyl}cyclobutyl]amino}octanoate (452 mg) as an oil.

### Example 1

To a mixture of 2-nonylundecyl 8-{[(1*r*,3*r*)-3-{2-[(2-octyldecyl)oxy]-2-oxoethyl}cyclobutyl]amino}octanoate (1200 mg) and DCM (24 mL), 1-methyl-L-proline (water content of 11.8%, 290 mg), HATU (910 mg), DMAP (27 mg), and DIPEA (510 µL) were added at room temperature, and the mixture was stirred at room temperature for four hours. Water was added to the reaction mixture, and the organic layer was separated and concentrated under reduced pressure. Heptane and aqueous 90% methanol solution were added to the obtained residue, and the heptane layer was separated and concentrated under reduced pressure. After the obtained residue was purified by silica gel column chromatography (heptane/ethyl acetate), the crudely purified product was purified by amino silica gel column chromatography (heptane/ethyl acetate) to obtain 2-nonylundecyl 8-{(1-methyl-L-prolyl)[(1*r*,3*S*)-3-{2-[(2-octyldecyl)oxy]-2-oxoethyl}cyclobutyl]amino}octanoate (1.2 g) as an oil.

### Example 2

To a mixture of 2-nonylundecyl 8-{[(1*r*,3*r*)-3-{2-[(2-octyldecyl)oxy]-2-oxoethyl}cyclobutyl]amino}octanoate (120 mg) and DCM (3 mL), 1-methyl-D-proline monohydrate (26 mg), HATU (68 mg), and DIPEA (0.038 mL) were added, and the mixture was stirred at room temperature for 15 hours. After the reaction mixture was purified by silica gel column chromatography (chloroform/methanol), the crudely purified product was purified by amino silica gel column chromatography (hexane/ethyl acetate) to obtain 2-nonylundecyl 8-{(1-methyl-D-prolyl)[(1*r*,3*R*)-3-{2-[(2-octyldecyl)oxy]-2-oxoethyl}cyclobutyl]amino}octanoate (129 mg) as an oil.

### Production Example 4-1

To a mixture of 2-octyldecan-1-ol (620 mg) and DCM (20 mL), {3-[(*tert-*butoxycarbonyl)amino]bicyclo[1.1.1]pentan-1-yl}acetic acid (500 mg), HATU (950 mg), DIPEA (0.54 mL), and DMAP (25 mg) were added, and the mixture was stirred at room temperature for 40 hours. The reaction mixture was diluted with ethyl acetate and washed with aqueous saturated sodium hydrogen carbonate solution. The organic layer was dried over anhydrous sodium sulfate and then concentrated under reduced pressure. The obtained residue was purified by silica gel column chromatography (hexane/ethyl acetate) to obtain 2-octyldecyl {3-[(*tert*-butoxycarbonyl)amino]bicyclo[1.1.1]pentan-1-yl}acetate (900 mg) as an oil.

### Production Example 4-2

After TFA (1.5 mL) was added to a mixture of 2-octyldecyl {3-[(*tert-*butoxycarbonyl)amino]bicyclo[1.1.1]pentan-1-yl}acetate (900 mg) and DCM (8 mL) under ice cooling, the mixture was stirred at room temperature for two hours. The reaction mixture was diluted with chloroform and then neutralized by adding aqueous saturated sodium hydrogen carbonate solution under ice cooling. After extraction with chloroform, the organic layer was dried over anhydrous sodium sulfate and then concentrated under reduced pressure. The obtained residue was purified by silica gel column chromatography (chloroform/methanol) to obtain 2-octyldecyl (3-aminobicyclo[1.1.1]pentan-1-yl)acetate (685 mg) as an oil.

### Production Example 4-3

To a mixture of 2-octyldecyl (3-aminobicyclo[1.1.1]pentan-1-yl)acetate (680 mg) and MeCN (6 mL), 2-nonylundecyl 8-bromooctanoate (450 mg), CPME (6 mL), DIPEA (0.4 mL), and KI (15 mg) were added, and the mixture was stirred under argon atmosphere in an oil bath at 80°C for 3.5 days. The reaction mixture was left to cool, then diluted with ethyl acetate and washed with water. The organic layer was dried over anhydrous sodium sulfate and then concentrated under reduced pressure. The obtained residue was purified by silica gel column chromatography (hexane/ethyl acetate) to obtain 2-nonylundecyl 8-[(3-{2-[(2-octyldecyl)oxy]-2-oxoethyl}bicyclo[1.1.1]pentan-1-yl)amino]octanoate (482 mg) as an oil.

### Example 4

To a mixture of 2-nonylundecyl 8-[(3-{2-[(2-octyldecyl)oxy]-2-oxoethyl}bicyclo[1.1.1]pentan-1-yl)amino]octanoate (150 mg) and DCM (3 mL), 1-methyl-L-proline (water content of 11.8%, 33 mg), HATU (84 mg), and DIPEA (0.031 mL) were added, and the mixture was stirred at room temperature for 24 hours. After the reaction mixture was purified by silica gel column chromatography (chloroform/methanol), the crudely purified product was purified by amino silica gel column chromatography (hexane/ethyl acetate) to obtain 2-nonylundecyl 8-[(1-methyl-L-prolyl)(3-{2-[(2-octyldecyl)oxy]-2-oxoethyl}bicyclo[1.1.1]pentan-1-yl)amino]octanoate (128 mg) as an oil.

### Production Example 5-1

To a mixture of (9*Z*,12*Z*)-octadeca-9,12-dien-1-ol (1.5 mL) and DCM (15 mL), DIPEA (1.7 mL) was added at room temperature, and chloroacetyl chloride (0.58 mL) was added dropwise under ice cooling. The mixture was stirred at room temperature for an hour. Water was added to the reaction mixture, and the separated aqueous layer was subjected to extraction with chloroform. The combined organic layer was washed with saturated brine, dried over anhydrous sodium sulfate and then concentrated under reduced pressure. The obtained residue was purified by silica gel column chromatography (hexane/ethyl acetate) to obtain (9*Z*,12*Z*)-octadeca-9,12-dien-1-yl chloroacetate (1.47 g) as an oil.

### Example 7

To a mixture of 2-nonylundecyl 8-{[(1*r*,3*r*)-3-{2-[(2-octyldecyl)oxy]-2-oxoethyl}cyclobutyl]amino}octanoate (120 mg) and DCM (3 mL)*, N,N-*diethyl-β-alanine (26 mg), HATU (68 mg), and DIPEA (0.038 mL) were added, and the mixture was stirred at room temperature for 18 hours. After the reaction mixture was purified by silica gel column chromatography (chloroform/methanol), the crudely purified product was purified by amino silica gel column chromatography (hexane/ethyl acetate/methanol) to obtain 2-nonylundecyl 8-{(*N,N*-diethyl-β-alanyl)[(1*r*,3*r*)-3-{2-[(2-octyldecyl)oxy]-2-oxoethyl}cyclobutyl]amino}octanoate (119 mg) as an oil.

### Production Example 9-1

To a mixture of *tert*-butyl azetidine-3-carboxylate monohydrochloride (500 mg), *N,N*-dimethylglycine (320 mg), HATU (1.18 g), and DCM (10 mL), DIPEA (1.1 mL) was added, and the mixture was stirred at room temperature for 18 hours. Aqueous saturated sodium hydrogen carbonate solution was added to the reaction mixture, and the separated organic layer was washed with saturated brine, dried over anhydrous sodium sulfate and then concentrated under reduced pressure. After the obtained residue was purified by silica gel column chromatography (chloroform/methanol/28% ammonia water), the crudely purified product was purified by amino silica gel column chromatography (hexane/ethyl acetate/methanol) to obtain *tert*-butyl 1-(*N,N*-dimethylglycyl)azetidine-3-carboxylate (350 mg) as an oil.

### Production Example 9-2

To a mixture of *tert*-butyl 1-(*N,N*-dimethylglycyl)azetidine-3-carboxylate (350 mg) and DCM (2 mL), TFA (2.2 mL) was added at room temperature, and the mixture was stirred at room temperature for 18 hours. To the reaction mixture, TFA (1.1 mL) was added, and after the mixture was stirred at room temperature for two hours, the reaction mixture was concentrated under reduced pressure. An operation of adding 4 M hydrogen chloride/1,4-dioxane solution (5 mL) to the obtained residue and concentrating under reduced pressure was conducted four times to obtain 1-(*N,N*-dimethylglycyl)azetidine-3-carboxylic acid hydrochloride (492 mg) as an oily crude material.

### Production Example 12-1

To a mixture of methyl 1,4-diazepane-6-carboxylate hydrochloride (491 mg) and MeCN (10 mL), DIPEA (1.82 mL) and ethyl iodide (0.51 mL) were added at room temperature, and the mixture was stirred in an oil bath at 60°C for 10 hours. The reaction mixture was left to cool to room temperature and then concentrated under reduced pressure, and ethyl acetate was added to the residue. After the mixture was stirred at room temperature for 15 minutes, the insoluble matter was removed by filtration. The filtrate was concentrated under reduced pressure. After the obtained residue was purified by amino silica gel column chromatography (hexane/ethyl acetate/methanol), the crudely purified product was purified by silica gel column chromatography (chloroform/methanol) to obtain methyl 1,4-diethyl-1,4-diazepane-6-carboxylate (167 mg) as an oil.

### Example 12

Aqueous 1 M sodium hydroxide solution (0.25 mL) was added to a mixture of methyl 1,4-diethyl-1,4-diazepane-6-carboxylate (35 mg) and methanol (2.5 mL) at room temperature, and the mixture was stirred at room temperature for 10 hours. To the reaction mixture, 1 M hydrochloric acid (0.25 mL) was added, and the mixture was concentrated under reduced pressure. An operation of adding methanol/chloroform to the residue and concentrating under reduced pressure was conducted three times. HATU (62 mg); a mixture of 2-nonylundecyl 8-{[(1*r*,3*r*)-3-{2-[(2-octyldecyl)oxy]-2-oxoethyl}cyclobutyl]amino}octanoate (110 mg) and DCM (3 mL); and DIPEA (0.035 mL); were added to the obtained residue, and the mixture was stirred at room temperature for 10 hours. After the reaction mixture was purified by silica gel column chromatography (chloroform/methanol), the crudely purified product was purified by amino silica gel column chromatography (hexane/ethyl acetate/methanol) to obtain 2-nonylundecyl 8-{(1,4-diethyl-1,4-diazepane-6-carbonyl)[(1*r*,3*r*)-3-{2-[(2-octyldecyl)oxy]-2-oxoethyl}cyclobutyl]amino}octanoate (74 mg) as an oil.

### Production Example 14-1

To a mixture of *tert*-butyl [(1*r*,3*r*)-3-(hydroxymethyl)cyclobutyl]carbamate (500 mg) and DCM (30 mL), (9*Z*,12*Z*)-octadeca-9,12-dienoic acid (0.81 mL), DIPEA (0.64 mL), EDCI·HCl (0.72 g), and DMAP (61 mg) were added, and the mixture was stirred at room temperature for 18 hours. The reaction mixture was diluted with DCM and washed with brine, and then the organic layer was dried over anhydrous sodium sulfate and concentrated under reduced pressure. The obtained residue was purified by silica gel column chromatography (hexane/ethyl acetate) to obtain {(1*r*,3*r*)-3-[(*tert*-butoxycarbonyl)amino]cyclobutyl}methyl (9*Z*,12*Z*)-octadeca-9,12-dienoate (1.06 g) as an oil.

### Production Example 16-1

A mixture of 2-octyldecyl [(1*r*,3*r*)-3-aminocyclobutyl]acetate (1.06 g) and CPME (8 mL); and DIPEA (0.6 mL); were added to a mixture of [(2-bromoethoxy)methyl]benzene (300 mg) and MeCN (8 mL) at room temperature, and the mixture was stirred in an oil bath at 80°C for 40 hours. After the reaction mixture was left to cool to room temperature, ethyl acetate and aqueous saturated sodium hydrogen carbonate solution were added to the reaction mixture, and the separated organic layer was washed with saturated brine, dried over anhydrous sodium sulfate and then concentrated under reduced pressure. The obtained residue was purified by amino silica gel column chromatography (ethyl acetate/hexane) to obtain 2-octyldecyl [(1*r*,3*r*)-3-{[2-(benzyloxy)ethyl]amino}cyclobutyl]acetate (474 mg) as an oil.

### Production Example 16-3

Palladium on activated carbon (Pd 10%) (26 mg) was added to a mixture of 2-octyldecyl [(1*S*,3*r*)-3-{[2-(benzyloxy)ethyl](1-methyl-L-prolyl)amino}cyclobutyl]acetate (263 mg) and 2-propanol (5 mL) under argon atmosphere at room temperature, and the mixture was stirred under hydrogen atmosphere at room temperature over a weekend. Palladium on activated carbon (Pd 10%) (26 mg) was added to the reaction mixture under argon atmosphere at room temperature, and the mixture was stirred under hydrogen atmosphere (3 atm) at room temperature for 24 hours. Palladium on activated carbon (Pd 10%) (26 mg) and acetic acid (0.072 mL) were added to the reaction mixture under argon atmosphere at room temperature, and the mixture was stirred under hydrogen atmosphere (3 atm) at room temperature for 24 hours. Palladium on activated carbon (Pd 10%) (26 mg) was added to the reaction mixture under argon atmosphere at room temperature, and the mixture was stirred under hydrogen atmosphere (3 atm) at room temperature for 24 hours. The reaction mixture was filtered through celite (registered trademark), and the filtrate was concentrated under reduced pressure. Ethyl acetate and aqueous saturated sodium hydrogen carbonate solution were added to the obtained residue, and the organic layer was separated, washed with saturated brine, dried over anhydrous sodium sulfate and then concentrated under reduced pressure. The obtained residue was purified by silica gel column chromatography (chloroform/methanol) to obtain 2-octyldecyl {(1*S*,3*r*)-3-[(2-hydroxyethyl)(1-methyl-L-prolyl)amino]cyclobutyl}acetate (140 mg) as an oil.

### Example 16

To a mixture of (9*Z*,12*Z*)-octadeca-9,12-dienoic acid (0.053 mL) and DCM (1 mL), HATU (64 mg); DIPEA (0.045 mL); a mixture of 2-octyldecyl {(1*S*,3*r*)-3-[(2-hydroxyethyl)(1-methyl-L-prolyl)amino]cyclobutyl}acetate (70 mg) and DCM (1 mL); and DMAP (1.6 mg); were added at room temperature, and the mixture was stirred at room temperature for 18 hours. After the reaction mixture was purified by silica gel column chromatography (chloroform/methanol), the crudely purified product was purified by amino silica gel column chromatography (hexane/ethyl acetate/methanol) to obtain 2-{(1-methyl-L-prolyl)[(1*r*,3*S*)-3-{2-[(2-octyldecyl)oxy]-2-oxoethyl}cyclobutyl]amino}ethyl (9*Z*,12*Z*)-octadeca-9,12-dienoate (78 mg) as an oil.

### Example 21

To a mixture of 2-nonylundecyl 8-{[(1*r*,3*r*)-3-{2-[(2-octyldecyl)oxy]-2-oxoethyl}cyclobutyl]amino}octanoate (151 mg) and DCM (3 mL), (4-methylpiperazin-1-yl)acetic acid (39.4 mg), DIPEA (0.096 mL), and HATU (92.7 mg) were added at room temperature, and the mixture was stirred at room temperature for three hours. Aqueous saturated sodium hydrogen carbonate solution was added to the reaction mixture, and the aqueous layer was subjected to extraction with chloroform. The organic layer was washed with saturated brine, dried over anhydrous magnesium sulfate and then concentrated under reduced pressure. The obtained residue was purified by amino silica gel column chromatography (hexane/ethyl acetate) to obtain 2-nonylundecyl 8-{[(4-methylpiperazin-1-yl)acetyl][(1*r*,3*r*)-3-{2-[(2-octyldecyl)oxy]-2-oxoethyl}cyclobutyl]amino}octanoate (132 mg) as an oil.

### Production Example 22-2

After TFA (3 mL) was added to a mixture of *tert*-butyl 4-({8-[(2-nonylundecyl)oxy]-8-oxooctyl}[(1*r*,3*r*)-3-{2-[(2-octyldecyl)oxy]-2-oxoethyl}cyclobutyl]carbamoyl)piperidine-1-carboxylate (614 mg) and DCM (6 mL) under ice cooling, the mixture was stirred at room temperature for two hours. The reaction mixture was diluted with chloroform, made basic by adding aqueous saturated sodium hydrogen carbonate solution under ice cooling and then subjected to extraction with chloroform. The organic layer was dried over anhydrous magnesium sulfate and then concentrated under reduced pressure, and the obtained residue was purified by amino silica gel column chromatography (hexane/ethyl acetate) to obtain 2-nonylundecyl 8-{[(1*r*,3*r*)-3-{2-[(2-octyldecyl)oxy]-2-oxoethyl}cyclobutyl](piperidine-4-carbonyl)amino}octanoate (521 mg) as an oil.

### Example 22

Sodium triacetoxyborohydride (131 mg) was added to a mixture of 2-nonylundecyl 8-{[(1*r*,3*r*)-3-{2-[(2-octyldecyl)oxy]-2-oxoethyl}cyclobutyl](piperidine-4-carbonyl)amino}octanoate (165 mg) and DCM (5 mL) at room temperature, and the mixture was stirred at room temperature for 10 minutes. Acetaldehyde (0.1 mL) was added to the reaction mixture under ice cooling, and the mixture was stirred at room temperature for 17 hours. Aqueous saturated sodium hydrogen carbonate solution was added to the reaction mixture, and after extraction with chloroform, the organic layer was washed with saturated brine, then dried over anhydrous magnesium sulfate and concentrated under reduced pressure. After the obtained residue was purified by silica gel column chromatography (chloroform/methanol), the crudely purified product was purified by amino silica gel column chromatography (hexane/ethyl acetate) to obtain 2-nonylundecyl 8-{(1-ethylpiperidine-4-carbonyl)[(1*r*,3*r*)-3-{2-[(2-octyldecyl)oxy]-2-oxoethyl}cyclobutyl]amino}octanoate (139 mg) as an oil.

### Production Example 24-1

Pyridine (6 mL), 4-nitrophenyl chloroformate (2.98 g), and DMAP (45 mg) were added to a mixture of 2-octyldecan-1-ol (2 g) and DCM (60 mL) under ice cooling, and the mixture was stirred at room temperature for 68 hours. The reaction mixture was concentrated under reduced pressure, and hexane was added to the obtained residue. The insoluble matter was separated by filtration. The filtrate was concentrated under reduced pressure, and 4-nitrophenyl 2-octyldecyl carbonate (2.96 g) was obtained as an oil.

### Production Example 24-2

Pyridine (5 mL), *tert*-butyl [(1*r*,3*r*)-3-hydroxycyclobutyl]carbamate (1.81 g), and DMAP (80 mg) were added to a mixture of 4-nitrophenyl 2-octyldecyl carbonate (1.4 g) and DCM (40 mL), and the mixture was stirred at room temperature for two days. The reaction mixture was concentrated under reduced pressure, then diluted with ethyl acetate/hexane and washed with aqueous saturated sodium hydrogen carbonate solution, water, and saturated brine. The organic layer was dried over anhydrous sodium sulfate and then concentrated under reduced pressure, The obtained residue was purified by silica gel column chromatography (hexane/ethyl acetate) to obtain (1*r*,3*r*)-3-[(*tert*-butoxycarbonyl)amino]cyclobutyl 2-octyldecyl carbonate (1.33 g) as an oil.

### Production Example 25-1

Pyridine (5 mL), *tert*-butyl [(1*r*,3*r*)-3-(hydroxymethyl)cyclobutyl]carbamate (1.95 g), and DMAP (80 mg) were added to a mixture of 4-nitrophenyl 2-octyldecyl carbonate (1.4 g) and DCM (40 mL), and the mixture was stirred at room temperature for two days. The reaction mixture was concentrated under reduced pressure, then diluted with ethyl acetate/hexane and washed with aqueous saturated sodium hydrogen carbonate solution, water, and saturated brine. The organic layer was dried over anhydrous sodium sulfate and then concentrated under reduced pressure. The obtained residue was purified by silica gel column chromatography (hexane/ethyl acetate) to obtain {(1*r*,3*r*)-3-[(*tert-*butoxycarbonyl)amino]cyclobutyl}methyl 2-octyldecyl carbonate (1.18 g) as an oil.

### Production Example 25-2

After TFA (5 mL) was added to a mixture of {(1*r*,3*r*)-3-[(*tert-*butoxycarbonyl)amino]cyclobutyl}methyl 2-octyldecyl carbonate (1.18 g) and DCM (10 mL) under ice cooling, the mixture was stirred at room temperature for two hours. The reaction mixture was neutralized by adding aqueous saturated sodium hydrogen carbonate solution and then subjected to extraction with chloroform. The organic layer was dried over anhydrous sodium sulfate and then concentrated under reduced pressure. The obtained residue was purified by amino silica gel column chromatography (hexane/ethyl acetate) to obtain [(1*r*,3*r*)-3-aminocyclobutyl]methyl 2-octyldecyl carbonate (883 mg) as an oil.

### Production Example 25-3

To a mixture of [(1*r*,3*r*)-3-aminocyclobutyl]methyl 2-octyldecyl carbonate (882 mg) and MeCN (8 mL), 2-nonylundecyl 8-bromooctanoate (560 mg), CPME (8 mL), DIPEA (0.5 mL), and KI (20 mg) were added under argon atmosphere, and the mixture was stirred in an oil bath at 80°C for 2.5 days. The reaction mixture was left to cool, then diluted with ethyl acetate and washed with aqueous saturated sodium hydrogen carbonate solution and saturated brine. The organic layer was dried over anhydrous sodium sulfate and then concentrated under reduced pressure. The obtained residue was purified by amino silica gel column chromatography (hexane/ethyl acetate) to obtain 2-nonylundecyl 8-({(1*r*,3*r*)-3-[({[(2-octyldecyl)oxy]carbonyl}oxy)methyl]cyclobutyl}amino)octanoate (630 mg) as an oil.

### Example 25

To a mixture of 2-nonylundecyl 8-({(1*r*,3*r*)-3-[({[(2-octyldecyl)oxy]carbonyl}oxy)methyl]cyclobutyl}amino)octanoate (160 mg) and DCM (3 mL), 1-methyl-L-proline monohydrate (35 mg), HATU (90 mg), and DIPEA (70 µL) were added, and the mixture was stirred at room temperature for 14 hours. The reaction mixture was diluted with ethyl acetate/hexane (1/1) and then washed with aqueous saturated sodium hydrogen carbonate solution, water, and saturated brine. The organic layer was dried over anhydrous sodium sulfate and then concentrated under reduced pressure. After the obtained residue was purified by silica gel column chromatography (chloroform/methanol), the crudely purified product was purified by amino silica gel column chromatography (hexane/ethyl acetate) to obtain 2-nonylundecyl 8-[(1-methyl-L-prolyl){(1*r*,3*S*)-3-[({[(2-octyldecyl)oxy]carbonyl}oxy)methyl]cyclobutyl}amino]octanoate (147 mg) as an oil.

### Example 26

To a mixture of 3-chloropropionyl chloride (360 mg), DIPEA (1 mL), and DCM (15 mL), 2-nonylundecyl 8-{[(1*r*,3*r*)-3-{2-[(2-octyldecyl)oxy]-2-oxoethyl}cyclobutyl]amino}octanoate (750 mg) was added at room temperature, and the mixture was stirred at room temperature for two hours. Heptane and aqueous 90% methanol solution were added to the reaction mixture, and the heptane layer was separated, washed with aqueous 90% methanol solution and then concentrated under reduced pressure to obtain 2-nonylundecyl 8-{3-chloro-*N*-[(1*r*,3*r*)-3-{2-[(2-octyldecyl)oxy]-2-oxoethyl}cyclobutyl]propanamido}octanoate (850 mg) as an oily crude material. To a mixture of diethanolamine (0.61 g) and EtOH (3 mL), 2-nonylundecyl 8-{3-chloro-*N*-[(1*r*,3*r*)-3-{2-[(2-octyldecyl)oxy]-2-oxoethyl}cyclobutyl]propanamido}octanoate (150 mg) was added at room temperature, and the mixture was stirred at room temperature for 20 hours. The reaction mixture was further stirred in an oil bath at 55°C for five hours and then left to cool to room temperature. Heptane and water were added to the reaction mixture, and the organic layer was separated, washed with aqueous 90% methanol solution and then concentrated under reduced pressure. The obtained residue was purified by amino silica gel column chromatography (heptane/ethyl acetate) to obtain 2-nonylundecyl 8-{[*N,N*-bis(2-hydroxyethyl)-β-alanyl][(1*r*,3*r*)-3-{2-[(2-octyldecyl)oxy]-2-oxoethyl}cyclobutyl]amino}octanoate (100 mg) as an oil.

### Example 28

To a mixture of glycinamide hydrochloride (520 mg), DIPEA (2 mL), and EtOH (2 mL), 2-nonylundecyl 8-{3-chloro-*N*-[(1*r*,3*r*)-3-{2-[(2-octyldecyl)oxy]-2-oxoethyl}cyclobutyl]propanamido}octanoate (120 mg), which was the intermediate of Example 26, was added at room temperature, and the mixture was stirred at room temperature for two hours. The reaction mixture was further stirred in an oil bath at 55°C for 17 hours, then stirred in an oil bath at 75°C for two hours and left to cool to room temperature. Heptane and water were added to the reaction mixture, and the organic layer was separated, washed with aqueous 90% methanol solution and then concentrated under reduced pressure. The obtained residue was purified by amino silica gel column chromatography (heptane/ethyl acetate) to obtain 2-nonylundecyl 8-{[*N*-(2-amino-2-oxoethyl)-β-alanyl][(1*r*,3*r*)-3-{2-[(2-octyldecyl)oxy]-2-oxoethyl}cyclobutyl]amino}octanoate (45 mg) as an oil.

### Production Example 30-1

To a mixture of 2-heptylnonan-1-ol (501 mg) and DCM (10 mL), 8-bromooctanoic acid (554 mg), DIPEA (0.891 mL), HATU (1.01 g), and DMAP (27.2 mg) were added in a water bath, and the mixture was stirred at room temperature for 6.5 hours. Chloroform and water were added to the reaction mixture, and then the separated aqueous layer was subjected to extraction with chloroform. The combined organic layer was dried over anhydrous magnesium sulfate and then concentrated under reduced pressure. The obtained residue was purified by silica gel column chromatography (hexane/ethyl acetate) to obtain 2-heptylnonyl 8-bromooctanoate (0.787 g) as an oil.

### Production Example 30-2

To a mixture of 2-heptylnonan-1-ol (508 mg) and DCM (20 mL), (1*r*,4*r*)-4-[(*tert-*butoxycarbonyl)amino]cyclohexane-1-carboxylic acid (767 mg), DIPEA (0.898 mL), HATU (1.19 g), and DMAP (26.6 mg) were added in a water bath, and the mixture was stirred at room temperature for 24 hours. Chloroform and water were added to the reaction mixture, and the separated aqueous layer was subjected to extraction with chloroform. The combined organic layer was dried over anhydrous magnesium sulfate and then concentrated under reduced pressure. The obtained residue was purified by silica gel column chromatography (hexane/ethyl acetate) to obtain 2-heptylnonyl (1*r*,4*r*)-4-[(*tert-*butoxycarbonyl)amino]cyclohexane-1-carboxylate (0.761 g) as an oil.

### Production Example 30-3

After DCM (10 mL) and TFA (1.3 mL) were added to 2-heptylnonyl (1*r*,4*r*)-4-[(*tert*-butoxycarbonyl)amino]cyclohexane-1-carboxylate (0.76 g) at room temperature, the reaction mixture was stirred at room temperature for four hours. The reaction mixture was diluted with chloroform and then neutralized with aqueous saturated sodium hydrogen carbonate solution, and the separated aqueous layer was subjected to extraction with chloroform. The combined organic layer was dried over anhydrous sodium sulfate and then concentrated under reduced pressure. The obtained residue was purified by amino silica gel column chromatography (hexane/ethyl acetate) to obtain 2-heptylnonyl (1*r*,4*r*)-4-aminocyclohexane-1-carboxylate (570 mg) as an oil.

### Production Example 30-4

CPME (4 mL); MeCN (5 mL); DIPEA (0.26 mL); KI (24.5 mg); and a mixture of 2-heptylnonyl 8-bromooctanoate (331 mg) and CPME (1 mL); were added to 2-heptylnonyl (1*r*,4*r*)-4-aminocyclohexane-1-carboxylate (570 mg) at room temperature, and the mixture was stirred in an oil bath at 80°C for two days. The reaction mixture was cooled to room temperature, and chloroform, water, and aqueous saturated sodium hydrogen carbonate solution were added to the reaction mixture. The separated aqueous layer was subjected to extraction with chloroform. The combined organic layer was dried over anhydrous sodium sulfate and then concentrated under reduced pressure. After the obtained residue was purified by silica gel column chromatography (hexane/ethyl acetate), the crudely purified product was purified by amino silica gel column chromatography (hexane/ethyl acetate) to obtain 2-heptylnonyl (1*r*,4*r*)-4-({8-[(2-heptylnonyl)oxy]-8-oxooctyl}amino)cyclohexane-1-carboxylate (456 mg) as an oil.

### Example 30

To a mixture of 2-heptylnonyl (1*r*,4*r*)-4-({8-[(2-heptylnonyl)oxy]-8-oxooctyl}amino)cyclohexane-1-carboxylate (151 mg) and DCM (3 mL), 1-methyl-L-proline monohydrate (36.4 mg), HATU (93.2 mg), and DIPEA (0.053 mL) were added, and the mixture was stirred at room temperature for 24 hours. To the reaction mixture, 1-methyl-L-proline monohydrate (18.1 mg), DIPEA (0.026 mL), and HATU (47.4 mg) were added, and the mixture was stirred at room temperature for five hours. Chloroform, water, and aqueous saturated sodium hydrogen carbonate solution were added to the reaction mixture, and the separated aqueous layer was subjected to extraction with chloroform. The combined organic layer was concentrated under reduced pressure, and ethyl acetate and a mixture of water/saturated brine (1/1) were added to the obtained residue. The separated organic layer was dried over anhydrous sodium sulfate and then concentrated under reduced pressure. After the obtained residue was purified by silica gel column chromatography (chloroform/methanol), the crudely purified product was purified by amino silica gel column chromatography (hexane/ethyl acetate) to obtain 2-heptylnonyl (1*S*,4*r*)-4-[{8-[(2-heptylnonyl)oxy]-8-oxooctyl}(1-methyl-L-prolyl)amino]cyclohexane-1-carboxylate (123 mg) as an oil.

### Example 31

To a mixture of 2-nonylundecyl 8-{[(1*r*,3*r*)-3-{2-[(2-octyldecyl)oxy]-2-oxoethyl}cyclobutyl]amino}octanoate (160 mg) and DCM (6 mL), 1-ethyl-D-proline (57 mg), HATU (151 mg), and DIPEA (0.085 mL) were added, and the mixture was stirred at room temperature over a weekend. After the reaction mixture was purified by silica gel column chromatography (chloroform/methanol), the crudely purified product was purified by amino silica gel column chromatography (hexane/ethyl acetate/methanol) to obtain 2-nonylundecyl 8-{(1-ethyl-D-prolyl)[(1*r*,3*R*)-3-{2-[(2-octyldecyl)oxy]-2-oxoethyl}cyclobutyl]amino}octanoate (133 mg) as an oil.

### Production Example 34-1

Palladium on activated carbon (Pd 10%) (40 mg) was added to a mixture of (1*R*,2*R*)-2-aminocyclopentane-1-carboxylic acid monohydrochloride (200 mg), methanol (4 mL), and formaldehyde (aqueous 37% solution, 0.33 mL) under argon atmosphere at room temperature, and the mixture was stirred under hydrogen atmosphere at room temperature for 18 hours. The reaction mixture was filtered through celite (registered trademark), and the filtrate was concentrated under reduced pressure. An operation of adding chloroform/methanol to the obtained residue and concentrating under reduced pressure was conducted three times, and diisopropyl ether was added. The mixture was stirred at room temperature for 10 minutes. The generated solid was collected by filtration and dried under reduced pressure to obtain (1*R*,2*R*)-2-(dimethylamino)cyclopentane-1-carboxylic acid hydrochloride (217 mg) as a solid.

### Example 39

To a mixture of *tert*-butyl 4-{(1-methyl-L-prolyl)[(1*r*,3*S*)-3-{2-[(2-octyldecyl)oxy]-2-oxoethyl}cyclobutyl]amino}butanoate (181 mg) and DCM (0.5 mL), 4M hydrogen chloride/ethyl acetate solution (1.4 mL) was added at room temperature, and the mixture was stirred at room temperature for 18 hours. The reaction mixture was concentrated under reduced pressure. To a mixture of the obtained oily material (175 mg) and DCM (5 mL), (9*Z*,12*Z*)-octadeca-9,12-dien-1-ol (0.11 mL), HATU (130 mg), DMAP (3 mg), and DIPEA (0.15 mL) were added at room temperature, and after the mixture was stirred at room temperature for 18 hours, the reaction mixture was concentrated under reduced pressure. After the obtained residue was purified by silica gel column chromatography (chloroform/methanol), the crudely purified product was purified by amino silica gel column chromatography (hexane/ethyl acetate/methanol). Subsequently, the crudely purified product was purified by silica gel column chromatography (hexane/ethyl acetate/methanol) to obtain (9*Z*,12*Z*)-octadeca-9,12-dien-1-yl 4-{(1-methyl-L-prolyl)[(1*r*,3*S*)-3-{2-[(2-octyldecyl)oxy]-2-oxoethyl}cyclobutyl]amino}butanoate (48 mg) as an oil.

### Production Example 49-1

To a mixture of 3-decyltridecan-1-ol (504 mg) and DCM (10 mL), 8-bromooctanoic acid (396 mg), DIPEA (0.633 mL), HATU (738 mg), and DMAP (19 mg) were added in a water bath, and the mixture was stirred at room temperature for seven hours. Aqueous saturated sodium hydrogen carbonate solution was added to the reaction mixture, and after extraction with chloroform, the organic layer was dried over anhydrous magnesium sulfate and then concentrated under reduced pressure. The obtained residue was purified by silica gel column chromatography (hexane/ethyl acetate) to obtain 3-decyltridecyl 8-bromooctanoate (783 mg) as an oil.

### Production Example 49-2

To a mixture of 3-decyltridecan-1-ol (502 mg) and DCM (10 mL), {(1*r*,3*r*)-3-[(*tert-*butoxycarbonyl)amino]cyclobutyl}acetic acid (405 mg), DIPEA (0.63 mL), HATU (731 mg), and DMAP (18.2 mg) were added at room temperature, and the mixture was stirred at room temperature for 24 hours. Aqueous saturated sodium hydrogen carbonate solution was added to the reaction mixture, and after extraction with chloroform, the organic layer was dried over anhydrous magnesium sulfate and then concentrated under reduced pressure. The obtained residue was purified by silica gel column chromatography (hexane/ethyl acetate) to obtain 3-decyltridecyl {(1*r*,3*r*)-3-[(*tert*-butoxycarbonyl)amino]cyclobutyl}acetate (763 mg) as an oil.

### Production Example 49-3

After TFA (5 mL) was added to a mixture of 3-decyltridecyl {(1*r*,3*r*)-3-[(*tert-*butoxycarbonyl)amino]cyclobutyl}acetate (763 mg) and DCM (15 mL) under ice cooling, the mixture was stirred at room temperature for four hours. The reaction mixture was diluted with chloroform and then made basic by adding aqueous saturated sodium hydrogen carbonate solution under ice cooling. The reaction mixture was subjected to extraction with chloroform, and the organic layer was dried over anhydrous magnesium sulfate and then concentrated under reduced pressure. The obtained residue was purified by amino silica gel column chromatography (hexane/ethyl acetate) to obtain 3-decyltridecyl [(1*r*,3*r*)-3-aminocyclobutyl]acetate (585 mg) as an oil.

### Production Example 49-4

To a mixture of 3-decyltridecyl [(1*r*,3*r*)-3-aminocyclobutyl]acetate (585 mg), 3-decyltridecyl 8-bromooctanoate (353 mg), MeCN (10 mL), and CPME (10 mL), DIPEA (0.277 mL) and KI (11 mg) were added at room temperature, and the mixture was stirred in an oil bath at 80°C for 48 hours. After the reaction mixture was left to cool at room temperature, aqueous saturated sodium hydrogen carbonate solution was added to the reaction mixture, and extraction with ethyl acetate was conducted. The organic layer was washed with saturated brine, dried over anhydrous magnesium sulfate and then concentrated under reduced pressure. The obtained residue was purified by silica gel column chromatography (chloroform/methanol) to obtain 3-decyltridecyl 8-{[(1*r*,3*r*)-3-{2-[(3-decyltridecyl)oxy]-2-oxoethyl}cyclobutyl]amino}octanoate (393 mg) as an oil.

### Example 49

To a mixture of 3-decyltridecyl 8-{[(1*r*,3*r*)-3-{2-[(3-decyltridecyl)oxy]-2-oxoethyl}cyclobutyl]amino}octanoate (129 mg) and DCM (5 mL), 1-methyl-L-proline (water content of 11.8%, 25.1 mg), HATU (64.9 mg), and DIPEA (0.036 mL) were added at room temperature, and the mixture was stirred at room temperature for four hours. Aqueous saturated sodium hydrogen carbonate solution was added to the reaction mixture, and extraction with chloroform was conducted. The organic layer was dried over anhydrous magnesium sulfate and then concentrated under reduced pressure. After the obtained residue was purified by silica gel column chromatography (chloroform/methanol), the crudely purified product was purified by amino silica gel column chromatography (hexane/ethyl acetate) to obtain 3-decyltridecyl 8-{[(1*r*,3*S*)-3-{2-[(3-decyltridecyl)oxy]-2-oxoethyl}cyclobutyl](1-methyl-L-prolyl)amino}octanoate (131 mg) as an oil.

### Production Example 55-1

A mixture of 2-octyldecyl [(1*r*,3*r*)-3-{[2-(benzyloxy)ethyl]amino}cyclobutyl]acetate (540 mg), DCM (5.4 mL), 1-ethyl-L-proline (225 mg), HATU (999 mg), and DIPEA (550 µL) was stirred at room temperature for five hours. Heptane and aqueous 90% methanol solution were added to the reaction mixture, and the separated organic layer was washed with aqueous 90% methanol solution and concentrated under reduced pressure. The obtained residue was purified by silica gel column chromatography (heptane/ethyl acetate) to obtain 2-octyldecyl [(1*S*,3*r*)-3-{[2-(benzyloxy)ethyl](1-ethyl-L-prolyl)amino}cyclobutyl]acetate (550 mg) as an oil.

### Production Example 55-2

To a mixture of 2-octyldecyl [(1*S*,3*r*)-3-{[2-(benzyloxy)ethyl](1-ethyl-L-prolyl)amino}cyclobutyl]acetate (500 mg) and acetic acid (4.2 mL), 20% palladium hydroxide on activated carbon (water content of 50%) (100 mg) was added at room temperature. The reaction system was replaced with nitrogen, and then the mixture was stirred under hydrogen atmosphere at room temperature for three hours. The reaction mixture was filtered, and the filtrate was added to a mixture of ethyl acetate and water. Potassium carbonate was added until the pH of the aqueous layer became 10 or more. The organic layer was collected, then washed with water and concentrated under reduced pressure. The obtained residue was purified by amino silica gel column chromatography (heptane/ethyl acetate) to obtain 2-octyldecyl {(1*S*,3*r*)-3-[(1-ethyl-L-prolyl)(2-hydroxyethyl)amino]cyclobutyl}acetate (320 mg) as an oil.

### Example 55

To a mixture of 2-octyldecyl {(1*S*,3*r*)-3-[(1-ethyl-L-prolyl)(2-hydroxyethyl)amino]cyclobutyl}acetate (200 mg) and DCM (4 mL), 4,4-bis(octyloxy)butanoic acid (250 mg), HATU (345 mg), DMAP (4.4 mg), and DIPEA (0.186 mL) were added at room temperature, and the mixture was stirred at room temperature for 21.5 hours. After the reaction mixture was concentrated under reduced pressure, heptane and aqueous 90% methanol solution were added to the obtained residue, and the separated organic layer was washed twice with aqueous 90% methanol solution. The aqueous 90% methanol solution layer was subjected again to extraction with heptane, and the combined organic layer was concentrated under reduced pressure. After the obtained residue was purified by amino silica gel column chromatography (heptane/ethyl acetate), the crudely purified product was purified by silica gel column chromatography (heptane/ethyl acetate) to obtain 2-{(1-ethyl-L-prolyl)[(1*r*,3*S*)-3-{2-[(2-octyldecyl)oxy]-2-oxoethyl}cyclobutyl]amino}ethyl 4,4-bis(octyloxy)butanoate (151 mg) as an oil.

### Production Example 56-1

Pyridine (5.4 mL), 4-nitrophenyl chloroformate (2.7 g), and DMAP (40 mg) were added to a mixture of 2-nonylundecan-1-ol (2 g) and DCM (60 mL) under ice cooling, and the mixture was stirred at room temperature for two days. The reaction mixture was concentrated under reduced pressure, and hexane was added to the obtained residue. The insoluble matter was separated by filtration. The filtrate was concentrated under reduced pressure, and 4-nitrophenyl 2-nonylundecyl carbonate (2.87 g) was obtained as an oil.

### Production Example 56-2

Pyridine (0.4 mL), DMAP (60 mg), and *tert*-butyl [(1*r*,3*r*)-3-(hydroxymethyl)cyclobutyl]carbamate (0.5 g) were added to a mixture of 4-nitrophenyl 2-nonylundecyl carbonate (1.4 g) and DCM (13 mL) at room temperature, and the mixture was stirred at room temperature for four days. Chloroform and aqueous saturated sodium hydrogen carbonate solution were added to the reaction mixture, and the separated aqueous layer was subjected to extraction with chloroform. The combined organic layer was washed with saturated brine, dried over anhydrous sodium sulfate and then concentrated under reduced pressure. The obtained residue was purified by silica gel column chromatography (hexane/ethyl acetate) to obtain {(1*r*,3*r*)-3-[(*tert*-butoxycarbonyl)amino]cyclobutyl}methyl 2-nonylundecyl carbonate (1.29 g) as an oil.

### Production Example 56-3

To a mixture of {(1*r*,3*r*)-3-[(*tert*-butoxycarbonyl)amino]cyclobutyl}methyl 2-nonylundecyl carbonate (1.28 g) and DCM (10 mL), TFA (5 mL) was added under ice cooling, and the mixture was stirred at room temperature for 2.5 hours. After the reaction mixture was added to a mixture of chloroform and aqueous saturated sodium hydrogen carbonate solution under water cooling, the organic layer was separated, and the aqueous layer was subjected to extraction with chloroform. The combined organic layer was dried over anhydrous sodium sulfate and then concentrated under reduced pressure. The obtained residue was purified by amino silica gel column chromatography (hexane/ethyl acetate) to obtain [(1*r*,3*r*)-3-aminocyclobutyl]methyl 2-nonylundecyl carbonate (916 mg) as an oil.

### Production Example 58-1

To a mixture of 4-bromobutan-1-ol (0.7 mL) and DCM (10 mL), 4-octyldodecanoic acid (1 g), DIPEA (1.4 mL), HATU (1.58 g), and DMAP (42 mg) were added under ice cooling, and the mixture was stirred at room temperature for seven hours. Chloroform and water were added to the reaction mixture, and the organic layer was separated. The aqueous layer was subjected to extraction with chloroform. The combined organic layer was dried over anhydrous magnesium sulfate and then concentrated under reduced pressure. The obtained residue was purified by silica gel column chromatography (hexane/ethyl acetate) to obtain 4-bromobutyl 4-octyldodecanoate (0.817 g) as an oil.

### Production Example 58-2

To a mixture of [(1*r*,3*r*)-3-aminocyclobutyl]methyl 2-nonylundecyl carbonate (471 mg) and CPME (4 mL), 4-bromobutyl 4-octyldodecanoate (250 mg), MeCN (4 mL), DIPEA (0.25 mL), and KI (10 mg) were added under argon atmosphere, and the mixture was stirred in an oil bath at 80°C for two days. The reaction mixture was left to cool, then diluted with ethyl acetate and washed with aqueous saturated sodium hydrogen carbonate solution and saturated brine. The organic layer was dried over anhydrous sodium sulfate and then concentrated under reduced pressure. The obtained residue was purified by amino silica gel column chromatography (hexane/ethyl acetate) to obtain 4-({(1*r*,3*r*)-3-[({[(2-nonylundecyl)oxy]carbonyl}oxy)methyl]cyclobutyl}amino)butyl 4-octyldodecanoate (333 mg) as an oil.

### Example 58

To a mixture of 4-({(1*r*,3*r*)-3-[({[(2-nonylundecyl)oxy]carbonyl}oxy)methyl]cyclobutyl}amino)butyl 4-octyldodecanoate (100 mg) and DCM (2 mL), 1-ethyl-L-proline (22 mg), HATU (58 mg), and DIPEA (35 µL) were added at room temperature, and the mixture was stirred at room temperature for three days. After the reaction mixture was purified by silica gel column chromatography (chloroform/methanol), the crudely purified product was purified by amino silica gel column chromatography (hexane/ethyl acetate) to obtain 4-[(1-ethyl-L-prolyl){(1*r*,3*S*)-3-[({[(2-nonylundecyl)oxy]carbonyl}oxy)methyl]cyclobutyl}amino]butyl 4-octyldodecanoate (101 mg) as an oil.

### Production Example 59-1

After pyridine (1.4 mL) and 4-nitrophenyl chloroformate (1.29 g) were added to a mixture of *tert*-butyl [(1*r*,3*r*)-3-(2-hydroxyethyl)cyclobutyl]carbamate (915 mg) and DCM (30 mL) under ice cooling, the mixture was stirred under ice cooling for 30 minutes and further at room temperature for 18 hours. Silica gel was added to the reaction mixture, and the mixture was concentrated under reduced pressure. The obtained residue was purified by silica gel column chromatography (hexane/ethyl acetate) to obtain 2-{(1*r*,3*r*)-3-[(*tert-*butoxycarbonyl)amino]cyclobutyl}ethyl 4-nitrophenyl carbonate (1.77 g) as a solid.

### Production Example 59-2

Pyridine (8 mL), 2-octyldecan-1-ol (3.78 g), and DMAP (114 mg) were added to a mixture of 2-{(1*r*,3*r*)-3-[(*tert*-butoxycarbonyl)amino]cyclobutyl}ethyl 4-nitrophenyl carbonate (1.77 g) and DCM (40 mL), and the mixture was stirred at room temperature for five days. The reaction mixture was concentrated under reduced pressure, then diluted with ethyl acetate/hexane and washed with aqueous saturated ammonium chloride solution, aqueous saturated sodium hydrogen carbonate solution, and saturated brine. The organic layer was dried over anhydrous sodium sulfate and then concentrated under reduced pressure. The obtained residue was purified by silica gel column chromatography (hexane/ethyl acetate) to obtain 2-{(1*r*,3*r*)-3-[(*tert*-butoxycarbonyl)amino]cyclobutyl}ethyl 2-octyldecyl carbonate (1.94 g) as an oil.

### Production Example 63-1

To a mixture of *tert*-butyl [(1*r*,3*r*)-3-(hydroxymethyl)cyclobutyl]carbamate (300 mg) and DCM (9 mL), DMAP (2 mg), 4-nitrophenyl chloroformate (602 mg), and pyridine (0.6 mL) were added under ice cooling, and the mixture was stirred at room temperature for 18 hours. To the reaction mixture, *N-*octyloctan-1-amine (2.3 mL) was added at room temperature, and the mixture was stirred at room temperature for three hours. The reaction mixture was concentrated under reduced pressure, and hexane/ethyl acetate and aqueous saturated sodium hydrogen carbonate solution were added to the obtained residue. The separated organic layer was washed with aqueous saturated sodium hydrogen carbonate solution, water, and saturated brine, dried over anhydrous sodium sulfate and then concentrated under reduced pressure. The obtained residue was purified by silica gel column chromatography (ethyl acetate/hexane) to obtain {(1*r*,3*r*)-3-[(*tert-*butoxycarbonyl)amino]cyclobutyl}methyl di(octyl)carbamate (612 mg) as an oil.

### Production Example 66-1

To a mixture of 4-nitrophenyl 2-nonylundecyl carbonate (500 mg) and DCM (15 mL), DIPEA (1.8 mL), 6-bromohexan-1-ol (0.74 mL), and DMAP (13 mg) were added at room temperature, and the mixture was stirred at room temperature for 18 hours. To the reaction mixture, CPME (15 mL) was added, and the mixture was stirred in an oil bath at 80°C for seven hours. After the reaction mixture was left to cool to room temperature, DMAP (526 mg) was added, and the mixture was stirred at room temperature over a weekend. Hexane and aqueous saturated sodium hydrogen carbonate solution were added to the reaction mixture, and the separated organic layer was washed with aqueous saturated sodium hydrogen carbonate solution, water, and saturated brine, dried over anhydrous sodium sulfate and then concentrated under reduced pressure. The obtained residue was purified by silica gel column chromatography (ethyl acetate/hexane) to obtain 6-bromohexyl 2-nonylundecyl carbonate (344 mg) as an oil.

### Production Example 72-1

Pyridine (1.9 mL), *tert*-butyl [(1*r*,4*r*)-4-hydroxycyclohexyl]carbamate (371 mg), and DMAP (561 mg) were added to a mixture of 4-nitrophenyl 2-octyldecyl carbonate (500 mg) and CPME (15 mL) at room temperature, and the mixture was stirred in an oil bath at 50°C for 18 hours. After the reaction mixture was left to cool to room temperature, hexane/ethyl acetate and aqueous saturated sodium hydrogen carbonate solution were added to the reaction mixture, and the separated organic layer was washed with water and saturated brine, dried over anhydrous sodium sulfate and then concentrated under reduced pressure. The obtained residue was purified by silica gel column chromatography (ethyl acetate/hexane) to obtain (1*r*,4*r*)-4-[(*tert*-butoxycarbonyl)amino]cyclohexyl 2-octyldecyl carbonate (495 mg) as an oil.

### Production Example 73-1

To a mixture of {(1*r*,3*r*)-3-[(*tert*-butoxycarbonyl)amino]cyclobutyl}acetic acid (350 mg) and DCM (7 mL), *N*-decyldecan-1-amine (545 mg), HATU (697 mg), and DIPEA (0.39 mL) were added at room temperature, and the mixture was stirred at room temperature for 18 hours. The reaction mixture was concentrated under reduced pressure, and ethyl acetate/hexane and aqueous saturated sodium hydrogen carbonate solution were added to the obtained residue. The separated organic layer was washed with water and saturated brine, dried over anhydrous sodium sulfate and then concentrated under reduced pressure. The obtained residue was purified by silica gel column chromatography (ethyl acetate/chloroform) to obtain *tert*-butyl [(1*r*,3*r*)-3-{2-[di(decyl)amino]-2-oxoethyl}cyclobutyl]carbamate (755 mg) as a solid.

### Production Example 74-1

To a mixture of *tert*-butyl [(1*r*,3*r*)-3-(hydroxymethyl)cyclobutyl]carbamate (500 mg) and DCM (10 mL), DIPEA (0.85 mL) and methanesulfonyl chloride (0.29 mL) were added under ice cooling, and the reaction was started under ice cooling. The mixture was stirred for 1.5 hours. Water and chloroform were added to the reaction mixture under ice cooling, and the separated organic layer was dried over anhydrous sodium sulfate and then concentrated under reduced pressure. To a mixture of the obtained solid (818 mg) and MeCN (10 mL), DIPEA (0.85 mL) and undecan-1-amine (2.66 mL) were added at room temperature, and the mixture was stirred in an oil bath at 50°C for two hours and in an oil bath at 80°C for 16 hours. After the reaction mixture was left to cool to room temperature, water and chloroform were added, and the separated organic layer was washed with saturated brine, dried over anhydrous sodium sulfate and then concentrated under reduced pressure. After the obtained residue was purified by amino silica gel column chromatography (ethyl acetate/hexane), the crudely purified product was purified by silica gel column chromatography (chloroform/methanol) to obtain *tert*-butyl {(1*r*,3*r*)-3-[(undecylamino)methyl]cyclobutyl}carbamate (644 mg) as a solid.

### Production Example 74-2

Undecanoic acid (409 mg), HATU (830 mg), and DIPEA (0.47 mL) were added to a mixture of *tert*-butyl {(1*r*,3*r*)-3-[(undecylamino)methyl]cyclobutyl}carbamate (644 mg) and DCM (10 mL) at room temperature, and the mixture was stirred at room temperature for 16 hours. The reaction mixture was concentrated under reduced pressure, and the obtained residue was charged to amino silica gel and purified by silica gel column chromatography (ethyl acetate/hexane) to obtain *tert*-butyl {(1*r*,3*r*)-3-[(*N-*undecylundecanamido)methyl]cyclobutyl}carbamate (895 mg) as an oil.

**[Table 10]**

| NUM | STR |
|---|---|
| PEx1-1 | 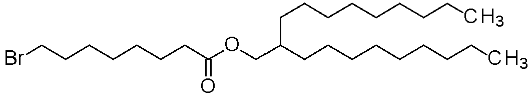 |
| PEx 1-2 | 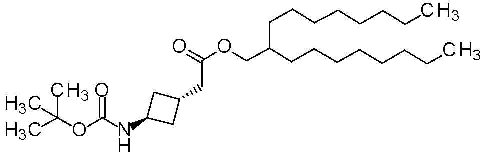 |
| PEx1-3 | 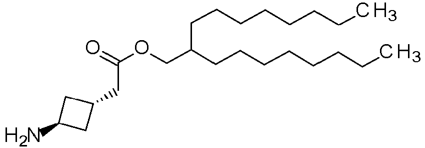 |
| PEx 1-4 | 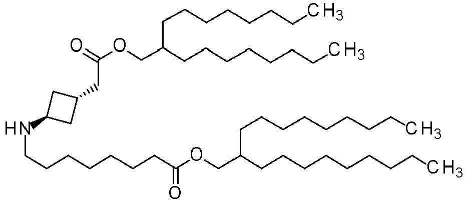 |
| Ex1 | 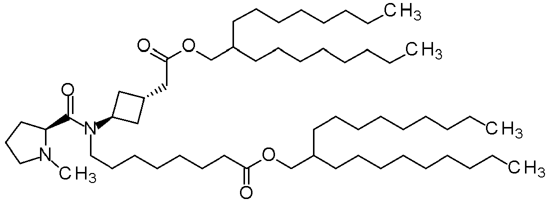 |
| Ex2 | 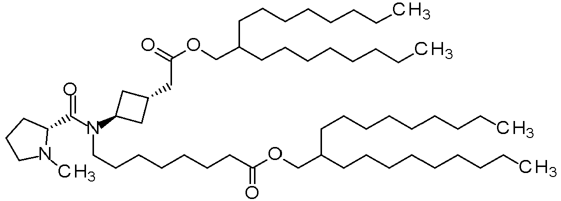 |

**[Table 11]**

| NUM | STR |
|---|---|
| Ex3 | 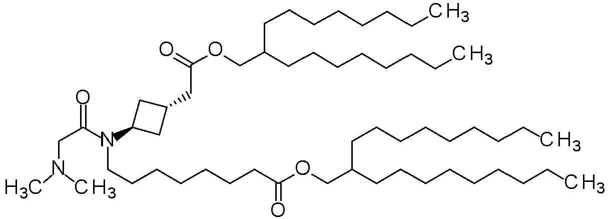 |
| PEx4-1 | 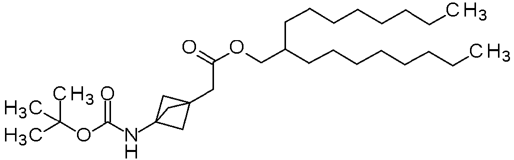 |
| PEx4-2 | 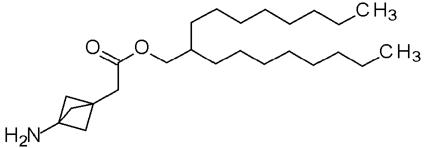 |
| PEx4-3 | 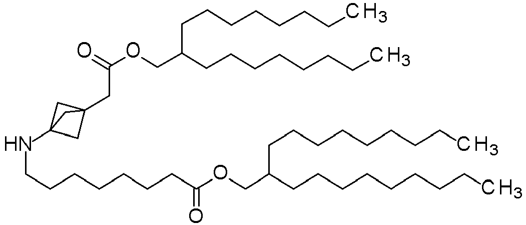 |
| Ex4 | 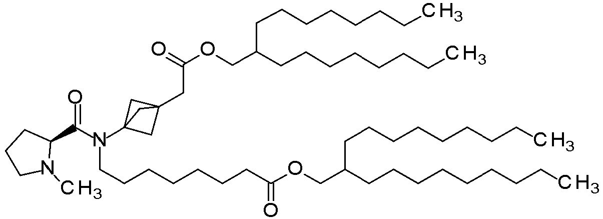 |

**[Table 12]**

| NUM | STR |
|---|---|
| PEx5-1 | 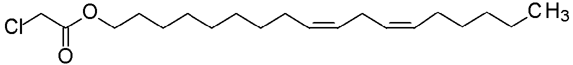 |
| PEx5-2 | 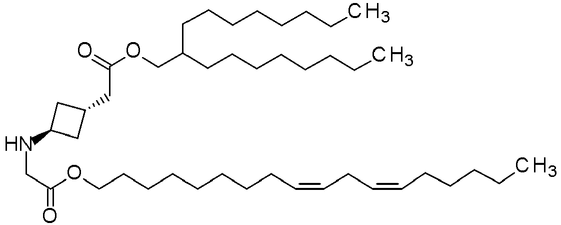 |
| Ex5 | 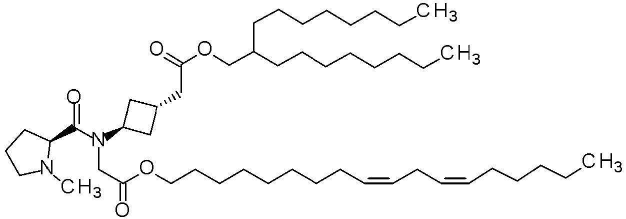 |
| Ex6 | 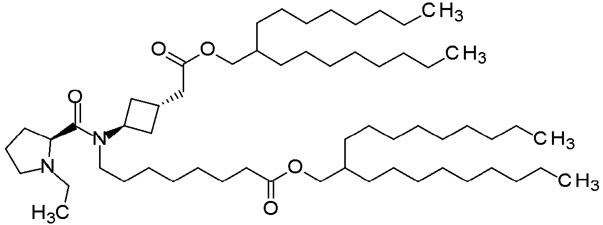 |
| Ex7 | 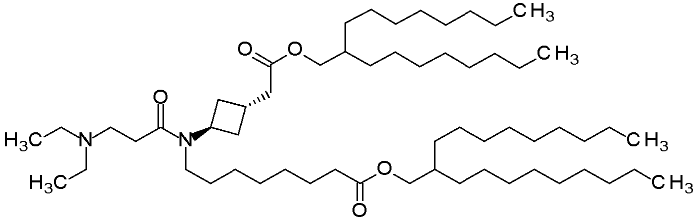 |

**[Table 13]**

| NUM | STR |
|---|---|
| Ex8 | 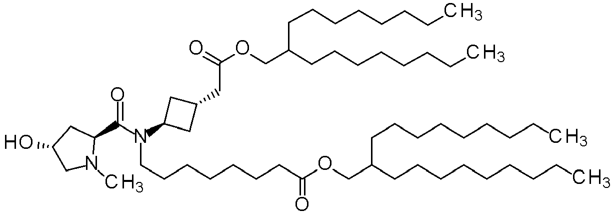 |
| PEx9-1 | 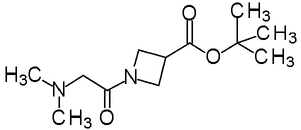 |
| PEx9-2/HCl | 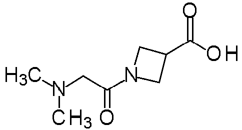 |
| Ex9 | 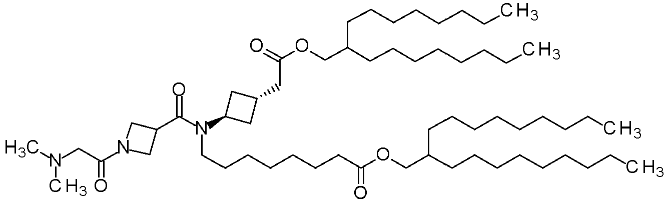 |
| Ex10 | 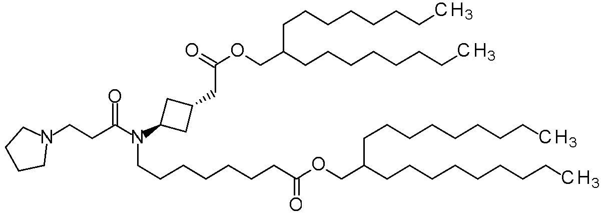 |
| PEx11-1 | 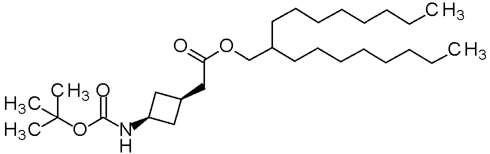 |

**[Table 14]**

| NUM | STR |
|---|---|
| PEx11-2 | 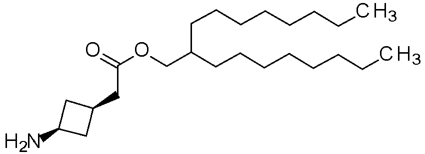 |
| PEx11-3 | 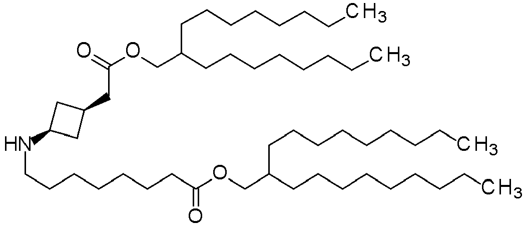 |
| Ex11 | 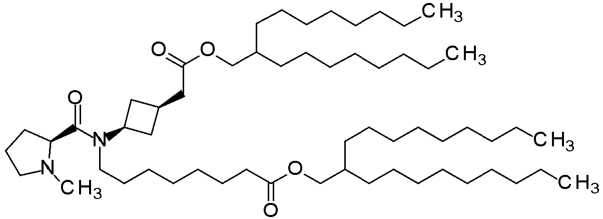 |
| PEx12-1 | 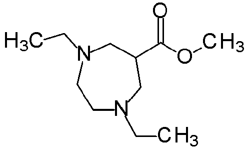 |
| Ex12 | 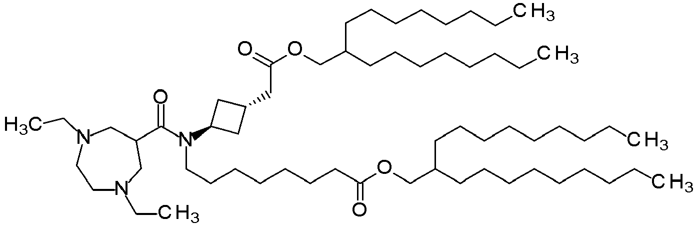 |

**[Table 15]**

| NUM | STR |
|---|---|
| Ex13 | 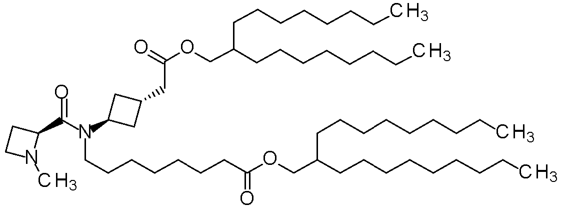 |
| PEx14-1 | 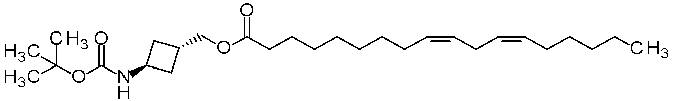 |
| PEx14-2 | 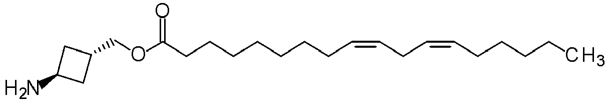 |
| PEx14-3 | 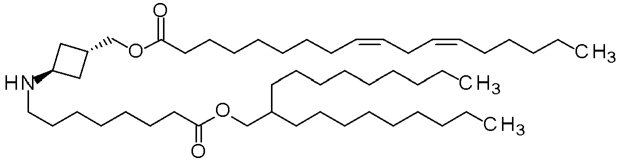 |
| Ex14 | 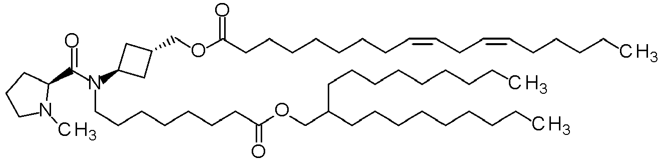 |
| PEx15-1 | 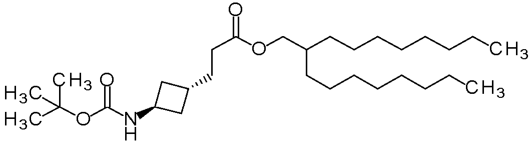 |
| PEx15-2 | 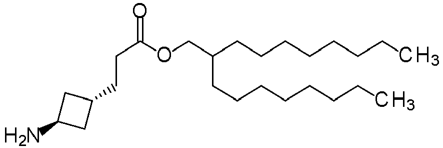 |

**[Table 16]**

| NUM | STR |
|---|---|
| PEx15-3 | 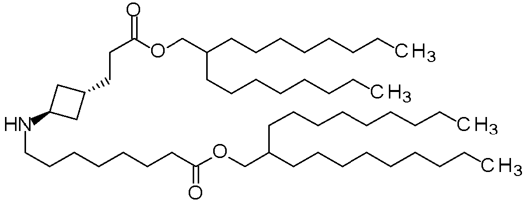 |
| Ex15 | 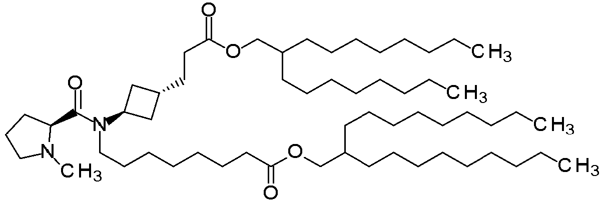 |
| PEx16-1 | 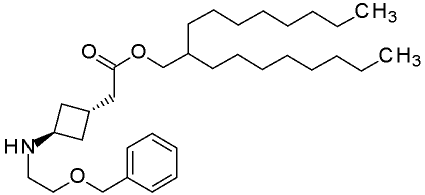 |
| PEx16-2 | 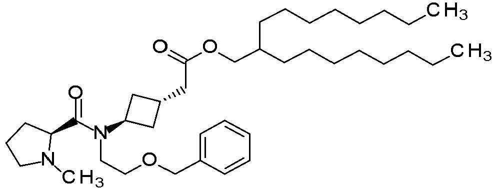 |
| PEx16-3 | 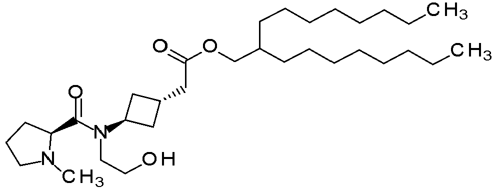 |

**[Table 17]**

| NUM | STR |
|---|---|
| Ex16 | 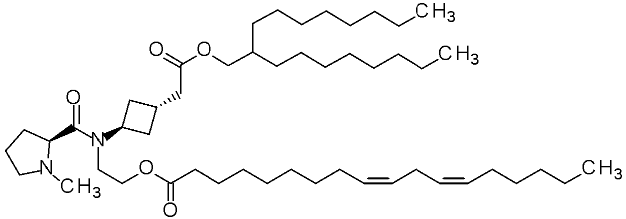 |
| Ex17 | 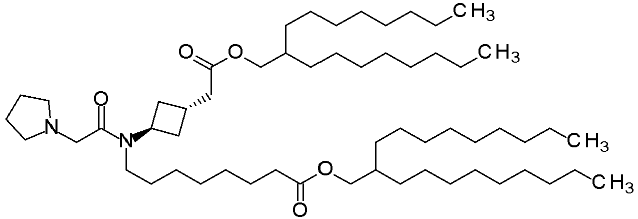 |
| Ex18 | 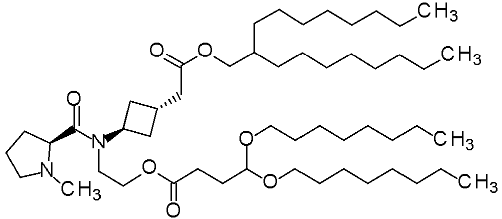 |
| Ex19 | 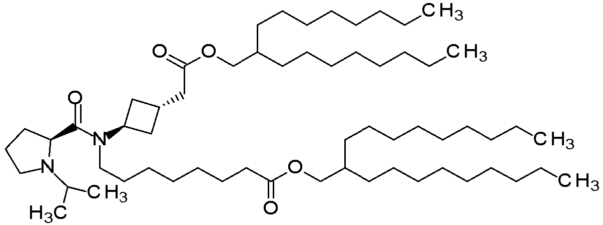 |
| PEx20-1 | 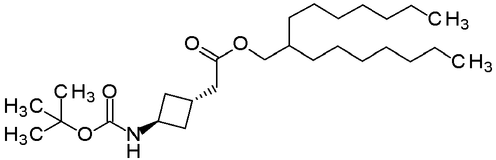 |

**[Table 18]**

| NUM | STR |
|---|---|
| PEx20-2 | 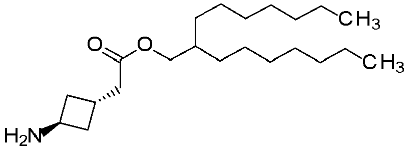 |
| PEx20-3 | 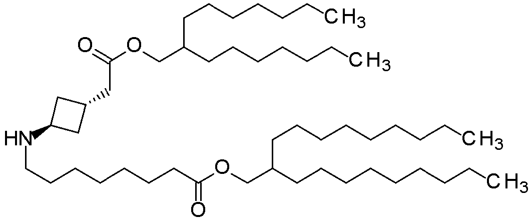 |
| Ex20 | 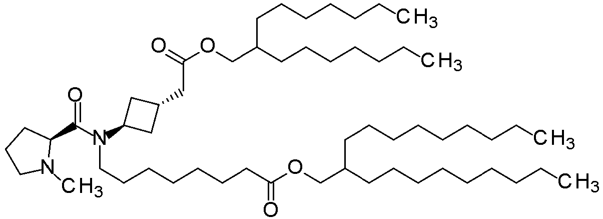 |
| Ex21 | 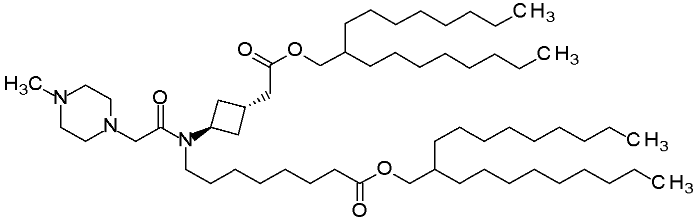 |
| PEx22-1 | 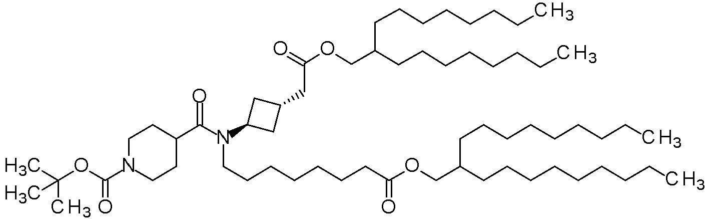 |

**[Table 19]**

| NUM | STR |
|---|---|
| PEx22-2 | 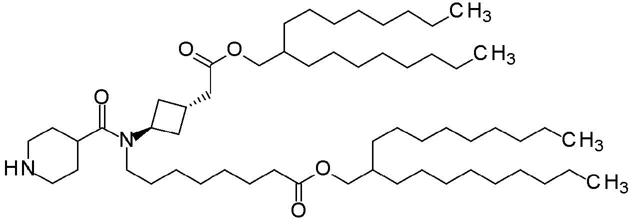 |
| Ex22 | 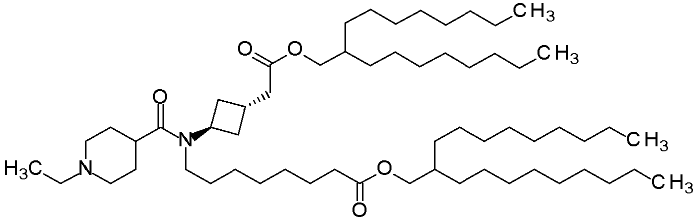 |
| PEx23-1 | 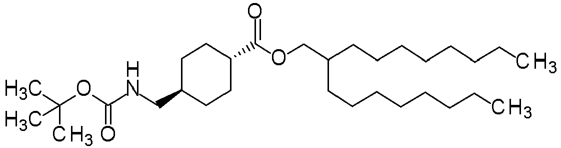 |
| PEx23-2 | 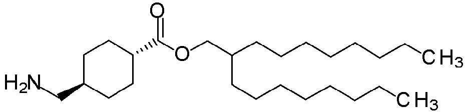 |
| PEx23-3 | 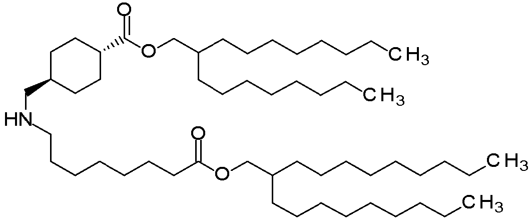 |

**[Table 20]**

| NUM | STR |
|---|---|
| Ex23 | 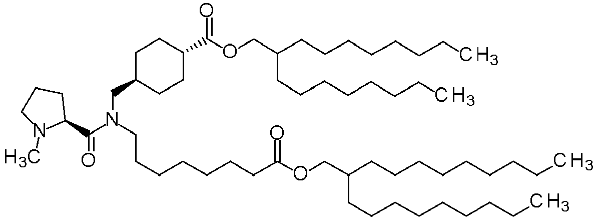 |
| PEx24-1 | 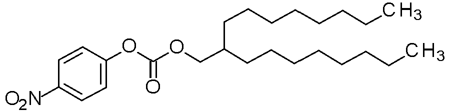 |
| PEx24-2 | 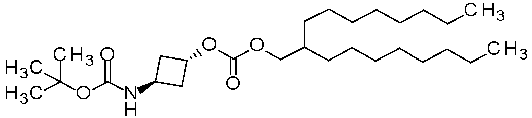 |
| PEx24-3 | 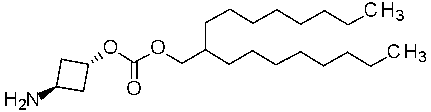 |
| PEx24-4 | 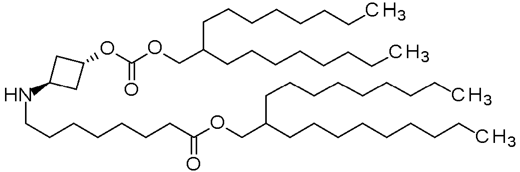 |
| Ex24 | 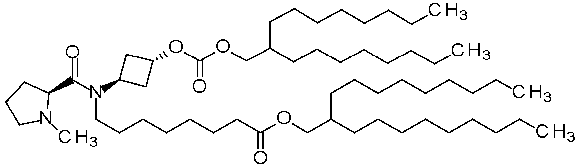 |
| PEx25-1 | 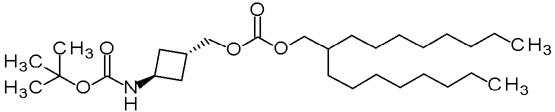 |

**[Table 21]**

| NUM | STR |
|---|---|
| PEx25-2 | 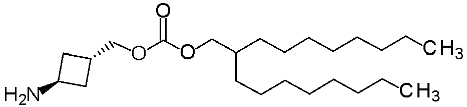 |
| PEx25-3 | 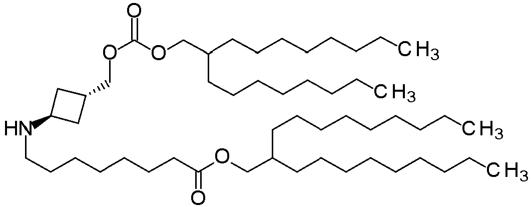 |
| Ex25 | 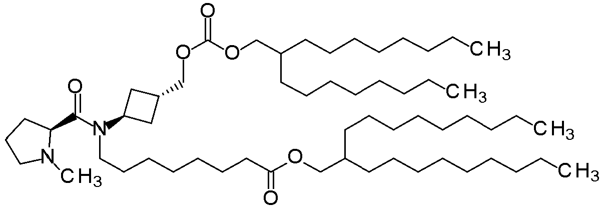 |
| Ex26 | 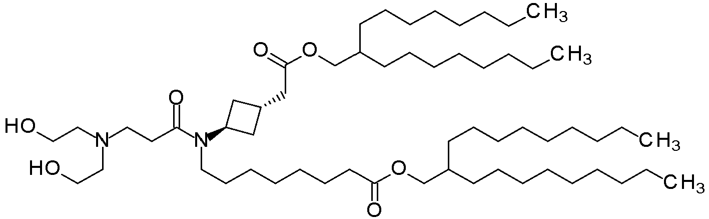 |
| Ex27 | 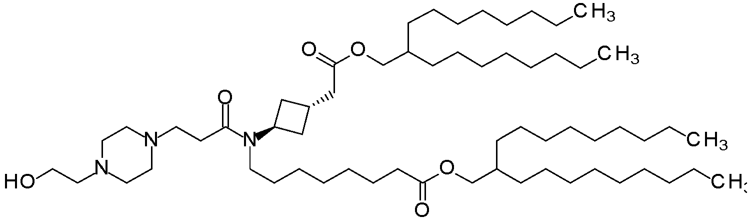 |

**[Table 22]**

| NUM | STR |
|---|---|
| Ex28 | 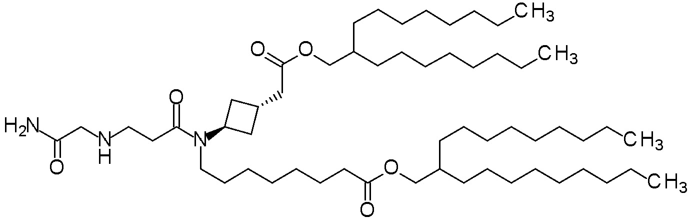 |
| Ex29 | 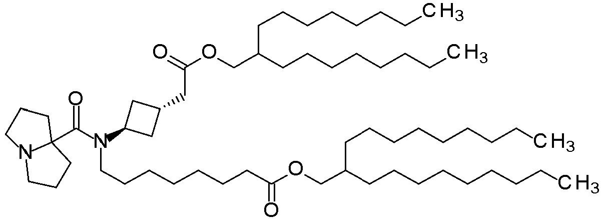 |
| PEx30-1 | 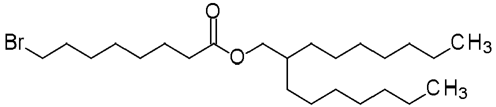 |
| PEx30-2 | 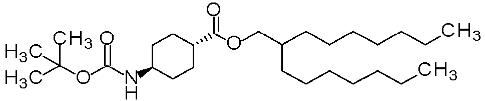 |
| PEx30-3 | 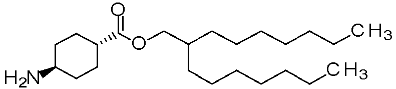 |
| PEx30-4 | 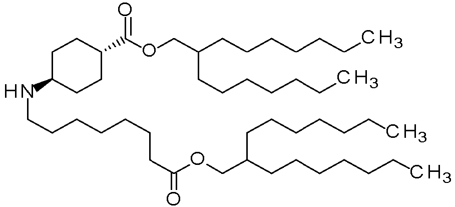 |

**[Table 23]**

| NUM | STR |
|---|---|
| Ex30 | 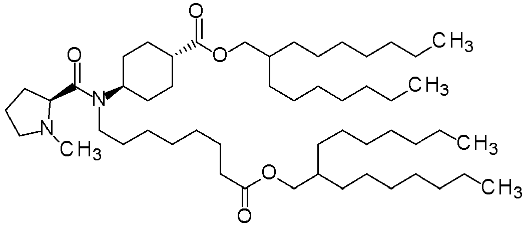 |
| Ex31 | 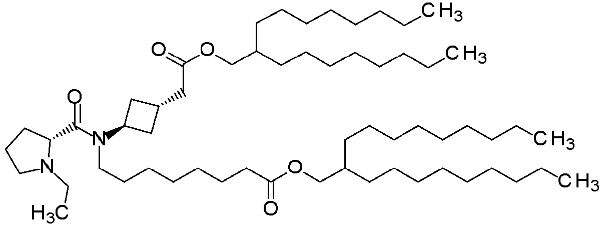 |
| Ex33 | 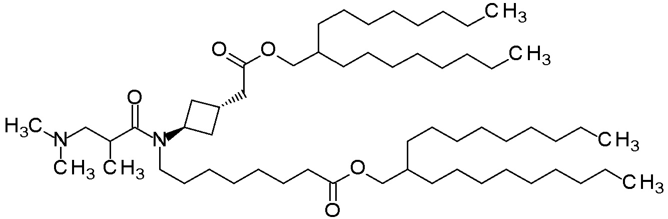 |
| PEx34-1/HCl | 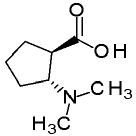 |
| Ex34 | 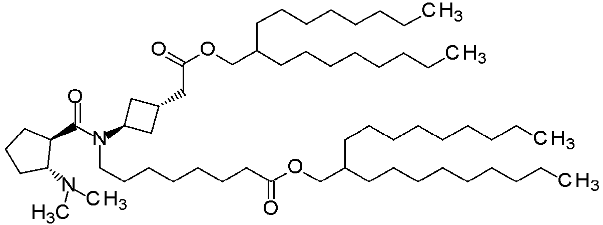 |

**[Table 24]**

| NUM | STR |
|---|---|
| PEx35-1 | 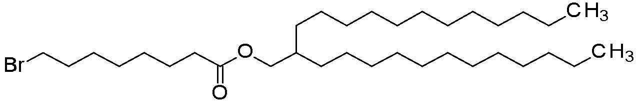 |
| PEx35-2 | 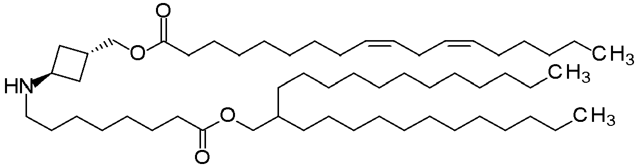 |
| Ex35 | 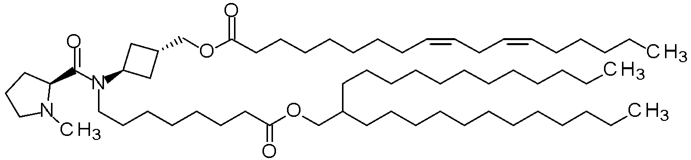 |
| PEx36-1 | 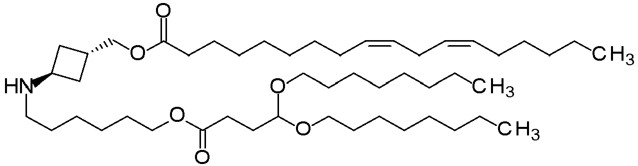 |
| Ex36 | 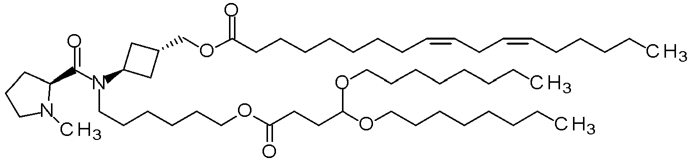 |
| PEx37-1 | 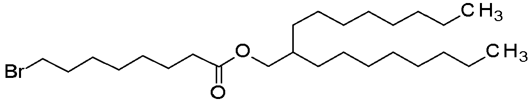 |
| PEx37-2 | 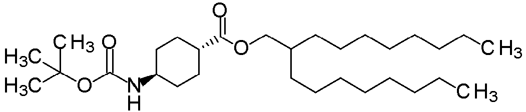 |

**[Table 25]**

| NUM | STR |
|---|---|
| PEx37-3 | 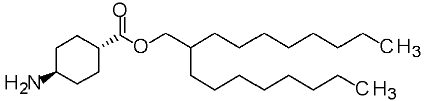 |
| PEx37-4 | 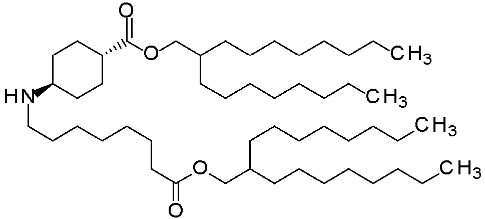 |
| Ex37 | 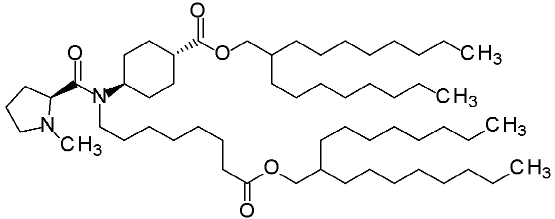 |
| PEx38-1 | 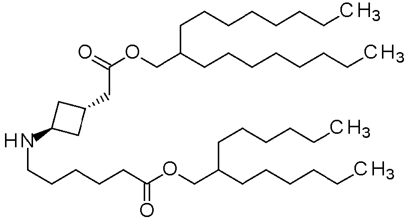 |
| Ex38 | 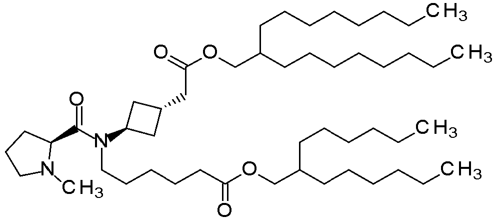 |

**[Table 26]**

| NUM | STR |
|---|---|
| PEx39-1 | 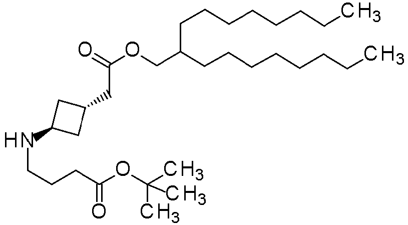 |
| PEx39-2 | 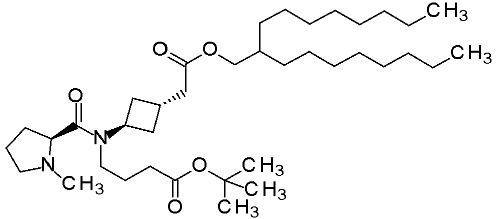 |
| Ex39 | 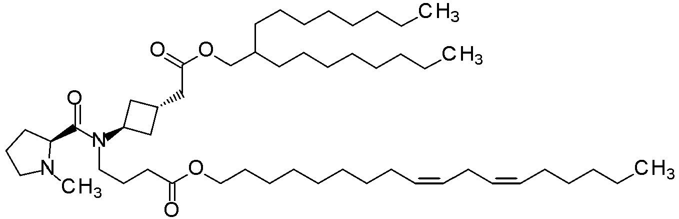 |
| PEx40-1 | 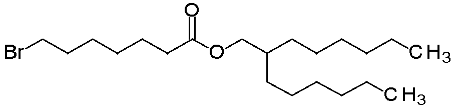 |
| PEx40-2 | 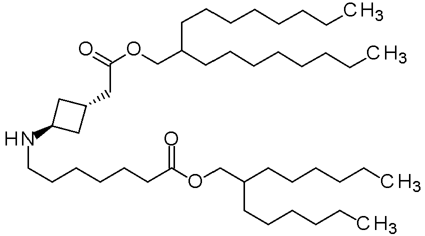 |

**[Table 27]**

| NUM | STR |
|---|---|
| Ex40 | 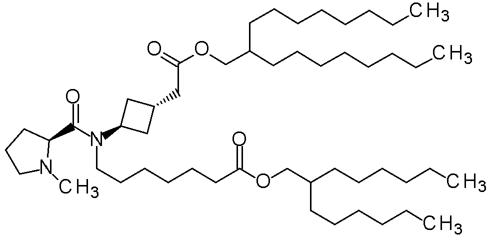 |
| PEx41-1 | 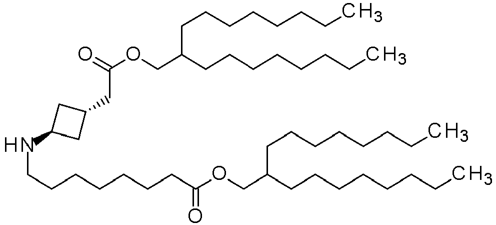 |
| Ex41 | 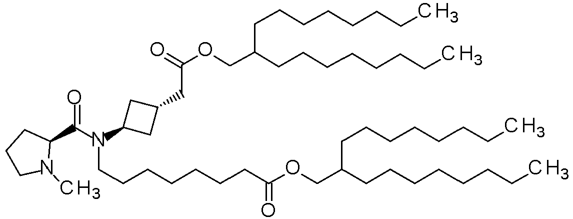 |
| PEx42-1 | 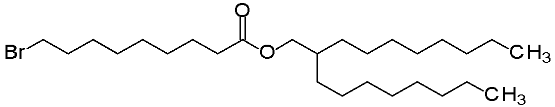 |
| PEx42-2 | 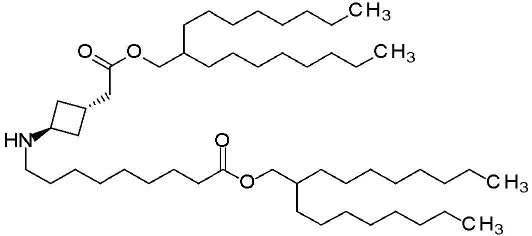 |

**[Table 28]**

| NUM | STR |
|---|---|
| Ex42 | 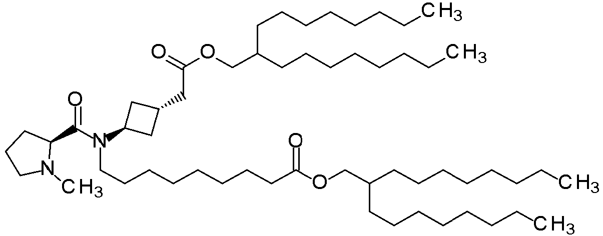 |
| PEx43-1 | 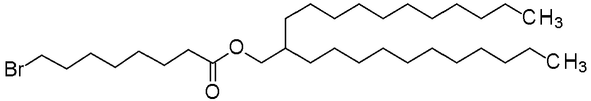 |
| PEx43-2 | 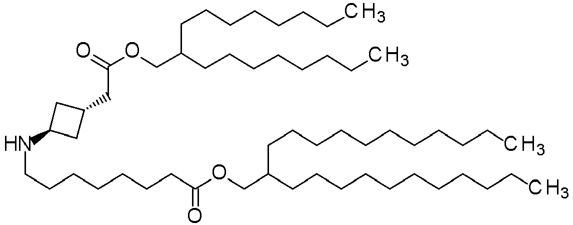 |
| Ex43 | 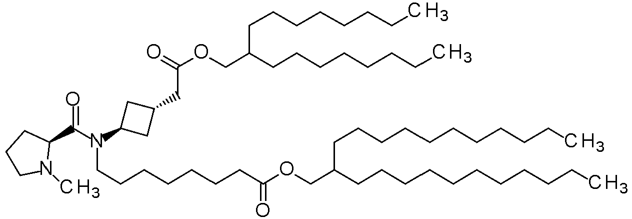 |
| PEx44-1 | 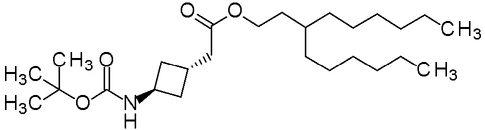 |
| PEx44-2 | 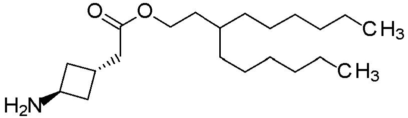 |

**[Table 29]**

| NUM | STR |
|---|---|
| PEx44-3 | 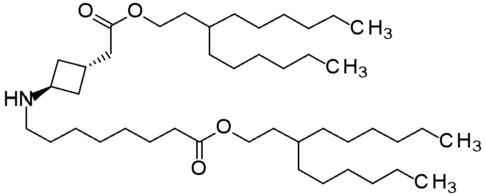 |
| Ex44 | 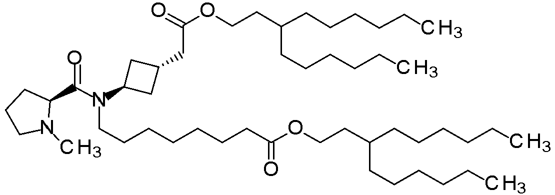 |
| Ex45 | 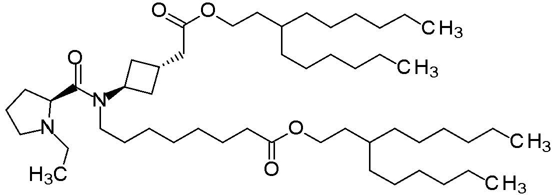 |
| Ex46 | 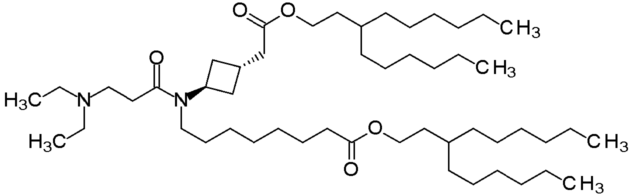 |
| PEx47-1 | 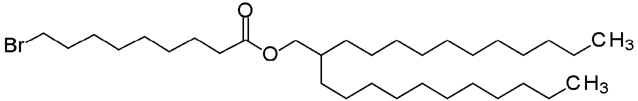 |

**[Table 30]**

| NUM | STR |
|---|---|
| PEx47-2 | 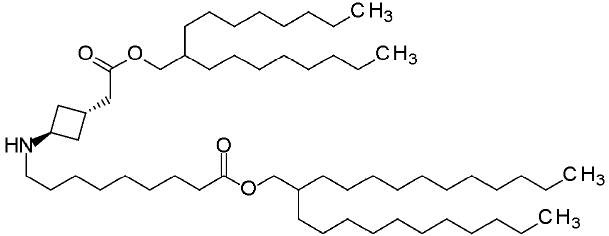 |
| Ex47 | 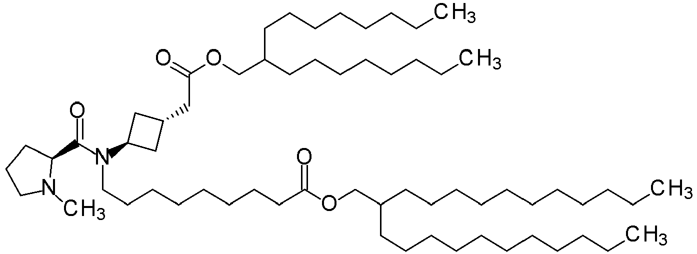 |
| PEx48-1 | 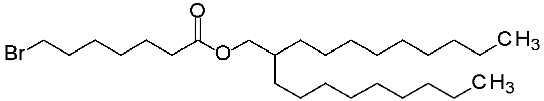 |
| PEx48-2 | 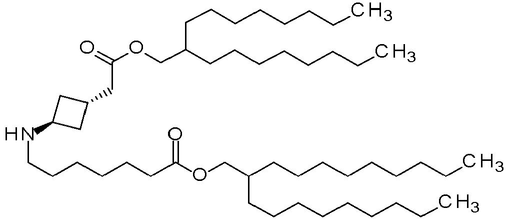 |
| Ex48 | 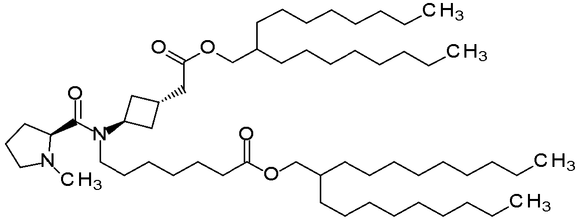 |

**[Table 31]**

| NUM | STR |
|---|---|
| PEx49-1 | 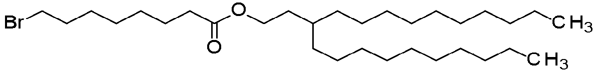 |
| PEx49-2 | 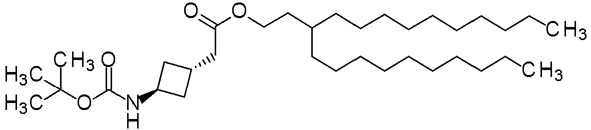 |
| PEx49-3 | 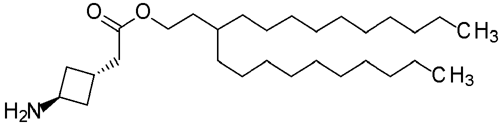 |
| PEx49-4 | 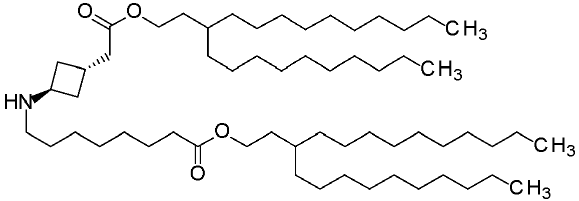 |
| Ex49 | 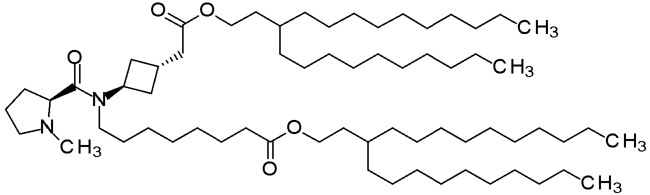 |
| Ex50 | 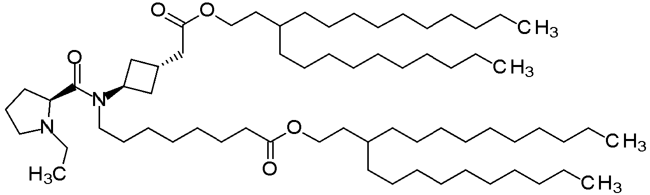 |

**[Table 32]**

| NUM | STR |
|---|---|
| Ex51 | 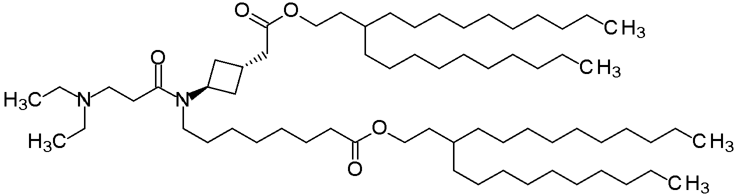 |
| PEx52-1 | 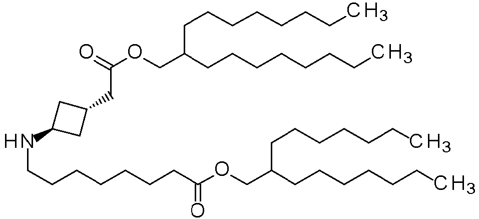 |
| Ex52 | 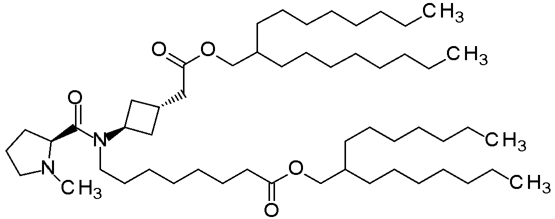 |
| PEx53-1 | 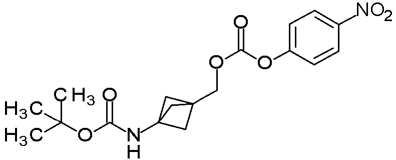 |
| PEx53-2 | 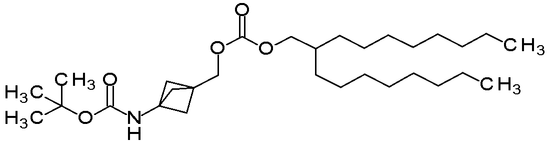 |

**[Table 33]**

| NUM | STR |
|---|---|
| PEx53-3 | 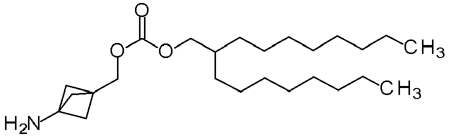 |
| PEx53-4 | 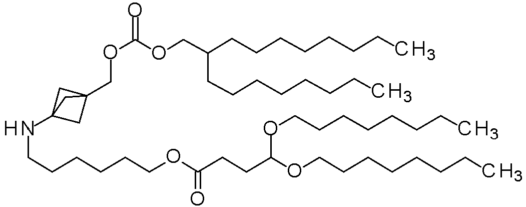 |
| Ex53 | 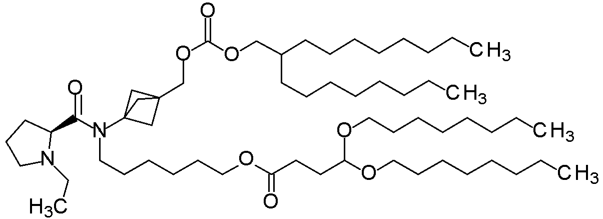 |
| PEx54-1 | 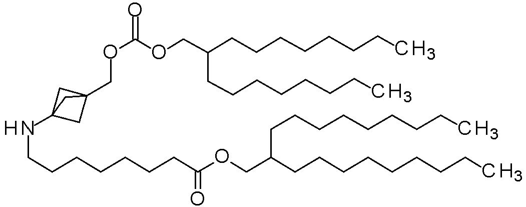 |
| Ex54 | 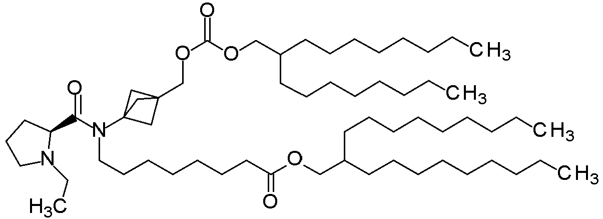 |

**[Table 34]**

| NUM | STR |
|---|---|
| PEx55-1 | 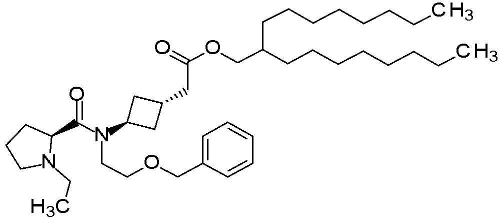 |
| PEx55-2 | 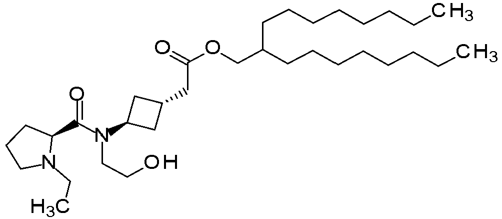 |
| Ex55 | 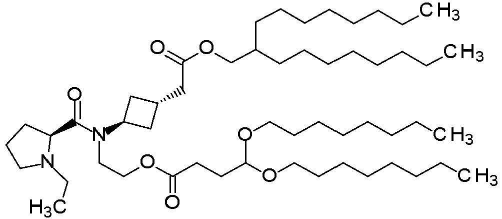 |
| PEx56-1 | 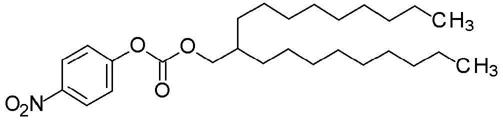 |
| PEx56-2 | 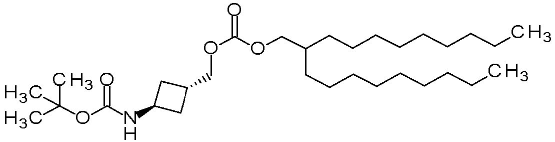 |

**[Table 35]**

| NUM | STR |
|---|---|
| PEx56-3 | 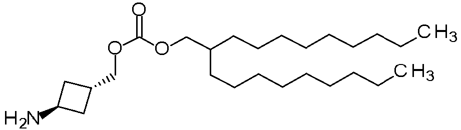 |
| PEx56-4 | 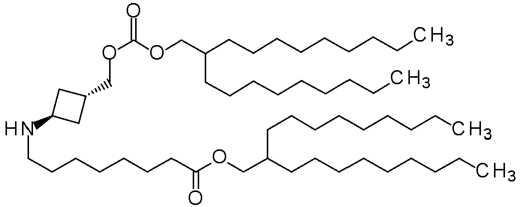 |
| Ex56 | 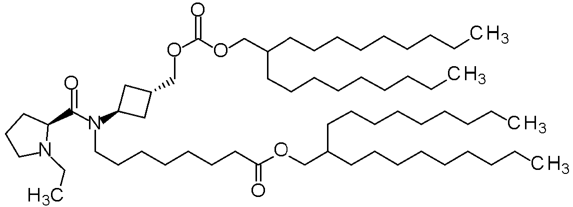 |
| PEx57-1 | 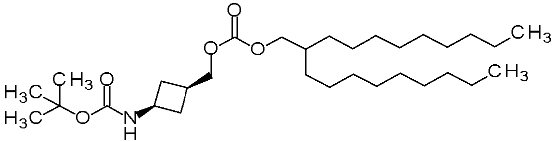 |
| PEx57-2 | 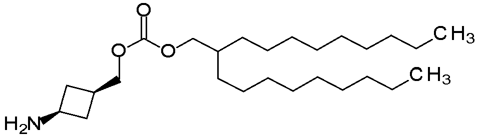 |
| PEx57-3 | 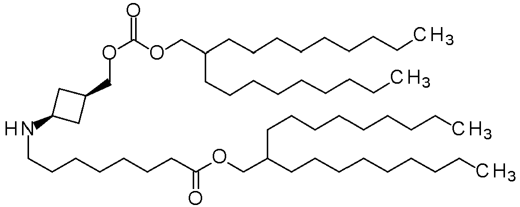 |

**[Table 36]**

| NUM | STR |
|---|---|
| Ex57 | 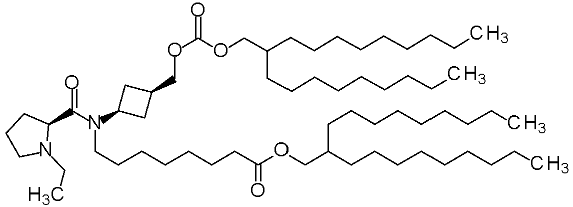 |
| PEx58-1 | 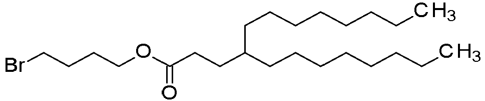 |
| PEx58-2 | 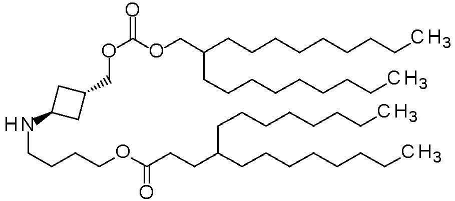 |
| Ex58 | 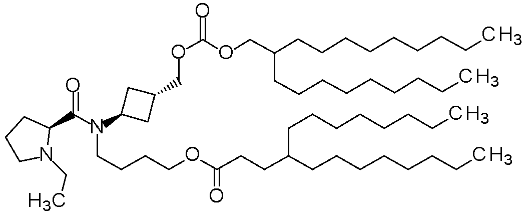 |
| PEx59-1 | 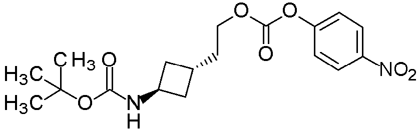 |
| PEx59-2 | 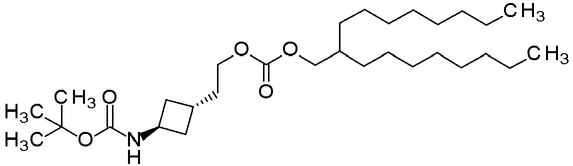 |

**[Table 37]**

| NUM | STR |
|---|---|
| PEx59-3 | 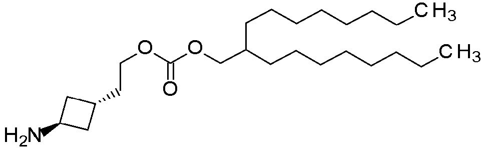 |
| PEx59-4 | 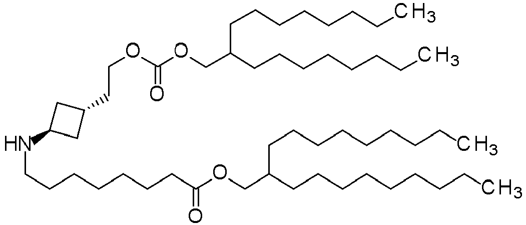 |
| Ex59 | 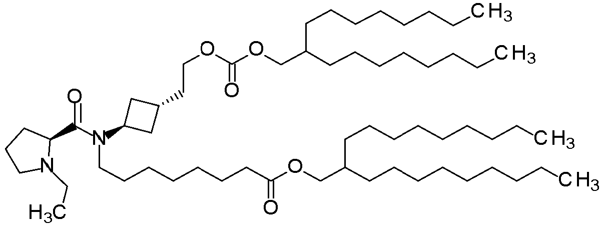 |
| PEx60-1 | 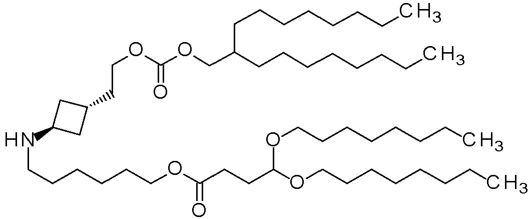 |
| Ex60 | 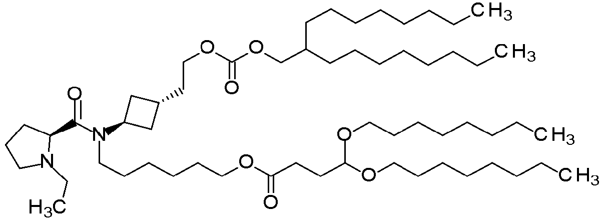 |

**[Table 38]**

| NUM | STR |
|---|---|
| PEx61-1 | 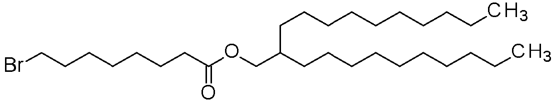 |
| PEx61-2 | 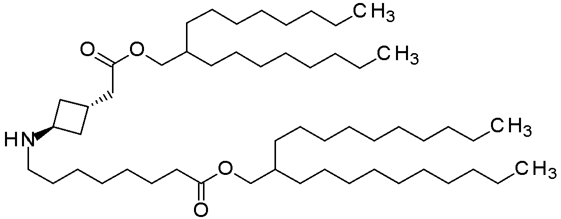 |
| Ex61 | 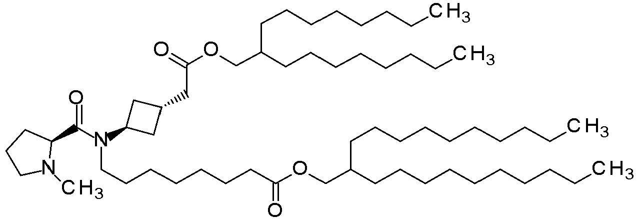 |
| PEx62-1 | 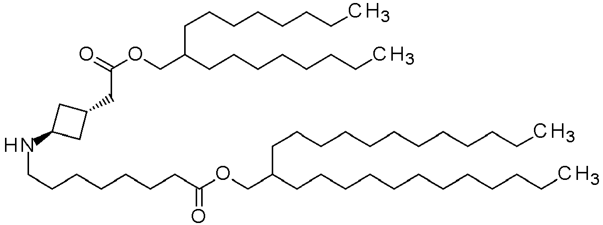 |
| Ex62 | 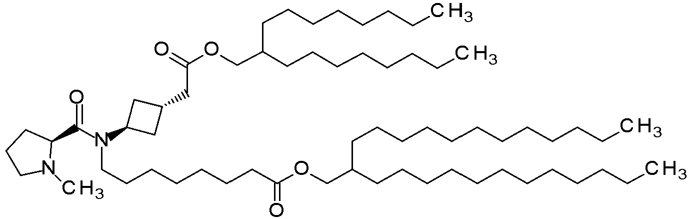 |

**[Table 39]**

| NUM | STR |
|---|---|
| PEx63-1 | 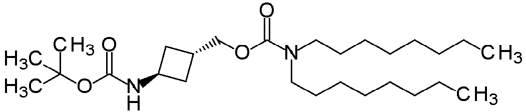 |
| PEx63-2 | 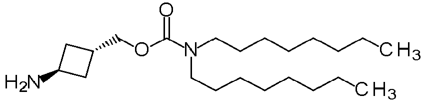 |
| PEx63-3 | 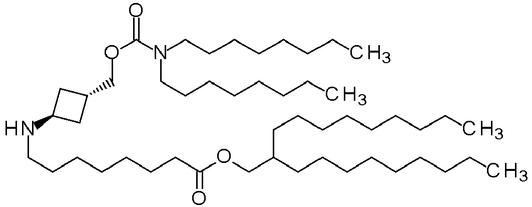 |
| Ex63 | 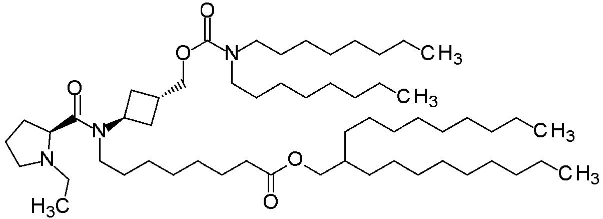 |
| PEx64-1 | 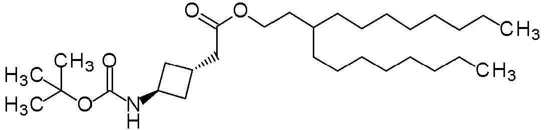 |
| PEx64-2 | 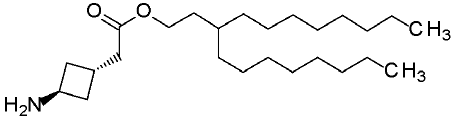 |

**[Table 40]**

| NUM | STR |
|---|---|
| PEx64-3 | 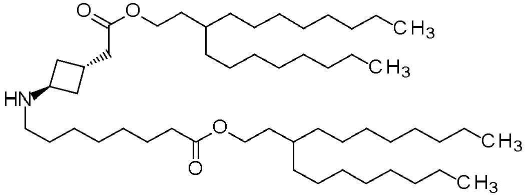 |
| Ex64 | 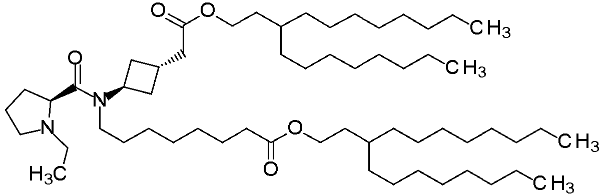 |
| PEx65-1 | 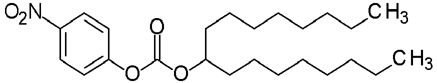 |
| PEx65-2 | 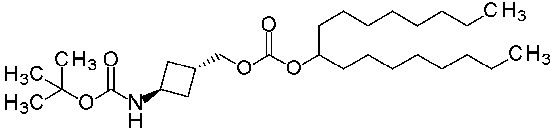 |
| PEx65-3 | 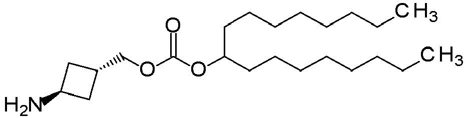 |
| PEx65-4 | 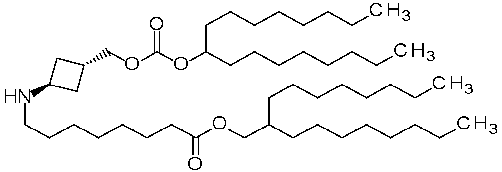 |

**[Table 41]**

| NUM | STR |
|---|---|
| Ex65 | 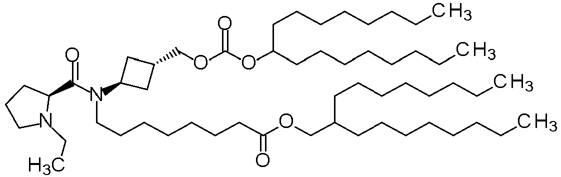 |
| PEx66-1 | 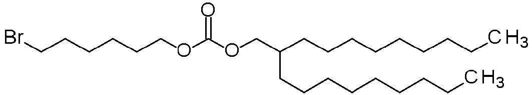 |
| PEx66-2 | 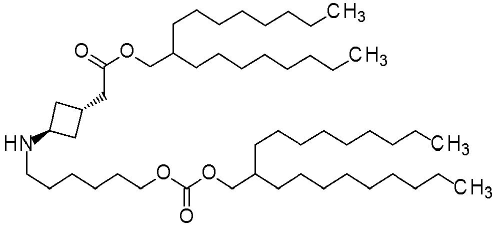 |
| Ex66 | 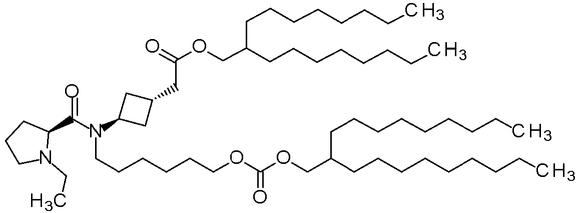 |
| PEx67-1 | 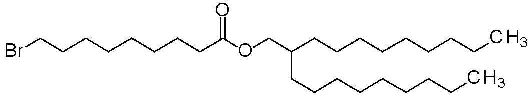 |
| PEx67-2 | 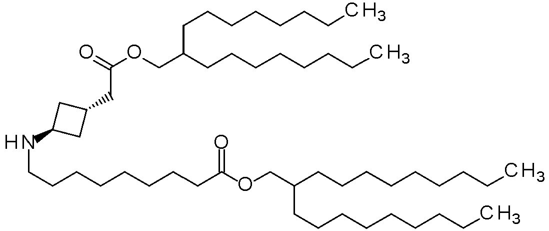 |

**[Table 42]**

| NUM | STR |
|---|---|
| Ex67 | 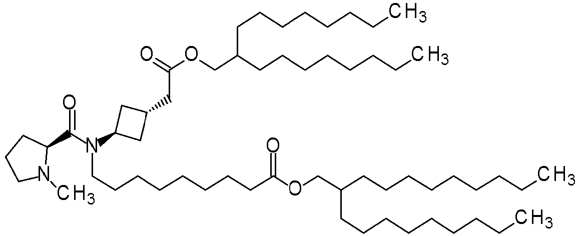 |
| Ex68 | 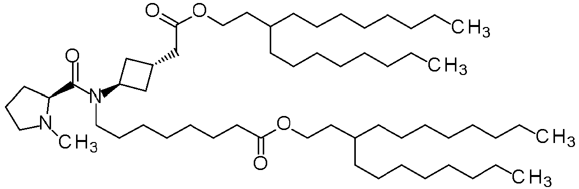 |
| Ex69 | 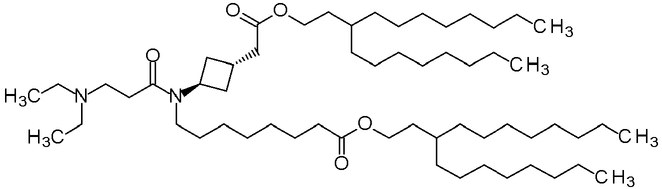 |
| Ex70 | 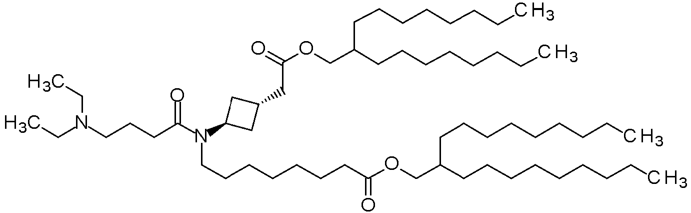 |
| PEx71-1 | 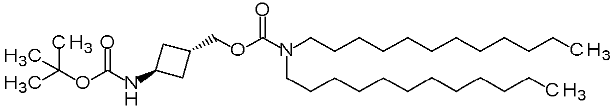 |
| PEx71-2 | 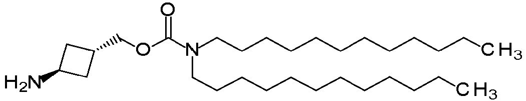 |

**[Table 43]**

| NUM | STR |
|---|---|
| PEx71-3 | 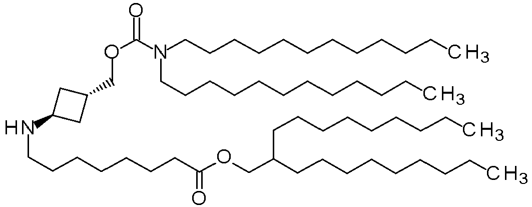 |
| Ex71 | 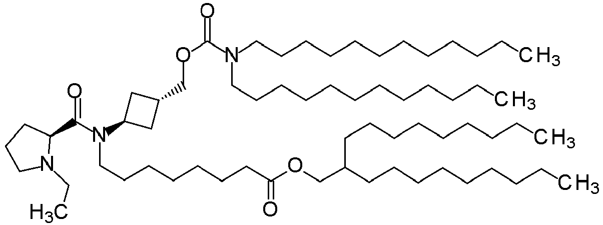 |
| PEx72-1 | 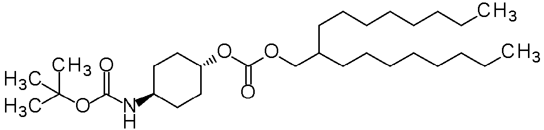 |
| PEx72-2 | 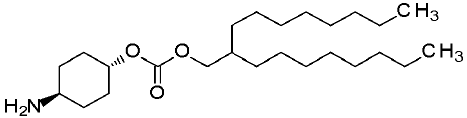 |
| PEx72-3 | 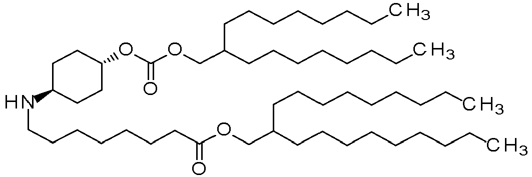 |
| Ex72 | 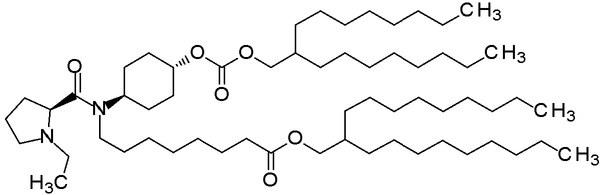 |

**[Table 44]**

| NUM | STR |
|---|---|
| PEx73-1 | 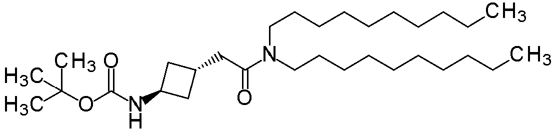 |
| PEx73-2 | 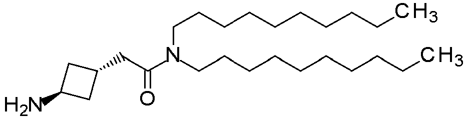 |
| PEx73-3 | 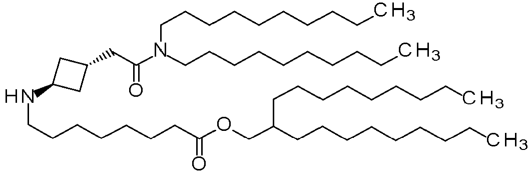 |
| Ex73 | 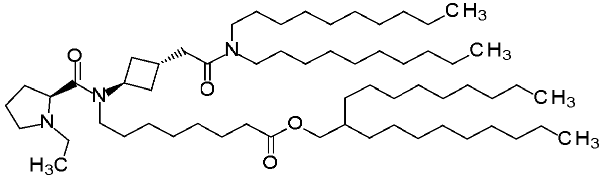 |

**[Table 45]**

| NUM | STR |
|---|---|
| PEx74-1 | 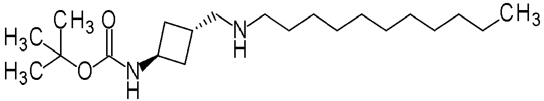 |
| PEx74-2 | 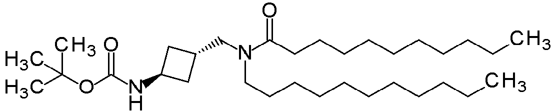 |
| PEx74-3 | 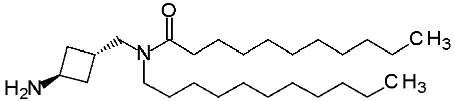 |
| PEx74-4 | 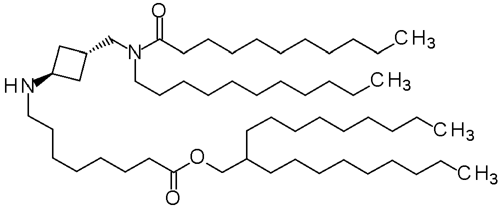 |
| Ex74 | 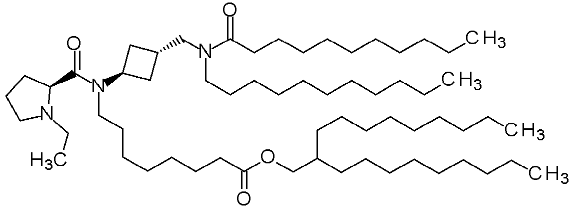 |

**[Table 46]**

| NUM | STR |
|---|---|
| PEx75-1 | 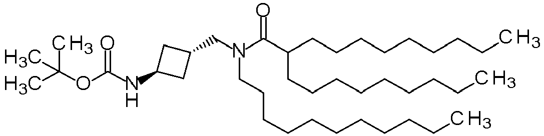 |
| PEx75-2 | 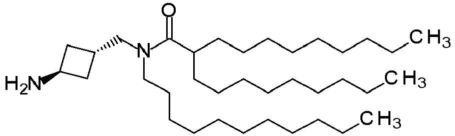 |
| PEx75-3 | 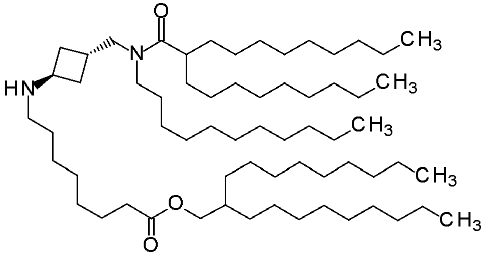 |
| Ex75 | 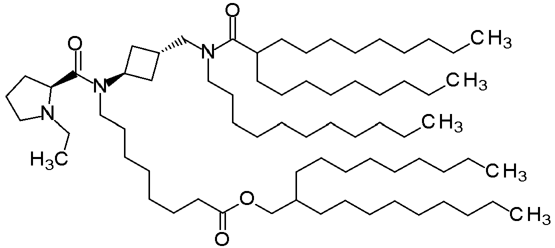 |

**[Table 47]**

| NUM | REF | DAT |
|---|---|---|
| PEx1-1 | PEx1-1 | CI+: 503.1, 505.3 |
| PEx1-2 | PEx1-2 | ESI+: 504.5 |
| PEx1-3 | PEx1-3 | ESI+: 382.5 |
| PEx1-4 | PEx1-4 | ESI+: 805.0 |
| Ex1 | Ex1 | ESI+: 916.1 |
| | | NMR (CDCl₃): 0.79-0.97 (m, 12H), 1.10-2.11 (m, 78H), 2.21-2.47 (m, 8H), 2.49-2.61 (m, 3H), 2.99-3.20 (m, 2H), 3.23-3.38 (m, 2H), 3.90-4.03 (m, 4H), 4.70-4.82 (m, 1H) |
| | | HPLC Rt: 24.7 min. |
| Ex2 | Ex2 | ESI+: 916.0 |
| | | HPLC Rt: 30.4 min. |
| Ex3 | Ex1 | ESI+: 889.9 |
| PEx4-1 | PEx4-1 | ESI+: 516.6 |
| PEx4-2 | PEx4-2 | ESI+: 394.5 |
| PEx4-3 | PEx4-3 | ESI+: 817.0 |
| Ex4 | Ex4 | ESI+: 928.0 |
| | | NMR (CDCl₃): 0.81-0.96 (m, 12H), 1.09-1.68 (m, 72H), 1.71-2.07 (m, 4H), 2.09-2.24 (m, 6H), 2.25-2.44 (m, 6H), 2.61 (br s, 2H), 3.01-3.50 (m, 4H), 3.91-4.05 (m, 4H) |
| PEx5-1 | PEx5-1 | ESI+: 365.3 |
| PEx5-2 | PEx1-4 | ESI+: 688.8 |
| Ex5 | Ex2 | ESI+: 799.9 |
| Ex6 | Ex2 | ESI+: 930.1 |
| | | HPLC Rt: 24.6 min. |
| Ex7 | Ex7 | ESI+: 932.2 |
| | | NMR (CDCl₃): 0.80-0.95 (m, 12H), 1.04 (t, *J* = 7.11 Hz, 6H), 1.19-1.70 (m, 72H), 1.97-2.11 (m, 2H), 2.22-2.61 (m, 13H), 2.72-2.84 (m, 2H), 3.14-3.40 (m, 2H), 3.89-4.03 (m, 4H), 4.40-4.86 (m, 1H) |
| Ex8 | Ex2 | ESI+: 932.1 |
| PEx9-1 | PEx9-1 | ESI+: 243.3 |
| PEx9-2/HCl | PEx9-2 | ESI+: 187.1 |
| Ex9 | Ex2 | ESI+: 973.2 |
| Ex10 | Ex2 | ESI+: 930.1 |

**[Table 48]**

| NUM | REF | DAT |
|---|---|---|
| PEx11-1 | PEx1-2 | ESI+: 504.6 |
| PEx11-2 | PEx1-3 | ESI+: 382.5 |
| PEx11-3 | PEx1-4 | ESI+: 805.1 |
| Ex11 | Ex2 | ESI+: 916.1 |
| PEx12-1 | PEx12-1 | ESI+: 215.2 |
| Ex12 | Ex12 | ESI+: 987.2 |
| | | NMR (CDCl₃): 0.80-0.96 (m, 12H), 0.98-1.10 (m, 6H), 1.10-1.71 (m, 72H), 1.96-2.12 (m, 2H), 2.20-2.35 (m, 3H), 2.35-2.49 (m, 1H), 2.49-2.66 (m, 9H), 2.67-2.93 (m, 6H), 2.94-3.10 (m, 1H), 3.19-3.36 (m, 2H), 3.89-4.06 (m, 4H), 4.52-4.80 (m, 1H) |
| Ex13 | Ex2 | ESI+: 902.0 |
| PEx14-1 | PEx14-1 | ESI+: 486.6 |
| PEx14-2 | PEx1-3 | ESI+: 364.5 |
| PEx14-3 | PEx1-4 | ESI+: 787.0 |
| Ex14 | Ex2 | ESI+: 898.1 |
| PEx15-1 | PEx1-2 | ESI+: 518.6 |
| PEx15-2 | PEx1-3 | ESI+: 396.6 |
| PEx15-3 | PEx1-4 | ESI+: 819.1 |
| Ex15 | Ex2 | ESI+: 930.1 |
| PEx16-1 | PEx16-1 | ESI+: 516.7 |
| PEx16-2 | Ex2 | ESI+: 627.8 |
| PEx16-3 | PEx16-3 | ESI+: 537.7 |
| Ex16 | Ex16 | ESI+: 800.0 |
| Ex17 | Ex2 | ESI+: 916.0 |
| Ex18 | Ex16 | ESI+: 864.0 |
| Ex19 | Ex2 | ESI+: 944.0 |
| PEx20-1 | PEx1-2 | ESI+: 476.6 |
| PEx20-2 | PEx1-3 | ESI+: 354.6 |
| PEx20-3 | PEx1-4 | ESI+: 776.9 |
| Ex20 | Ex2 | ESI+: 888.1 |

**[Table 49]**

| NUM | REF | DAT |
|---|---|---|
| Ex21 | Ex21 | ESI+: 945.1 |
| | | NMR (CDCl₃): 0.82-0.95 (m, 12H), 1.07-1.70 (m, 72H), 1.98-2.11 (m, 2H), 2.21-2.74 (m, 18H), 3.13 (s, 2H), 3.26-3.35 (m, 2H), 3.91-4.04 (m, 4H), 4.56-4.76 (m, 1H) |
| PEx22-1 | Ex2 | ESI+: 1038.3 |
| PEx22-2 | PEx22-2 | ESI+: 916.0 |
| Ex22 | Ex22 | ESI+: 944.2 |
| PEx23-1 | PEx1-2 | ESI+: 532.6 |
| PEx23-2 | PEx1-3 | ESI+: 410.6 |
| PEx23-3 | PEx1-4 | ESI+: 833.1 |
| Ex23 | Ex2 | ESI+: 944.2 |
| PEx24-1 | PEx24-1 | ESI+: 458.5 |
| PEx24-2 | PEx24-2 | ESI+: 506.6 |
| PEx24-3 | PEx1-3 | ESI+: 384.5 |
| PEx24-4 | PEx1-4 | ESI+: 807.0 |
| Ex24 | Ex2 | ESI+: 918.0 |
| PEx25-1 | PEx25-1 | ESI+: 520.6 |
| PEx25-2 | PEx25-2 | ESI+: 398.5 |
| PEx25-3 | PEx25-3 | ESI+: 821.0 |
| Ex25 | Ex25 | ESI+: 932.1 |
| | | NMR (CDCl₃): 0.81-0.96 (m, 12H), 1.06-1.71 (m, 72H), 1.71-2.45 (m, 14H), 2.45-2.60 (m, 1H), 2.96-3.09 (m, 1H), 3.09-3.20 (m, 1H), 3.22-3.40 (m, 2H), 3.92-4.10 (m, 4H), 4.16-4.25 (m, 2H), 4.56-4.86 (m, 1H) |
| Ex26 | Ex26 | ESI+: 964.1 |
| Ex27 | Ex26 | ESI+: 989.1 |
| Ex28 | Ex28 | ESI+: 933.2 |
| Ex29 | Ex2 | ESI+: 942.2 |
| PEx30-1 | PEx30-1 | ESI+: 469.5, 471.4 |
| PEx30-2 | PEx30-2 | ESI+: 490.6 |
| PEx30-3 | PEx30-3 | ESI+: 368.5 |
| PEx30-4 | PEx30-4 | ESI+: 734.9 |
| Ex30 | Ex30 | ESI+: 846.1 |
| | | NMR (CDCl₃): 0.83-0.94 (m, 12H), 1.08-2.15 (m, 72H), 2.17-2.40 (m, 7H), 2.98-3.22 (m, 4H), 3.80-4.33 (m, 5H) |

**[Table 50]**

| NUM | REF | DAT |
|---|---|---|
| Ex31 | Ex31 | ESI+: 930.0 |
| | | NMR (CDCl₃): 0.83-0.93 (m, 12H), 1.03-1.68 (m, 75H), 1.69-1.87 (m, 2H), 1.87-2.13 (m, 4H), 2.17-2.47 (m, 6H), 2.48-2.60 (m, 3H), 2.61-2.74 (m, 1H), 3.14-3.40 (m, 4H), 3.90-4.05 (m, 4H), 4.66-4.99 (m, 1H) |
| | | HPLC Rt: 25.7 min. |
| Ex33 | Ex2 | ESI+: 918.2 |
| PEx34-1/HCl | PEx34-1 | ESI+: 158.1 |
| Ex34 | Ex2 | ESI+: 944.2 |
| PEx35-1 | PEx1-1 | ESI+: 611.7 |
| PEx35-2 | PEx1-4 | ESI+: 871.1 |
| Ex35 | Ex2 | ESI+: 982.1 |
| PEx36-1 | PEx1-4 | ESI+: 791.0 |
| Ex36 | Ex2 | ESI+: 901.9 |
| PEx37-1 | PEx1-1 | ESI+: 497.5, 499.5 |
| PEx37-2 | PEx1-2 | ESI+: 518.6 |
| PEx37-3 | PEx1-3 | ESI+: 396.5 |
| PEx37-4 | PEx1-4 | ESI+: 791.1 |
| Ex37 | Ex2 | ESI+: 902.0 |
| PEx38-1 | PEx1-4 | ESI+: 692.5 |
| Ex38 | Ex2 | ESI+: 803.9 |
| PEx39-1 | PEx1-4 | ESI+: 524.7 |
| PEx39-2 | Ex2 | ESI+: 635.8 |
| Ex39 | Ex39 | ESI+: 827.9 |
| PEx40-1 | PEx1-1 | ESI+: 429.4 |
| PEx40-2 | PEx1-4 | ESI+: 706.5 |
| Ex40 | Ex2 | ESI+: 818.0 |
| PEx41-1 | PEx1-4 | ESI+: 776.9 |
| Ex41 | Ex2 | ESI+: 888.0 |
| PEx42-1 | PEx1-1 | ESI+: 513.3 |
| PEx42-2 | PEx1-4 | ESI+: 790.2 |
| Ex42 | Ex2 | ESI+: 901.9 |

**[Table 51]**

| NUM | REF | DAT |
|---|---|---|
| PEx43-1 | PEx1-1 | ESI+: 581.3, 583.2 |
| PEx43-2 | PEx1-4 | ESI+: 861.0 |
| Ex43 | Ex2 | ESI+: 972.0 |
| PEx44-1 | PEx1-2 | ESI+: 462.5 |
| PEx44-2 | PEx1-3 | ESI+: 340.4 |
| PEx44-3 | PEx1-4 | ESI+: 692.8 |
| Ex44 | Ex2 | ESI+: 803.9 |
| Ex45 | Ex2 | ESI+: 817.9 |
| Ex46 | Ex2 | ESI+: 819.9 |
| PEx47-1 | PEx1-1 | ESI+: 595.3, 597.2 |
| PEx47-2 | PEx1-4 | ESI+: 875.0 |
| Ex47 | Ex2 | ESI+: 986.0 |
| PEx48-1 | PEx1-1 | ESI+: 513.2 |
| PEx48-2 | PEx1-4 | ESI+: 791.1 |
| Ex48 | Ex2 | ESI+: 902.0 |
| PEx49-1 | PEx49-1 | ESI+: 567.5 |
| PEx49-2 | PEx49-2 | ESI+: 574.6 |
| PEx49-3 | PEx49-3 | ESI+: 452.5 |
| PEx49-4 | PEx49-4 | ESI+: 917.0 |
| Ex49 | Ex49 | ESI+: 1028.0 |
| | | NMR (CDCl₃): 0.81-0.95 (m, 12H), 1.06-1.69 (m, 88H), 1.70-2.13 (m, 6H), 2.20-2.47 (m, 8H), 2.48-2.62 (m, 3H), 2.96-3.20 (m, 2H), 3.21-3.42 (m, 2H), 3.99-4.17 (m, 4H), 4.66-4.83 (m, 1H) |
| Ex50 | Ex2 | ESI+: 1042.1 |
| Ex51 | Ex2 | ESI+: 1044.1 |
| PEx52-1 | PEx1-4 | ESI+: 748.8 |
| Ex52 | Ex2 | ESI+: 859.9 |
| PEx53-1 | PEx59-1 | ESI+: 401.2 |
| PEx53-2 | PEx59-2 | ESI+: 532.5 |
| PEx53-3 | PEx1-3 | ESI+: 410.4 |
| PEx53-4 | PEx1-4 | ESI+: 836.8 |
| Ex53 | Ex2 | ESI+: 961.9 |

**[Table 52]**

| NUM | REF | DAT |
|---|---|---|
| PEx54-1 | PEx1-4 | ESI+: 833.0 |
| Ex54 | Ex2 | ESI+: 957.9 |
| PEx55-1 | PEx55-1 | ESI+: 641.6 |
| PEx55-2 | PEx55-2 | ESI+: 551.6 |
| Ex55 | Ex55 | ESI+: 877.8 |
| | | NMR (CDCl₃): 0.81-0.94 (m, 12H), 1.02-2.15 (m, 64H), 2.17-2.75 (m, 10H), 3.14-3.35 (m, 2H), 3.36-3.45 (m, 2H), 3.50-3.72 (m, 4H), 3.90-4.23 (m, 4H), 4.45-4.52 (m, 1H), 4.60-5.05 (m, 1H) |
| PEx56-1 | PEx56-1 | ESI+: 486.4 |
| PEx56-2 | PEx56-2 | ESI+: 548.5 |
| PEx56-3 | PEx56-3 | ESI+: 426.5 |
| PEx56-4 | PEx1-4 | ESI+: 848.9 |
| Ex56 | Ex2 | ESI+: 973.8 |
| PEx57-1 | PEx24-2 | ESI+: 548.5 |
| PEx57-2 | PEx1-3 | ESI+: 426.5 |
| PEx57-3 | PEx1-4 | ESI+: 848.9 |
| Ex57 | Ex2 | ESI+: 974.0 |
| PEx58-1 | PEx58-1 | ESI+: 471.4 |
| PEx58-2 | PEx58-2 | ESI+: 792.8 |
| Ex58 | Ex58 | ESI+: 918.0 |
| | | NMR (CDCl₃): 0.83-0.93 (m, 12H), 1.02-1.87 (m, 73H), 1.88-2.43 (m, 10H), 2.44-2.60 (m, 1H), 2.60-2.75 (m, 1H), 3.14-3.29 (m, 2H), 3.29-3.45 (m, 2H), 3.98-4.13 (m, 4H), 4.15-4.25 (m, 2H), 4.55-5.04 (m, 1H) |
| PEx59-1 | PEx59-1 | ESI+: 403.2 |
| PEx59-2 | PEx59-2 | ESI+: 534.5 |
| PEx59-3 | PEx1-3 | ESI+: 412.5 |
| PEx59-4 | PEx1-4 | ESI+: 834.9 |
| Ex59 | Ex2 | ESI+: 960.0 |
| PEx60-1 | PEx1-4 | ESI+: 838.9 |
| Ex60 | Ex2 | ESI+: 963.9 |
| PEx61-1 | PEx1-1 | ESI+: 555.2 |
| PEx61-2 | PEx1-4 | ESI+: 833.0 |
| Ex61 | Ex2 | ESI+: 944.1 |

**[Table 53]**

| NUM | REF | DAT |
|---|---|---|
| PEx62-1 | PEx1-4 | ESI+: 889.0 |
| Ex62 | Ex2 | ESI+: 1000.0 |
| PEx63-1 | PEx63-1 | ESI+: 491.5 |
| PEx63-2 | PEx1-3 | ESI+: 369.4 |
| PEx63-3 | PEx1-4 | ESI+: 791.9 |
| Ex63 | Ex2 | ESI+: 916.9 |
| PEx64-1 | PEx1-2 | ESI+: 518.5 |
| PEx64-2 | PEx1-3 | ESI+: 396.5 |
| PEx64-3 | PEx1-4 | ESI+: 805.0 |
| Ex64 | Ex2 | ESI+: 930.0 |
| PEx65-1 | PEx24-1 | ESI+: 444.4 |
| PEx65-2 | PEx24-2 | ESI+: 506.5 |
| PEx65-3 | PEx1-3 | ESI+: 384.4 |
| PEx65-4 | PEx1-4 | ESI+: 778.9 |
| Ex65 | Ex2 | ESI+: 903.9 |
| PEx66-1 | PEx66-1 | ESI+: 529.4 |
| PEx66-2 | PEx1-4 | ESI+: 806.9 |
| Ex66 | Ex2 | ESI+: 931.9 |
| PEx67-1 | PEx1-1 | ESI+: 541.5 |
| PEx67-2 | PEx1-4 | ESI+: 819.0 |
| Ex67 | Ex2 | ESI+: 930.0 |
| Ex68 | Ex2 | ESI+: 915.9 |
| Ex69 | Ex2 | ESI+: 931.9 |
| Ex70 | Ex2 | ESI+: 946.0 |
| PEx71-1 | PEx63-1 | ESI+: 603.6 |
| PEx71-2 | PEx1-3 | ESI+: 481.6 |
| PEx71-3 | PEx1-4 | ESI+: 904.0 |
| Ex71 | Ex2 | ESI+: 1029.1 |
| PEx72-1 | PEx72-1 | ESI+: 534.5 |
| PEx72-2 | PEx1-3 | ESI+: 412.5 |
| PEx72-3 | PEx1-4 | ESI+: 835.0 |
| Ex72 | Ex2 | ESI+: 960.0 |

**[Table 54]**

| NUM | REF | DAT |
|---|---|---|
| PEx73-1 | PEx73-1 | ESI+: 531.6 |
| PEx73-2 | PEx1-3 | ESI+: 409.5 |
| PEx73-3 | PEx1-4 | ESI+: 832.0 |
| Ex73 | Ex2 | ESI+: 957.0 |
| PEx74-1 | PEx74-1 | ESI+: 355.5 |
| PEx74-2 | PEx74-2 | ESI+: 523.6 |
| PEx74-3 | PEx1-3 | ESI+: 423.5 |
| PEx74-4 | PEx1-4 | ESI+: 846.2 |
| Ex74 | Ex2 | ESI+: 971.3 |
| PEx75-1 | PEx74-2 | ESI+: 649.8 |
| PEx75-2 | PEx1-3 | ESI+: 549.7 |
| PEx75-3 | PEx1-4 | ESI+: 972.2 |
| Ex75 | Ex2 | ESI+: 1097.2 |

### Example 76: Production of Nucleic Acid Lipid Nanoparticle

### (Raw Materials of Nucleic Acid Lipid Nanoparticle)

DOTAP manufactured by NOF CORPORATION (product name: COATSOME (registered trademark) CL-8181TA), DOTMA manufactured by NOF CORPORATION (product name: COATSOME (registered trademark) CL-E8181TA), DSPC manufactured by NOF CORPORATION (product name: COATSOME (registered trademark) MC-8080), DPPC manufactured by NOF CORPORATION (product name: COATSOME (registered trademark) MC-6060), DHSM manufactured by NIPPON FINE CHEMICAL CO., LTD., SOPC manufactured by Avanti Polar Lipids (catalog number: 850467P), DoPhPE manufactured by Avanti Polar Lipids (catalog number: 999985P), DOPS manufactured by Larodan (catalog number: 38-1810), DOPE manufactured by NOF CORPORATION (product name: COATSOME (registered trademark) ME-8181), choresterol manufactured by NIPPON FINE CHEMICAL CO., LTD. (product name: Cholesterol HP), β-sitosterol manufactured by Sigma-Aldrich (catalog number: S1270), 7α-hydroxycholesterol manufactured by Avanti Polar Lipids (catalog number: 700034P), campesterol manufactured by Tama Biochemical Co., Ltd. (catalog number: 306-01391), DMG-PEG2000 manufactured by NOF CORPORATION (SUNBRIGHT (registered trademark) GM-020), C8 PEG2000 ceramide manufactured by Avanti Polar Lipids (catalog number: 880170P), PEG monostearate (polyethylene glycol monostearate) manufactured by FUJIFILM Wako Pure Chemical Corporation (catalog number: 320-32585), and mRNA (FLuc mRNA, ND1 mRNA, eGFP mRNA) manufactured by TriLink BioTechnologies were used.

### (Preparation of Nucleic Acid Lipid Nanoparticle)

In Ex1-L1 to Ex1-L54, the compound of the formula (I) or the salt thereof, which is a cationic lipid, a phospholipid, a sterol, and a PEGylated lipid were dissolved in ethanol with the lipid composition ratio and the N/P ratio shown in the tables below, and thus oil phases were obtained. FLuc mRNA was diluted with 10 mM citrate buffer (pH 4) to 85 µg/mL, and thus an aqueous phase was obtained. The oil phase and the aqueous phase were mixed at a volume ratio of oil phase:aqueous phase=1:3 with a microfluidic device (NanoAssemblr (registered trademark), manufactured by Precision NanoSystems), and the mixture solution was diluted two-fold or three-fold with phosphate-buffered saline (PBS). Thus, a dispersion of nucleic acid lipid nanoparticle was obtained. The dispersion was dialyzed using PBS to remove ethanol and concentrated by ultrafiltration to adjust to a certain concentration, and nucleic acid lipid nanoparticle of an Example lipid were thus obtained. The particle sizes and the encapsulation efficiencies of the nucleic acid lipid nanoparticle (nucleic acid: Fluc mRNA) are shown in the tables below. Ex1-L1 to Ex1-L54 shown in NUM in the tables below indicate Ex1-L1-FLuc mRNA to Ex1-L54-FLuc mRNA, respectively.

**[Table 55]**

| NUM | Lipid Composition (mol%) Ex1 \| DSPC \| CHO \| DMG-PEG2000 | N/P ratio |
|---|---|---|
| Ex1-L1 | 50 \| 10 \| 38.5 \| 1.5 | 6 |
| Ex1-L2 | 20 \| 16.2 \| 62.3 \| 1.5 | 6 |
| Ex1-L3 | 30 \| 14.1 \| 54.4 \| 1.5 | 6 |
| Ex1-L4 | 37.5 \| 12.5 \| 48.5 \| 1.5 | 6 |
| Ex1-L5 | 40 \| 6 \| 52.5 \| 1.5 | 6 |
| Ex1-L6 | 40 \| 12 \| 46.5 \| 1.5 | 6 |
| Ex1-L7 | 40 \| 12 \| 47 \| 1 | 6 |
| Ex1-L8 | 40 \| 18.1 \| 40.4 \| 1.5 | 6 |
| Ex1-L9 | 42.5 \| 11.5 \| 44.5 \| 1.5 | 6 |
| Ex1-L10 | 45 \| 11 \| 42.5 \| 1.5 | 6 |
| Ex1-L11 | 47.5 \| 10.5 \| 40.5 \| 1.5 | 6 |
| Ex1-L12 | 50 \| 5 \| 43.5 \| 1.5 | 6 |
| Ex1-L13 | 50 \| 10 \| 37.5 \| 2.5 | 6 |
| Ex1-L14 | 50 \| 10 \| 38 \| 2 | 6 |
| Ex1-L15 | 50 \| 10 \| 39 \| 1 | 6 |
| Ex1-L16 | 50 \| 15 \| 33.5 \| 1.5 | 6 |
| Ex1-L17 | 52.5 \| 9.5 \| 36.5 \| 1.5 | 6 |
| Ex1-L18 | 55 \| 9 \| 34.5 \| 1.5 | 6 |
| Ex1-L19 | 55 \| 13.5 \| 30 \| 1.5 | 6 |
| Ex1-L20 | 57.5 \| 8.5 \| 32.5 \| 1.5 | 6 |
| Ex1-L21 | 60 \| 4 \| 34.5 \| 1.5 | 6 |
| Ex1-L22 | 60 \| 8 \| 30.5 \| 1.5 | 6 |
| Ex1-L23 | 60 \| 13 \| 25.5 \| 1.5 | 6 |
| Ex1-L24 | 70 \| 5.9 \| 22.6 \| 1.5 | 6 |
| Ex1-L25 | 80 \| 4 \| 14.5 \| 1.5 | 6 |
| Ex1-L26 | 50 \| 10 \| 38.5 \| 1.5 | 3 |
| Ex1-L27 | 40 \| 12 \| 46.5 \| 1.5 | 4.5 |
| Ex1-L28 | 45 \| 11 \| 42.5 \| 1.5 | 4.5 |
| Ex1-L29 | 50 \| 10 \| 38.5 \| 1.5 | 4.5 |
| Ex1-L30 | 55 \| 9 \| 34.5 \| 1.5 | 4.5 |
| Ex1-L31 | 50 \| 10 \| 38.5 \| 1.5 | 7.5 |
| Ex1-L32 | 50 \| 10 \| 38.5 \| 1.5 | 9 |
| Ex1-L49 | 55.6 \| 0 \| 42.8 \| 1.7 | 6 |
| Ex1-L50 | 81.3 \| 16.3 \| 0 \| 2.4 | 6 |
| Ex1-L51 | 51 \| 10.2 \| 38.3 \| 0.5 | 6 |

**[Table 56]**

| NUM | Lipid Composition (mol%) | N/P ratio |
|---|---|---|
| Ex1-L33 | Ex1 \| DOTAP \| CHO \| DMG = 35 \| 40 \| 23.5 \| 1.5 | 6 |
| Ex1-L34 | Ex1 \| DPPC \| CHO \| DMG = 40 \| 12 \| 46.5 \| 1.5 | 6 |
| Ex1-L35 | Ex1 \| DPPC \| CHO \| DMG = 50 \| 10 \| 38.5 \| 1.5 | 6 |
| Ex1-L36 | Ex1 \| DPPC \| CHO \| DMG = 50 \| 15 \| 33.5 \| 1.5 | 6 |
| Ex1-L37 | Ex1 \| DPPC \| CHO \| DMG = 60 \| 13 \| 25.5 \| 1.5 | 6 |
| Ex1-L38 | Ex1 \| DHSM \| CHO \| DMG = 40 \| 12 \| 46.5 \| 1.5 | 6 |
| Ex1-L39 | Ex1 \| DHSM \| CHO \| DMG = 50 \| 10 \| 38.5 \| 1.5 | 6 |
| Ex1-L40 | Ex1 \| DHSM \| CHO \| DMG = 50 \| 15 \| 33.5 \| 1.5 | 6 |
| Ex1-L41 | Ex1 \| DHSM \| CHO \| DMG = 60 \| 13 \| 25.5 \| 1.5 | 6 |
| Ex1-L42 | Ex1 \| SOPC \| CHO \| DMG = 50 \| 10 \| 38.5 \| 1.5 | 6 |
| Ex1-L43 | Ex1 \| DoPhPE \| CHO \| DMG = 50 \| 10 \| 38.5 \| 1.5 | 6 |
| Ex1-L44 | Ex1 \| DSPC \| DOPS \| CHO \| DMG = 50 \| 7.5 \| 2.5 \| 38.5 \| 1.5 | 6 |
| Ex1-L45 | Ex1 \| DSPC \| CHO \| β-sitosterol \| DMG = 50 \| 10 \| 20 \| 18.5 \| 1.5 | 6 |
| Ex1-L46 | Ex1 \| DSPC \| CHO \| 7α-OH-CHO \| DMG = 50 \| 10 \| 10 \| 28.5 \| 1.5 | 6 |
| Ex1-L47 | Ex1 \| DSPC \| CHO \| PEG Ceramide = 50 \| 10 \| 38.5 \| 1.5 | 6 |
| Ex1-L48 | Ex1 \| DSPC \| CHO \| PEG Monostearate = 50 \| 10 \| 38.5 \| 1.5 | 6 |
| Ex1-L52 | Ex1 \| DOTAP \| DOPE \| CHO \| DMG = 40 \| 10 \| 10 \| 38.5 \| 1.5 | 6 |
| Ex1-L53 | Ex1 \| DOTMA \| DOPE \| CHO \| DMG = 40 \| 10 \| 10 \| 38.5 \| 1.5 | 6 |
| Ex1-L54 | Ex1 \| DSPC \| Campesterol \| DMG = 50 \| 10 \| 38.5 \| 1.5 | 6 |

The abbreviations in the tables are CHO: Cholesterol, DMG: DMG-PEG2000, 7α-OH-CHO: 7α-hydroxycholesterol, PEG Ceramide: C8 PEG2000 ceramide, and PEG Monostearate: Polyethylene Glycol Monostearate.

Ex1-L1-FLuc mRNA was pharmacologically evaluated in Test Example 1, and the ratios of each lipid component of the nucleic acid lipid nanoparticles that exhibited fluorescent intensities of 0.01 or more and 0.1 or more were determined when the fluorescent intensity thereof was set as standard (=1). As a result, the composition ratio of Ex1 was 20.0 to 80.0 mol% or 30.0 to 60.0 mol% based on the total amount of the lipid nanoparticle, respectively. The composition ratio of the neutral lipid was 18.5 to 78.5 mol% or 38.5 to 68.5 mol% based on the total amount of the lipid nanoparticle, respectively. The composition ratio of the PEGylated lipid was 0.5 to 2.5 mol% or 0.5 to 2.0 mol% based on the total amount of the lipid nanoparticle,respectively.

### (Raw Materials of ND1 Nucleic Acid Lipid Nanoparticle - ND1 Nucleic Acids)

Two types of ND1 mRNA encoding human ND1 ((i) mRNA which had a base sequence containing a 5' UTR and a 3' UTR provided from TriLink BioTechnologies, CDS of human ND1 gene (SEQ ID NO: 1), and a poly(A) sequence of 120 bases and in which the 5' cap structure was Cap-1 (called "ND1-1" below) and (ii) mRNA which had a base sequence (SEQ ID NO: 5) in which all the uridines were substituted with N1-methylpseudouridines in a base sequence (SEQ ID NO: 4) containing a 5' UTR derived from human α-globin gene, a 3' UTR derived from human α-globin gene, CDS of human ND1 gene (SEQ ID NO: 3), and a poly(A) sequence of 79 polynucleotides and in which the 5' cap structure was Cap-1 (called "ND1-2" below)) were produced (outsourced to TriLink BioTechnologies). In this regard, in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) sequence. FLuc mRNA and eGFP mRNA manufactured by TriLink BioTechnologies (product names CleanCap (registered trademark) FLuc mRNA and CleanCap (registered trademark) EGFP mRNA) were used.

### (Preparation of ND1 Nucleic Acid Lipid Nanoparticle)

ND1 nucleic acid lipid nanoparticle were prepared specifically by the method below. Example compound Ex1, which is a cationic lipid, DSPC, cholesterol, and DMG-PEG2000 were dissolved in ethanol at N/P ratio=6, and thus an oil phase was obtained. The oil phase was mixed with 10 mM citrate buffer (pH 4) containing the mRNA as an aqueous phase at a volume ratio of oil phase:aqueous phase=1:3 with a microfluidic device (NanoAssemblr (registered trademark), Precision NanoSystems),, and the mixture solution was diluted two-fold with PBS. Thus, a dispersion of ND1 nucleic acid lipid nanoparticle was obtained. Ethanol was removed through dialysis of the dispersion. Subsequently, the dispersion was concentrated through ultrafiltration, and ND1 nucleic acid lipid nanoparticle prepared at a certain concentration were thus obtained.

In Test Example 4, ND1 nucleic acid lipid nanoparticle were prepared specifically by the method below. Example compound Ex1, which is a cationic lipid, DSPC (manufactured by NIPPON FINE CHEMICAL CO., LTD.), cholesterol (manufactured by Merck KGaA), and DMG-PEG2000 (manufactured by Merck KGaA) were dissolved in ethanol at N/P ratio=6, and thus an oil phase was obtained. Using 10 mM citrate buffer (pH 4) containing the mRNA as an aqueous phase, the oil phase and the aqueous phase were mixed at a volume ratio of oil phase:aqueous phase=1:3 with a microfluidic device (NanoAssemblr (registered trademark), Ignite+ (registered trademark), Precision NanoSystems). The mixture solution was diluted with Tris buffer, and a dispersion of ND1 nucleic acid lipid nanoparticle was obtained. The dispersion was adjusted to around pH 7 using 0.1 M Tris buffer and then purified using tangential flow filtration (TFF), and ND1 nucleic acid lipid nanoparticle prepared at a certain concentration were thus obtained.

The lipid nanoparticle in which the nucleic acid lipid nanoparticle were prepared using 10 mM citrate buffer (pH 4) and which contained Ex1, DSPC, cholesterol, and DMG-PEG2000 (ratio by mole of 50/10/38.5/1.5) as the components are called Ex1-L1-ND1 mRNA. Similarly, the lipid nanoparticle which encapsulated eGFP mRNA and which had the lipid composition of Ex1-L1 are called Ex1-L1-eGFP mRNA.

### (Raw Materials of Fluc eGFP Mixture Nucleic Acid Lipid Nanoparticle)

Fluc mRNA, which encodes firefly luciferase protein, and eGFP mRNA (TriLink BioTechnologies), which encodes green fluorescent protein, were used and mixed at a mixture ratio by mole of 1:1, and thus Fluc eGFP mixture mRNA was obtained.

### (Preparation of Fluc eGFP Mixture Nucleic Acid Lipid Nanoparticle)

Fluc eGFP mixture nucleic acid lipid nanoparticle were prepared specifically by the method below. An Example compound (Ex6 or Ex7), DSPC, the cholesterol, and DMG-PEG2000 were dissolved in ethanol at N/P ratio=6, and thus an oil phase was obtained. The oil phase was added to 10 mM citrate buffer (pH 4) containing the mRNA as an aqueous phase in such a manner that the volume ratio of the aqueous phase and the oil phase became oil phase:aqueous phase=1:3 and mixed with a microfluidic device (NanoAssemblr (registered trademark), manufactured by Precision NanoSystems), and the mixture solution was diluted two-fold with PBS. Thus, a dispersion of Fluc eGFP mixture nucleic acid lipid nanoparticle was obtained. Ethanol was removed through dialysis of the dispersion. Subsequently, the dispersion was concentrated through ultrafiltration, and Fluc eGFP mixture nucleic acid lipid nanoparticle adjusted to a certain concentration were thus obtained.

The Fluc eGFP mixture nucleic acid lipid nanoparticle in which the nucleic acid lipid nanoparticle were prepared using 10 mM citrate buffer (pH 4) and which contained the Example compounds (Ex6 and Ex7), DSPC, the cholesterol, and DMG-PEG2000 (ratio by mole of 50/10/38.5/1.5) as the components are called Ex6-L1-Fluc eGFP mRNA and Ex7-L1-Fluc eGFP mRNA, respectively.

### (Measurement of Particle Size)

The particle sizes of the nucleic acid lipid nanoparticle were measured using a particle size analyzer (Zetasizer (registered trademark) Nano ZSP or Ultra, manufactured by Malvern Panalytical) for the nucleic acid lipid nanoparticle dispersions.

### (Evaluation of Encapsulation Efficiency of mRNA)

Regarding the nucleic acid lipid nanoparticle, the encapsulation efficiencies of mRNA in the nucleic acid lipid nanoparticle dispersions which were diluted to an mRNA concentration of around 150 to 1000 ng/mL were measured. Specifically, the nucleic acid lipid nanoparticle obtained as described above were diluted with TE buffer (10 mM Tris/1 mM EDTA, pH 8.0), and the mRNA concentration (A) measured using Quant-iT RiboGreen RNA Reagent (manufactured by Thermo Fisher Scientific) was regarded as the mRNA existing in the external liquid of the nucleic acid lipid nanoparticle. Moreover, the mRNA concentration (B) measured after diluting the nucleic acid lipid nanoparticle with 2% Triton X-100 was regarded as the total mRNA concentration in the composition. Subsequently, the encapsulation efficiency of mRNA was calculated by the following equation (F1).$Encapsulation efficiency \left(%\right) = 100 - \left(A/B\right) \times 100$ (F1)

The particle sizes and the encapsulation efficiencies of the nucleic acid lipid nanoparticle (nucleic acid: Fluc mRNA) are shown in the tables below. Ex1-L1 to Ex75-L1 shown in NUM in the tables below indicate Ex1-L1-FLuc mRNA to Ex75-L1-FLuc mRNA, respectively.

**[Table 57]**

| NUM | Particle Size (nm) | Encapsulation Efficiency (%) |
|---|---|---|
| Ex1-L1 | 101.9 | 98.0 |
| Ex2-L1 | 87.7 | 98.0 |
| Ex3-L1 | 71.8 | 96.6 |
| Ex4-L1 | 81.7 | 98.3 |
| Ex5-L1 | 95.4 | 96.9 |
| Ex6-L1 | 101.1 | 97.2 |
| Ex7-L1 | 103.2 | 96.3 |
| Ex8-L1 | 81.8 | 97.6 |
| Ex9-L1 | 98.6 | 98.7 |
| Ex10-L1 | 83.3 | 97.5 |
| Ex11-L1 | 90.3 | 98.4 |
| Ex12-L1 | 82.0 | 96.4 |
| Ex13-L1 | 84.9 | 97.7 |
| Ex14-L1 | 88.6 | 91.9 |
| Ex15-L1 | 84.1 | 97.2 |
| Ex16-L1 | 88.6 | 94.7 |
| Ex17-L1 | 69.7 | 95.2 |
| Ex18-L1 | 76.6 | 97.3 |
| Ex19-L1 | 85.8 | 97.0 |
| Ex20-L1 | 89.6 | 95.2 |
| Ex21-L1 | 100.9 | 98.3 |
| Ex22-L1 | 91.1 | 97.6 |
| Ex23-L1 | 95.5 | 95.0 |
| Ex24-L1 | 88.0 | 94.6 |
| Ex25-L1 | 89.8 | 95.5 |
| Ex26-L1 | 89.2 | 96.7 |
| Ex27-L1 | 107.9 | 97.0 |
| Ex28-L1 | 93.5 | 97.0 |
| Ex29-L1 | 132.7 | 98.8 |
| Ex30-L1 | 94.1 | 93.2 |
| Ex31-L1 | 101.1 | 93.3 |
| Ex33-L1 | 90.7 | 91.0 |
| Ex34-L1 | 96.0 | 93.9 |
| Ex35-L1 | 104.6 | 93.6 |
| Ex36-L1 | 116.6 | 93.6 |
| Ex37-L1 | 112.6 | 88.4 |
| Ex38-L1 | 91.2 | 93.2 |
| Ex39-L1 | 102.6 | 94.2 |
| Ex40-L1 | 85.9 | 95.3 |
| Ex41-L1 | 91.4 | 94.1 |
| Ex42-L1 | 95.4 | 93.1 |
| Ex43-L1 | 104.2 | 94.1 |
| Ex44-L1 | 93.6 | 93.0 |
| Ex45-L1 | 99.8 | 92.1 |
| Ex46-L1 | 107.2 | 93.3 |
| Ex47-L1 | 108.8 | 94.9 |
| Ex48-L1 | 93.7 | 93.5 |
| Ex49-L1 | 93.7 | 96.1 |
| Ex50-L1 | 102.8 | 92.1 |
| Ex51-L1 | 100.4 | 92.8 |
| Ex52-L1 | 76.9 | 93.2 |
| Ex53-L1 | 92.5 | 94.3 |
| Ex54-L1 | 99.7 | 97.7 |
| Ex55-L1 | 96.5 | 89.8 |
| Ex56-L1 | 101.0 | 92.2 |
| Ex57-L1 | 91.2 | 92.9 |
| Ex58-L1 | 91.3 | 93.0 |
| Ex59-L1 | 90.8 | 92.3 |
| Ex60-L1 | 93.5 | 95.3 |
| Ex61-L1 | 84.7 | 93.9 |
| Ex62-L1 | 93.3 | 94.0 |
| Ex63-L1 | 111.0 | 83.5 |
| Ex64-L1 | 96.4 | 91.7 |
| Ex65-L1 | 90.6 | 93.1 |
| Ex66-L1 | 90.9 | 92.1 |
| Ex67-L1 | 94.2 | 94.7 |
| Ex68-L1 | 82.2 | 95.4 |
| Ex69-L1 | 88.5 | 93.6 |
| Ex70-L1 | 88.2 | 95.8 |
| Ex71-L1 | 95.8 | 88.3 |
| Ex72-L1 | 92.7 | 94.6 |
| Ex73-L1 | 95.5 | 86.0 |
| Ex74-L1 | 105.6 | 83.7 |
| Ex75-L1 | 125.5 | 93.2 |

**[Table 58]**

| NUM | Particle Size (nm) | Encapsulation Efficiency (%) |
|---|---|---|
| Ex1-L1a | 99.2 | 98.1 |
| Ex1-L1b | 101.9 | 98.0 |
| Ex1-L1c | 97.2 | 98.7 |
| Ex1-L2 | 62.5 | 98.7 |
| Ex1-L3 | 68.9 | 98.4 |
| Ex1-L4 | 80.7 | 98.3 |
| Ex1-L5 | 82.4 | 98.6 |
| Ex1-L6 | 86.6 | 98.4 |
| Ex1-L7 | 101.4 | 97.6 |
| Ex1-L8 | 74.5 | 98.1 |
| Ex1-L9 | 86.6 | 98.2 |
| Ex1-L10 | 91.7 | 97.9 |
| Ex1-L11 | 97.9 | 98.0 |
| Ex1-L12 | 103.1 | 98.0 |
| Ex1-L13 | 81.6 | 97.5 |
| Ex1-L14 | 85.4 | 98.0 |
| Ex1-L15 | 117.5 | 98.1 |
| Ex1-L16 | 87.7 | 97.7 |
| Ex1-L17 | 110.0 | 97.5 |
| Ex1-L18 | 115.8 | 98.3 |
| Ex1-L19 | 106.4 | 97.3 |
| Ex1-L20 | 120.8 | 98.0 |
| Ex1-L21 | 131.8 | 97.6 |
| Ex1-L22 | 121.4 | 97.9 |
| Ex1-L23 | 116.5 | 97.4 |
| Ex1-L24 | 141.0 | 96.7 |
| Ex1-L25 | 152.7 | 93.6 |
| Ex1-L26 | 106.8 | 97.6 |
| Ex1-L27 | 83.1 | 97.8 |
| Ex1-L28 | 100.9 | 97.9 |
| Ex1-L29 | 101.6 | 96.8 |
| Ex1-L30 | 124.8 | 97.7 |
| Ex1-L31 | 89.1 | 98.3 |
| Ex1-L32 | 102.7 | 98.4 |
| Ex1-L33 | 130.3 | 99.1 |
| Ex1-L34 | 89.1 | 97.5 |
| Ex1-L35 | 89.9 | 98.7 |
| Ex1-L36 | 99.2 | 96.8 |
| Ex1-L37 | 108.3 | 97.6 |
| Ex1-L38 | 92.9 | 98.2 |
| Ex1-L39 | 106.1 | 98.0 |
| Ex1-L40 | 108.9 | 97.6 |
| Ex1-L41 | 134.0 | 96.7 |
| Ex1-L42 | 98.8 | 98.1 |
| Ex1-L43 | 114.1 | 98.9 |
| Ex1-L44 | 100.8 | 98.3 |
| Ex1-L45 | 98.1 | 98.2 |
| Ex1-L46 | 88.0 | 97.9 |
| Ex1-L47 | 91.4 | 98.9 |
| Ex1-L48 | 133.7 | 91.8 |
| Ex1-L49 | 121.6 | 97.0 |
| Ex1-L50 | 99.8 | 89.6 |
| Ex1-L51 | 179.3 | 98.5 |
| Ex1-L52 | 107.9 | 99.1 |
| Ex1-L53 | 109.7 | 98.2 |
| Ex1-L54 | 100.6 | 98.1 |

The particle sizes and the encapsulation efficiencies of the nucleic acid lipid nanoparticle (nucleic acid: Flue eGFP mixture mRNA) used in Test Example 1-2 are shown in the table below.

**[Table 59]**

| NUM | Particle Size (nm) | Encapsulation Efficiency (%) |
|---|---|---|
| Ex6-L1 | 100.8 | 96.9 |
| Ex7-L1 | 107.9 | 97.0 |

The particle sizes and the encapsulation efficiencies of mRNA of the nucleic acid lipid nanoparticle (nucleic acid: Fluc mRNA) used in Test Example 2 are shown in the table below.

**[Table 60]**

| NUM | Particle Size (nm) | Encapsulation Efficiency (%) |
|---|---|---|
| Ex4-L1 | 98.1 | 97.1 |
| Ex7-L1 | 102.3 | 92.7 |
| Ex12-L1 | 106.6 | 89.0 |
| Ex21-L1 | 97.8 | 98.1 |
| Ex25-L1 | 83.9 | 96.9 |
| Ex30-L1 | 82.4 | 96.5 |
| Ex31-L1 | 101.1 | 93.3 |
| Ex49-L1 | 93.7 | 96.1 |
| Ex55-L1 | 88.6 | 96.4 |
| Ex58-L1 | 91.7 | 95.9 |

The particle size and the encapsulation efficiency of mRNA of the nucleic acid lipid nanoparticle (nucleic acid: NeuroD1 mRNA) used in Test Example 4 are shown in the table below.

**[Table 61]**

| NUM | Particle Size (nm) | Encapsulation Efficiency (%) |
|---|---|---|
| Ex1-L1 | 72.8 | 97.2 |

### [Industrial Applicability]

The compounds of the formula (I) or salts thereof could form lipid nanoparticle. The lipid nanoparticle containing the compounds of the formula (I) or the salts thereof were incorporated into astrocytes or liver cells. Using lipid nanoparticle of the compound of the formula (I) or a salt thereof, a pharmaceutical composition can also be produced by encapsulating a nucleic acid. Lipid nanoparticle and a pharmaceutical composition encapsulating a nucleic acid such as mRNA are expected to be useful for preventing and/or treating an astrocyte-related disease or the like.

### Sequence Listing Free Text

SEQ ID NO: 1: Human NeuroD1 gene (CDS)
SEQ ID NO: 2: Human NeuroD1 protein
SEQ ID NO: 3: Human NeuroD1 gene (CDS)
SEQ ID NO: 4: Human NeuroD1 mRNA sequence (ND1-2)
SEQ ID NO: 5: Human NeuroD1 mRNA sequence containing modified nucleotides (ND1-2)

## Claims

1. A compound of a formula (I) or a salt thereof: 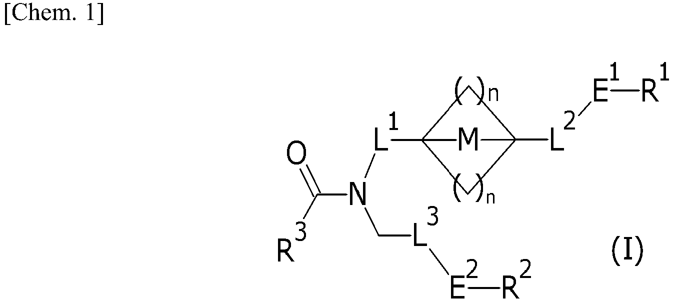 (wherein in the formula,
L¹ and L² are the same or different and are -CH₂-, -CH₂CH₂-, or a bond,
L³ is a bond or a C₁₋₁₀ alkylene,
M is -CH₂- or absent,
n is 1 or 2,
wherein n is 1 when M is -CH₂-,
E¹ and E² are the same or different and are -C(=O)O-*, -OC(=O)-*, -OC(=O)O-*, - C(=O)-*, or a bond,
wherein * indicates that the group binds to R¹ or R² at the position, and
one of E¹ and E² is -C(=O)O-* or -OC(=O)-*,
R¹ and R² are the same or different and are -CH(-R^{x})R^{y}, -CH₂CH(-R^{x})R^{y}, - CH₂CH₂CH(-R^{x})R^{y}, -CH₂CH(-OR^{x})OR^{y}, -CH₂CH₂CH(-OR^{x})OR^{y}, -CH₂-(C₅₋₁₅ alkyl), -CH₂-(C₅₋₂₀ alkenyl), -N(-R^{x})R^{y}, -NR^{y}(-C(=O)R^{x}), or -NR^{y}C(=O)CH(-R^{x})R^{Z},
wherein, when one of R¹ and R² is -N(-R^{x})R^{y}, one of E¹-R¹ and E²-R² is -OC(=O)-N(-R^{x})R^{y} or -C(=O)-N(-R^{x})R^{y},
when one of R¹ and R² is -NR^{y}(-C(=O)R^{x}) or -NR^{y}C(=O)CH(-R^{x})R^{Z}, E¹ bonded to the R¹ or E² bonded to the R² is a bond,
when E¹ and E² are both -OC(=O)-*, R¹ and R² are the same or different and are - CH₂CH₂CH(-OR^{x})OR^{y} or -CH₂-(C₅₋₂₀ alkenyl), and
R^{x}, R^{y}, and R^{Z} are the same or different and are a C₅₋₁₅ alkyl, and
R³ is a group selected from the group consisting of the formulae (a) to (i): 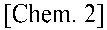 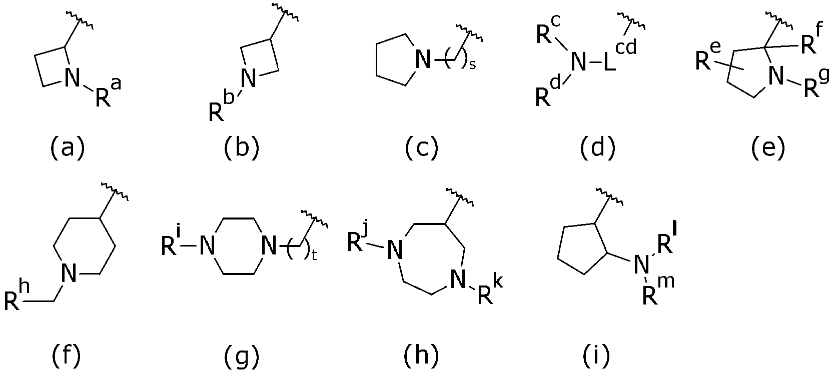
wherein R^{a} is a C₁₋₆ alkyl,
R^{b} is -CH₂-C₁₋₆ alkyl or -C(=O)CH₂N(CH₃)₂,
R^{c} and R^{d} are the same or different and are -CH₃, -CH₂CH₃, or -CH₂CH₂OH, or
R^{d} is -CH₂C(=O)NH₂ when R^{c} is H,
L^{cd} is -CH₂-, -CH₂CH₂-, -CH₂CH₂CH₂-, or -CH₂CH(CH₃)-,
R^{e} is H or OH,
R^{f} is H,
R^{g} is a C₁₋₆ alkyl,
wherein R^{f} and R^{g} may form a pyrrolidine ring together with the carbon atom and the nitrogen atom to which they are bonded,
R^{h} is a C₁₋₆ alkyl,
Rⁱ is a C₁₋₆ alkyl or -CH₂CH₂OH,
R^{j} and R^{k} are the same or different and are a C₁₋₆ alkyl,
R^{l} and R^{m} are the same or different and are a C₁₋₆ alkyl, and
s and t are the same or different and are 1 or 2.)

2. The compound or the salt thereof according to claim 1,
wherein -E¹-R¹ is -C(=O)O-CH₂CH(-R^{x})R^{y},
-C(=O)O-CH₂CH₂CH(-R^{x})R^{y},
-OC(=O)-C₃H₆-(C₁₋₆ alkylene)-CH=CH-CH=CH-(C₁₋₆ alkyl),
-OC(=O)-N(-R^{x})R^{y},
-OC(=O)O-CH(-R^{x})R^{y},
-OC(=O)O-CH₂CH(-R^{x})R^{y},
-NR^{y}(-C(=O)R^{x}),
or
-NR^{y}C(=O)CH(-R^{x})R^{Z},
and
-E²-R² is -C(=O)O-CH₂CH(-R^{x})R^{y},
-C(=O)O-CH₂CH₂CH(-R^{x})R^{y},
-C(=O)O-C₃H₆-(C₁₋₆ alkylene)-CH=CH-CH=CH-(C₁₋₆ alkyl),
-OC(=O)-CH₂CH(-R^{x})R^{y},
-OC(=O)-CH₂CH₂CH(-R^{x})R^{y},
-OC(=O)-CH₂CH₂CH(-OR^{x})OR^{y},
-OC(=O)-C₃H₆-(C₁₋₆ alkylene)-CH=CH-CH=CH-(C₁₋₆ alkyl), or
-OC(=O)O-CH₂CH(-R^{x})R^{y}.

3. The compound of the formula (I) or the salt thereof according to claim 1,
wherein E¹ and E² are the same or different and are -C(=O)O-*, -OC(=O)-*, or - OC(=O)O-*,
wherein * indicates that the group binds to R¹ or R² at the position, and
one of E¹ and E² is -C(=O)O-*,
R¹ and R² are the same or different and are -CH₂CH(-R^{x})R^{y}, -CH₂CH(-OR^{x})OR^{y}, - CH₂CH₂CH(-OR^{x})OR^{y}, -CH₂-(C₅₋₁₅ alkyl), or -CH₂-(C₅₋₂₀ alkenyl),
wherein R^{x} and R^{y} are the same or different and are a C₅₋₁₅ alkyl, and
R³ is a group selected from the group consisting of the formulae (a) to (h): 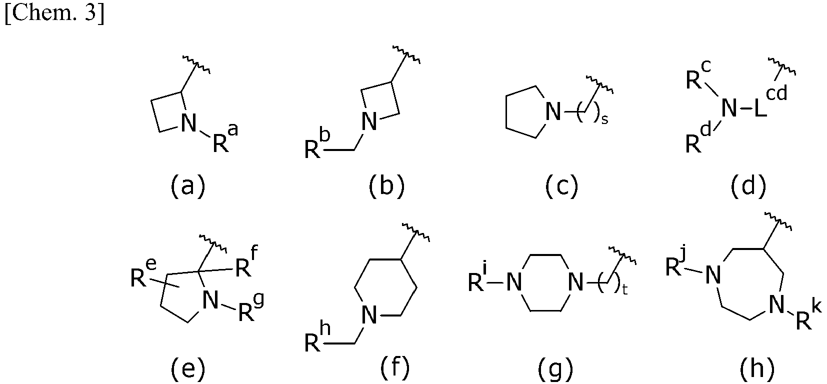
wherein R^{c} and R^{d} are both -CH₃, -CH₂CH₃, or -CH₂CH₂OH, or
R^{d} is -CH₂C(=O)NH₂ when R^{c} is H, and
L^{cd} is -CH₂- or -CH₂CH₂-,
wherein, when R^{c} and R^{d} are both -CH₃, -CH₂CH₃, or -CH₂CH₂OH, L^{cd} is -CH₂-, - CH₂CH₂-, or -CH₂CH₂-.

4. The compound or the salt thereof according to claim 3,
wherein -E¹-R¹ is -C(=O)O-CH₂CH(-R^{x})R^{y},
-OC(=O)-C₃H₆-(C₁₋₆ alkylene)-CH=CH-CH=CH-(C₁₋₆ alkyl),
or
-OC(=O)O-CH₂CH(-R^{x})R^{y},
and
-E²-R² is -C(=O)O-CH₂CH(-R^{x})R^{y},
-C(=O)O-C₃H₆-(C₁₋₆ alkylene)-CH=CH-CH=CH-(C₁₋₆ alkyl),
or
-OC(=O)-C₃H₆-(C₁₋₆ alkylene)-CH=CH-CH=CH-(C₁₋₆ alkyl).

5. The compound or the salt thereof according to claim 2,
wherein L¹ is a bond,
L² is -CH₂- or a bond,
L³ is a C₁₋₆ alkylene,
-E¹-R¹ is -C(=O)O-CH₂CH(-R^{x})R^{y},
-C(=O)O-CH₂CH₂CH(-R^{x})R^{y},
-OC(=O)O-CH(-R^{x})R^{y},
or
-OC(=O)O-CH₂CH(-R^{x})R^{y},
-E²-R² is -C(=O)O-CH₂CH(-R^{x})R^{y},
-C(=O)O-CH₂CH₂CH(-R^{x})R^{y},
-OC(=O)-CH₂CH₂CH(-R^{x})R^{y},
or
-OC(=O)-CH₂CH₂CH(-OR^{x})OR^{y},
wherein R^{x} and R^{y} are the same or different and are a C₅₋₁₅ alkyl, and
R³ is a group selected from the group consisting of the formulae (d), (e), (g), and (h): 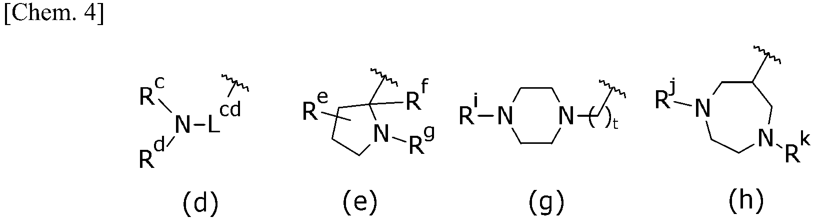
wherein R^{c} and R^{d} are the same or different and are -CH₃, -CH₂CH₃, or - CH₂CH₂OH,
L^{cd} is -CH₂-, -CH₂CH₂-, or -CH₂CH₂CH₂-,
R^{e} is H,
R^{f} is H,
R^{g} is a C₁₋₆ alkyl,
Rⁱ is a C₁₋₆ alkyl,
R^{j} and R^{k} are the same or different and are a C₁₋₆ alkyl, and
t is 1.

6. The compound or the salt thereof according to claim 5,
wherein L³ is a C₆ alkylene,
-E¹-R¹ is -C(=O)O-CH₂CH(-R^{x})R^{y} or -OC(=O)O-CH₂CH(-R^{x})R^{y},
-E²-R² is -C(=O)O-CH₂CH(-R^{x})R^{y},
R^{c} and R^{d} are the same or different and are -CH₃, -CH₂CH₃, or -CH₂CH₂OH, and
L^{cd} is -CH₂-, -CH₂CH₂-, or -CH₂CH₂CH₂-,
wherein, when R^{c} and R^{d} are both -CH₃, -CH₂CH₃, or -CH₂CH₂OH, L^{cd} is -CH₂-, - CH₂CH₂-, or -CH₂CH₂-.

7. The compound or the salt thereof according to claim 1,
wherein the compound is selected from the group consisting of
2-nonylundecyl 8-{(1-methyl-L-prolyl)[(1*r*,3*S*)-3-{2-[(2-octyldecyl)oxy]-2-oxoethyl}cyclobutyl]amino}octanoate,
2-nonylundecyl 8-[(1-methyl-L-prolyl)(3-{2-[(2-octyldecyl)oxy]-2-oxoethyl}bicyclo[1.1.1]pentan-1-yl)amino]octanoate,
2-nonylundecyl 8-{(1-ethyl-L-prolyl)[(1*r*,3*S*)-3-{2-[(2-octyldecyl)oxy]-2-oxoethyl}cyclobutyl]amino}octanoate,
2-nonylundecyl 8-{(*N,N*-diethyl-β-alanyl)[(1*r*,3*r*)-3-{2-[(2-octyldecyl)oxy]-2-oxoethyl}cyclobutyl]amino}octanoate,
2-nonylundecyl 8-{(1,4-diethyl-1,4-diazepane-6-carbonyl)[(1*r*,3*r*)-3-{2-[(2-octyldecyl)oxy]-2-oxoethyl}cyclobutyl]amino}octanoate,
2-nonylundecyl 8-{[(4-methylpiperazin-1-yl)acetyl][(1*r*,3*r*)-3-{2-[(2-octyldecyl)oxy]-2-oxoethyl}cyclobutyl]amino}octanoate,
2-nonylundecyl 8-[(1-methyl-L-prolyl){(1*r*,3*S*)-3-[({[(2-octyldecyl)oxy]carbonyl}oxy)methyl]cyclobutyl}amino]octanoate,
2-heptylnonyl (1*S*,4*r*)-4-[{8-[(2-heptylnonyl)oxy]-8-oxooctyl}(1-methyl-L-prolyl)amino]cyclohexane-1-carboxylate,
2-nonylundecyl 8-{(1-ethyl-D-prolyl)[(1*r*,3*R*)-3-{2-[(2-octyldecyl)oxy]-2-oxoethyl}cyclobutyl]amino}octanoate,
3-decyltridecyl 8-{[(1*r*,3*S*)-3-{2-[(3-decyltridecyl)oxy]-2-oxoethyl}cyclobutyl](1-methyl-L-prolyl)amino}octanoate,
2-{(1-ethyl-L-prolyl)[(1*r*,3*S*)-3-{2-[(2-octyldecyl)oxy]-2-oxoethyl}cyclobutyl]amino}ethyl 4,4-bis(octyloxy)butanoate, and
4-[(1-ethyl-L-prolyl){(1*r*,3*S*)-3-[({[(2-nonylundecyl)oxy]carbonyl}oxy)methyl]cyclobutyl}amino]butyl 4-octyldodecanoate.

8. A lipid nanoparticle comprising the compound or the salt thereof according to claim 1.

9. A lipid nanoparticle comprising the compound or the salt thereof according to claim 1, a neutral lipid, and a PEGylated lipid.

10. The lipid nanoparticle according to claim 9 which encapsulates a nucleic acid.

11. The lipid nanoparticle according to claim 10, wherein the nucleic acid is mRNA.

12. The lipid nanoparticle according to claim 11,
wherein the neutral lipid is a phospholipid and a sterol,
wherein the phospholipid is DPPC, DSPC, SOPC, DoPhPE, DOPS, or DHSM, and
the sterol is cholesterol, 7α-hydroxycholesterol, or β-sitosterol, and
the PEGylated lipid is DMG-PEG2000, PEG monostearate, or C8 PEG2000 ceramide.

13. The lipid nanoparticle according to claim 12 which can express a protein in an astrocyte.

14. The lipid nanoparticle according to claim 13, wherein the nucleic acid is mRNA which is useful for preventing and/or treating an astrocyte-related disease.

15. The lipid nanoparticle according to claim 11,
wherein the nucleic acid is mRNA encoding NeuroD1 protein,
the compound of the formula (I) or the salt thereof is 2-nonylundecyl 8-{(1-methyl-L-prolyl)[(1*r*,3*S*)-3-{2-[(2-octyldecyl)oxy]-2-oxoethyl}cyclobutyl]amino}octanoate or a salt thereof,
the neutral lipid is DSPC and cholesterol, and
the PEGylated lipid is DMG-PEG2000.

16. The lipid nanoparticle according to claim 10, wherein the nucleic acid is mRNA encoding NeuroD1 protein containing a base sequence encoding a protein having the amino acid sequence of SEQ ID NO: 2.

17. The lipid nanoparticle according to claim 15, wherein the nucleic acid is mRNA encoding NeuroD1 protein containing a base sequence encoding a protein having the amino acid sequence of SEQ ID NO: 2.

18. The lipid nanoparticle according to claim 10 which comprises 20.0 to 80.0 mol% of the compound or the salt thereof according to claim 1, 18.5 to 78.5 mol% of the neutral lipid, and 0.5 to 2.5 mol% of the PEGylated lipid based on the total amount of the lipid nanoparticle.

19. The lipid nanoparticle according to claim 10 which comprises 30.0 to 60.0 mol% of the compound or the salt thereof according to claim 1, 38.5 to 68.5 mol% of the neutral lipid, and 0.5 to 2.0 mol% of the PEGylated lipid based on the total amount of the lipid nanoparticle.

20. A pharmaceutical composition comprising the lipid nanoparticle according to any one of claims 10 to 19.

21. A pharmaceutical composition comprising the lipid nanoparticle according to any one of claims 10 to 19 and one or more pharmaceutically acceptable pharmaceutical additives.

22. The pharmaceutical composition according to claim 21 which is a pharmaceutical composition for preventing and/or treating an astrocyte-related disease.

23. Use of the lipid nanoparticle according to any one of claims 10 to 19 for the manufacture of a pharmaceutical composition for preventing and/or treating an astrocyte-related disease.

24. Use of the lipid nanoparticle according to any one of claims 10 to 19 for the prevention and/or the treatment of an astrocyte-related disease.

25. The lipid nanoparticle according to any one of claims 10 to 19 for use in prevention and/or treatment of an astrocyte-related disease.

26. A method for preventing and/or treating an astrocyte-related disease comprising administering an effective amount of the lipid nanoparticle according to any one of claims 10 to 19 to a subject.
